(19) Europäisches Patentamt
European Patent Office
Office européen des brevets

(11) Veröffentlichungsnummer: **0 385 405 A2**

(12) # EUROPÄISCHE PATENTANMELDUNG

(21) Anmeldenummer: **90103813.3**

(22) Anmeldetag: **27.02.90**

(51) Int. Cl.5: **C07D 498/18, A61K 31/495, //(C07D498/18,323:00,307:00, 263:00)**

(30) Priorität: **02.03.89 CH 776/89**
**21.12.89 US 454325**

(43) Veröffentlichungstag der Anmeldung: **05.09.90 Patentblatt 90/36**

(84) Benannte Vertragsstaaten:
**AT BE CH DE DK ES FR GB GR IT LI LU NL SE**

(71) Anmelder: **CIBA-GEIGY AG**
**Klybeckstrasse 141**
**CH-4002 Basel(CH)**

(72) Erfinder: **Kump, Wilhelm, Dr.**
**Friedrich-Oser-Strasse 10**
**CH-4105 Biel-Benken(CH)**
Erfinder: **Borel, Christian, Dr.**
**86, route de la Plaine**
**CH-1283 La Plaine/Genève(CH)**
Erfinder: **Chen, Jen, Dr.**
**Delsbergerallee 11**
**CH-4053 Basel(CH)**
Erfinder: **Veenstra, Siem J., Dr.**
**38 West Street**
**Madison, New Jersey 07940(US)**

(74) Vertreter: **Zumstein, Fritz, Dr. et al**
**Bräuhausstrasse 4**
**D-8000 München 2(DE)**

(54) **Acyl-Derivate.**

(57) Gegenstand der Erfindung sind neue Acyl-Derivate von Rifamycinen der Formel

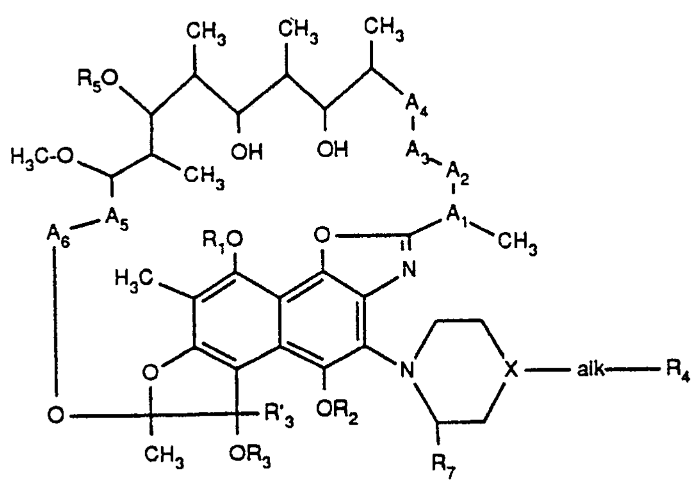

(I),

und ihre Salze, worin die Strukturelemente $-A_1-A_2-$, $-A_3-A_4-$ und $-A_5-A_6-$ jeweils Ethylen oder Vinylen oder die Elemente $-A_1-A_2-$ und $-A_3-A_4-$ jeweils Ethylen und $-A_5-A_6-$Vinylen bedeuten; X für $>C(R_6)-$ oder $>N-$ steht und $R_6$ Wasserstoff oder Alkyl bedeutet; alk einen aliphatischen Kohlenwasserstoffrest bedeutet; $R_1$ Wasserstoff oder Acyl bedeutet; $R_2$ Acyl bedeutet und $R_3$ und $R_3'$ für eine gemeinsame Bindung stehen oder $R_3$ Wasserstoff oder Acyl bedeutet und $R_3'$ Wasserstoff ist; $R_4$ Wasserstoff, Cycloalkyl oder Aryl bedeutet; $R_5$ Wasserstoff oder Acetyl bedeutet; $R_7$ Wasserstoff oder Alkyl bedeutet,

EP 0 385 405 A2

die als Arzneimittelwirkstoffe verwendet werden können, ihre Herstellung und Verwendung sowie pharmazeutische Präparate und ihre Herstellung.

## Acyl-Derivate

Gegenstand der Erfindung sind neue Acyl-Derivate von Rifamycinen der Formel

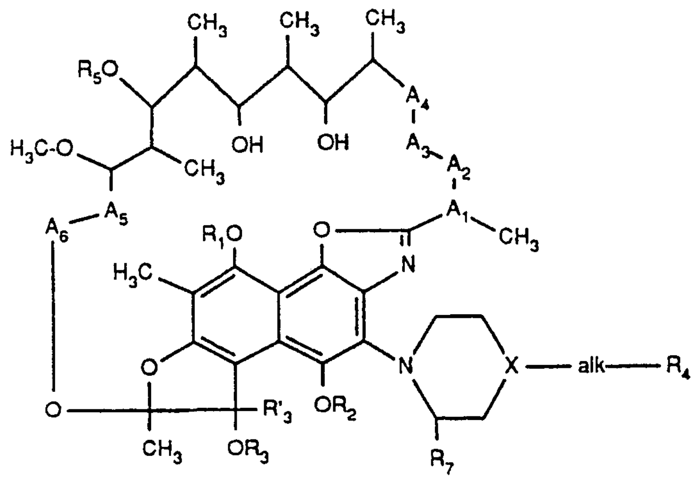

(I),

und ihre Salze, worin die Strukturelemente $-A_1-A_2-$, $-A_3-A_4-$ und $-A_5-A_6-$ jeweils Ethylen oder Vinylen oder die Elemente $-A_1-A_2-$ und $-A_3-A_4-$ jeweils Ethylen und $-A_5-A_6-$Vinylen bedeuten; X für $>C(R_6)-$ oder $>N-$ steht und $R_6$ Wasserstoff oder Alkyl bedeutet; alk einen aliphatischen Kohlenwasserstoffrest bedeutet; $R_1$ Wasserstoff oder Acyl bedeutet; $R_2$ Acyl bedeutet und $R_3$ und $R_3'$ für eine gemeinsame Bindung stehen oder $R_3$ Wasserstoff oder Acyl bedeutet und $R_3'$ Wasserstoff ist; $R_4$ Wasserstoff, Cycloalkyl oder Aryl bedeutet; $R_5$ Wasserstoff oder Acetyl bedeutet; $R_7$ Wasserstoff oder Alkyl bedeutet; ihre Herstellung und Verwendung sowie pharmazeutische Präparate und ihre Herstellung.

Die zugrundeliegende Numerierung des Ringsystems entspricht im wesentlichen derjenigen, die z.B. im US Patent Nr. 4,005,077 angewandt wurde.

Die Verbindungen der Formel I besitzen mehrere Chiralitätszentren, dementsprechend umfasst die vorliegende Erfindung auch die entsprechenden optischen Isomeren, z.B. Diastereoisomeren.

Die Verbindungen der Formel I können als, insbesondere pharmazeutisch verwendbare, Salze vorliegen. Da die erfindungsgemässen Verbindungen mindestens ein basisches Zentrum aufweisen, können sie somit Säureadditionssalze bilden. Diese werden beispielsweise mit anorganischen Säuren, wie Mineralsäuren, z.B. Schwefelsäure, eine Phosphor- oder Halogenwasserstoffsäure, oder mit organischen Carbonsäuren, wie gegebenenfalls, z.B. durch Halogen, substituierte $C_1$-$C_4$-Alkancarbonsäuren, z.B. Essigsäure, wie gegebenenfalls ungesättigte Dicarbonsäuren, z.B. Oxal-, Malon-, Bernstein-, Malein-, Fumar-, Phthal-, oder Terephthalsäure, wie Hydroxycarbonsäuren, z.B. Ascorbin-, Glykol-, Milch-, Aepfel-, Wein- oder Citronensäure, wie Aminosäuren, z.B. Asparagin- oder Glutaminsäure, einer Benzoesäure oder mit organischen Sulfonsäuren, wie gegebenenfalls, z.B. durch Halogen, substituierte $C_1$-$C_4$-Alkan- oder Arylsulfonsäuren, z.B. Methan-, Brombenzol- oder p-Toluolsulfonsäure, gebildet. Entsprechende Säureadditionssalze können auch mit einem gegebenenfalls zusätzlich vorhandenen (z.B. X = $>N-$) basischen Zentrum gebildet werden. Ferner können die erfindungsgemässen Verbindungen mit einer aciden phenolischen Hydroxygruppe Salze ($R_2$ = H) mit Basen bilden, z.B. Alkalimetall-, wie Natrium- oder Kaliumsalze. Weiterhin können entsprechende innere Salze gebildet werden. Umfasst sind ferner für pharmazeutische Verwendungen nicht geeignete Salze, da diese beispielsweise für die Isolierung bzw. Reinigung erfindungsgemässer Verbindungen oder deren pharmazeutisch verwendbarer Salze eingesetzt werden können.

Ein aliphatischer Kohlenwasserstoffrest bedeutet insbesondere Alkylen, Alkenylen und Alkinylen, wobei die Mehrfachbindung in höherer als der $\alpha$-Stellung zum Piperazinstickstoffatom (X = $>N-$) lokalisiert ist.

Acyl leitet sich z.B. von einer organischen Carbonsäure, einer substituierten Kohlensäure oder einer organischen Sulfonsäure ab. Derartige Reste sind beispielsweise Alkanoyl, Halogenalkanoyl, Arylalkanoyl, carbocyclisches oder heterocyclisches Aroyl, Alkoxycarbonyl, Arylalkoxycarbonyl, gegebenenfalls durch Alkyl mono- oder disubstituiertes Amino- carbonyl, Alkansulfonyl, Halogenalkansulfonyl, Arylalkansulfonyl, Cycloalkansulfonyl oder Arylsulfonyl. Dabei bedeuten jeweils Aryl insbesondere Phenyl oder Naphthyl und carbocyclisches Aroyl insbesondere Benzoyl oder Naphthoyl und heterocyc lisches Aroyl insbesondere 5-oder 6-gliedriges monocyclisches Monoaza-, Monooxa-oder Monothiaaroyl, wie Pyrroloyl, Pyridoyl, Furoyl oder Thenoyl, wobei derartige Aryl-oder Aroylreste unsubstituiert oder ein- oder mehrfach, z.B. zwei- oder dreifach, substituiert sind, beispielsweise durch Substituenten ausgewählt aus der Gruppe bestehend aus Halogen, Alkyl, Alkoxy, Hydroxy, Alkanoyloxy, Trifluormethyl und Nitro.

Aryl $R_4$ leitet sich z.B. von einem mono- oder polycyclischen, wie bicyclischen, C-Ringsystem ab, das mindestens einen aromatischen Ring aufweist, wie Phenyl, Biphenylyl, wie 2-, 3- oder insbesondere 4-Biphenylyl, oder Naphthyl, wie 1- oder 2-Naphthyl, und unsubstituiert oder substituiert ist, z.B. ein- oder mehrfach, wie zwei- oder dreifach, beispielsweise durch Substituenten ausgewählt aus der Gruppe bestehend aus Halogen, ($C_1$-$C_7$-)Alkyl, ($C_1$-$C_7$-)Alkoxy, Hydroxy, ($C_2$-$C_8$-)Alkanoyloxy, Trifluormethyl und Nitro.

Die vor- und nachstehend verwendeten Allgemeindefinitionen haben, sofern nicht abweichend definiert, in erster Linie die folgenden Bedeutungen:

Alkyl bedeutet insbesondere $C_1$-$C_7$-Alkyl und ist z.B. Methyl, Ethyl, Propyl, Isopropyl, n-Butyl, Isobutyl, sek.-Butyl, tert.-Butyl und umfasst ferner entsprechende Pentyl-, Hexyl-und Heptylreste. Bevorzugt ist $C_1$-$C_4$-Alkyl.

Cycloalkyl bedeutet insbesondere $C_3$-$C_7$-Cycloalkyl und bedeutet z.B. Cyclopropyl, -butyl, -pentyl, -hexyl oder -heptyl. Bevorzugt ist Cyclopentyl und Cyclohexyl.

Alkylen bedeutet insbesondere $C_1$-$C_7$-Alkylen und ist geradkettig oder verzweigt und bedeutet z.B. Methylen, Ethylen, Propylen und Butylen sowie 1,2-Propylen, 2-Methyl-1,3-propylen oder 2,2-Dimethyl-1,3-propylen. Bevorzugt ist $C_1$-$C_4$-Alkylen, in erster Linie Methylen.

Alkenylen bedeutet insbesondere $C_3$-$C_7$-Alkenylen und ist geradkettig oder verzweigt und bedeutet z.B. 1,3-Prop-2-enylen, 1,4-But-2-, 1,4-But-3-enylen, 1,3-But-2-enylen, 2,4-But-3-enylen, 1,5-Pent-2-, -3-, -4-enylen, ferner entsprechende Hexenylen- und Heptenylenreste. Bevorzugt ist $C_3$-$C_5$-Alkenylen.

Alkinylen bedeutet insbesondere $C_3$-$C_7$-Alkinylen und ist geradkettig oder verzweigt und bedeutet z.B. 1,3-Prop-2-inylen, 1,4-But-2-, 1,4-But-3-inylen, 1,5-Pent-2-, -3- und -5-inylen, ferner entsprechende Hexinylen- und Heptinylenreste. Bevorzugt ist $C_3$-$C_5$-Alkinylen.

Alkanoyl bedeutet insbesondere $C_2$-$C_8$-Alkanoyl und ist z.B. Acetyl, Propionyl, Butyryl, Isobutyryl oder Pivaloyl. Bevorzugt,insbesondere für $R_1$ ist verzweigtes $C_2$-$C_6$-Alkanoyl, in erster Linie Pivaloyl, während insbesondere für $R_2$ und $R_3$ $C_2$-$C_6$Alkanoyl, in erster Linie Acetyl oder Propionyl, steht.

Halogen ist insbesondere Halogen mit Atomnummer bis und mit 35, wie Fluor, Chlor und Brom, ferner Iod.

Alkoxy bedeutet insbesondere $C_1$-$C_7$-Alkoxy und ist z.B. Methoxy, Ethoxy, Propyloxy, Isopropyloxy, n-Butyloxy, Isobutyloxy und tert.-Butyloxy. Bevorzugt ist $C_1$-$C_4$-Alkoxy.

In Alkoxycarbonyl hat Alkoxy die vorstehend angegebenen Bedeutungen.

Von Derivaten, die sich beispielsweise vom Rifamycin SV ableiten, ist bekannt, dass sie ausgeprägte antibiotische Eigenschaften besitzen und beispielsweise zur Behandlung von Tuberkulose eingesetzt werden können. Umso bedeutender ist die experimentell verifizierte Feststellung, dass die Verbindungen der Formel I und ihre pharmazeutisch verwendbaren Salze in den üblichen pharmakologischen Testmodellen keine entsprechende antibiotische Aktivität zeigen.

Ueberraschenderweise dagegen besitzen sie jedoch eine signifikante lipidsenkende Wirkung, die in Tierversuchen, vorzugsweise an Säugetieren, z.B. an Ratten, nachgewiesen werden kann. So kann die Senkung von "very low density"-, "low-density"- bzw. "high density"-Lipoproteinen (VLDL, LDL bzw. HDL) im Serum in zwei Testanordnungen, und zwar in genetisch hypercholesterolämischen männlichen Ratten (Anordnung A) und normolipämischen Ratten beiden Geschlechts (Anordnung B) gezeigt werden.

Albino-Ratten (Sprague Dawley Abkömmlinge des Stammes Tif:RAI) mit einem Körpergewicht von 180-240 g, die freien Zugang zu Standard-Rattenfutter und Trinkwasser haben, werden verwendet. Die Testverbindung wird in einer verstärkten Maisstärkelösung (3% wässrige Maisstärke mit 0,33 % Tween 80 und 5% Polyethylenglykollösung mit einem mittleren Molekulargewicht von 400) oral an Gruppen von 6 Ratten täglich während 5 aufeinanderfolgenden Tagen verabreicht. Die Tiere werden zwei Stunden nach der letzten Gabe getötet. Während 16 Stunden vor ihrem Tod erhalten die Tiere keine Nahrung mehr. Das Blut wird in einer 0,05%igen wässrigen Ethylendiamintetraessigsäure-Lösung gesammelt. Nach Zentrifugation bis zur Sedimentierung der Blutzellen werden der Gehalt an Cholesterin und Triglyceriden enzymatisch mit Hilfe der z.B. von Sigma Chemical Co. (St.Louis, MO, USA) gelieferten Testsysteme analysiert. Zur HDL-Cholesterin-Bestimmung wird eine Lösung von Heparin und Manganchlorid (Endkonzentration = 1,3 g/l bzw. 46 mMol) zu 0,5 ml EDTA-Plasma gegeben. Das gebildete Präzipitat wird durch Zentrifugation sedimentiert, und die Cholesterin-Konzentration des Ueberstandes wird wie für das totale Cholesterin enzymatisch analysiert. Der Unterschied zwischen diesem Wert und dem Cholesterin-Wert, der von einem Aliquot des Gesamtserums direkt bestimmt wird, ist gemäss Warnick, G.R. et al., J.Lipid Res. 19; 65-76-(1978), äquivalent dem VLDL- und LDL-Cholesterin.

Die Prüfung auf antibiotische Wirkung erfolgt z.B. einerseits in vitro durch die Bestimmung der mittleren effektiven Konzentration $EC_{50}$ für die Hemmung der RNA-Polymerase von Escherichia coli, sowie der minimalen Hemmkonzentration MIC ("minimum inhibitory concentration") im konventionellen Plattentest, andererseits in vivo an infizierten Mäusen und Ratten durch die Bestimmung von $ED_{50}$ (effektive Dosis, die für 50 % der Versuchstiere lebenserhaltend ist). Als Mikroorganismen werden für den vorliegenden Zweck insbesondere Mycobacterium tüberculosis TB $H_{37}Rv$ und Staphylococcus aureus verwendet. Bei Verbindungen mit lipidsenkender Indikation wird eine antibiotische Wirksamkeit als nachteilig erachtet, da sie, insbesondere bei langfristiger Verabreichung, zur Bildung von gegen Antibiotika resistenten Stämmen von Mikroorganismen führen kann.

In den oben beschriebenen Testmethoden weisen die erfindungsgemässen Verbindungen bei wiederholter Verabreichung im Dosisbereich von etwa 0,1 bis etwa 50 mg/kg/Tag eine signifikante hypolipidämische Wirksamkeit auf; dagegen sind sie in den oben erwähnten Tests frei von nennenswerter antibiotischer Aktivität.

So kann z.B. gezeigt werden, dass, je nach Versuchsanordnung, die minimale effektive Dosis der erfindungsgemässen Verbindungen bei einmaliger Verabreichung bei etwa 0,1 bis etwa 10 mg/kg liegt, und durch eine wiederholte Verabreichung von täglich 30 mg/kg eine 50-70%ige Senkung des totalen Cholesterins erreicht werden kann. Dabei haben die Verbindungen praktisch keine antibiotische Wirksamkeit; eine $EC_{50}$ für die Inhibition der RNA-Polymerase wird mit 100 µg/ml noch nicht erreicht, und die MIC für verschiedene pathogene Stämme von Staphylococcus aureus liegt über 130 µg/ml. Solche Werte sind etwa 1000-fach höher als Konzentrationen, die für einen entsprechenden Effekt normalerweise erforderlich sind. Auch in vivo unter Verwendung von mit Staphylococcus aureus infizierten Mäusen erweist sich die Verbindung bei einer Einzeldosis von 200 mg/kg als antibiotisch unwirksam.

Dank ihrer LDL-senkenden Wirkung können die erfindungsgemässen Verbindungen z.B. als Hypolipidämika zur Behandlung von Hyperlipidämien, hauptsächlich der Typen IIa und IIb, und Arteriosclerose, z.B. bei Vorliegen von Hyperlipoproteinämie als Risikofaktor, verwendet werden. Weiterhin erweisen sich die erfindungsgemässen Verbindungen in hohem Masse als stabil.
Dementsprechend können die Verbindungen der Formel I und ihre pharmazeutisch verwendbaren Salze z.B. als Pharmazeutika, beispielsweise als Hypolipidämika zur Behandlung von Hyperlipidämien, hauptsächlich der Typen IIa und IIb, und von Arteriosklerose bei Vorliegen von Hyperlipoproteinamie als Risikofaktor, verwendet werden. Ein weiterer Gegenstand der Erfindung ist die Verwendung der erfindungsgemässen Verbindungen zur Herstellung von Arzneimitteln, insbesondere von Hypolipidämika und Antiarteriosklerotika, und zur therapeutischen und prophylaktischen Behandlung. Dabei ist auch die gewerbsmässige Herrichtung der Wirksubstanzen eingeschlossen.

Die Erfindung betrifft insbesondere Verbindungen der Formel I und ihre Salze, worin die Strukturelemente $-A_1-A_2-$, $-A_3-A_4-$ und $-A_5-A_6-$ jeweils Ethylen oder Vinylen oder die Elemente $-A_1-A_2-$ und $-A_3-A_4-$ jeweils Ethylen und $-A_5-A_6-$ Vinylen bedeuten; X für $>$ $C(R_6)$- oder $>$ N- steht und $R_6$ Wasserstoff ist; alk einen aliphatischen Kohlenwasserstoffrest bedeutet; $R_1$ Wasserstoff oder Acyl bedeutet; $R_2$ und $R_3$ unabhängig voneinander Acyl bedeuten; $R_3'$ Wasserstoff bedeutet; $R_4$ Wasserstoff, Cycloalkyl oder Aryl bedeutet; $R_5$ Wasserstoff oder Acetyl bedeutet; $R_7$ Wasserstoff bedeutet.

Die Erfindung betrifft insbesondere Verbindungen der Formel I und ihre Salze, worin $-A_1-A_2-$, $-A_3-A_4-$, $-A_5-A_6-$ und $R_5$ die angegebenen Bedeutungen haben; X für $>$ $C(R_6)$- oder $>$ N- steht und $R_6$ Wasserstoff oder $C_1$-$C_7$-Alkyl bedeutet; alk $C_1$-$C_7$-Alkylen, $C_3$-$C_7$-Alkenylen oder $C_3$-$C_7$-Alkinylen bedeutet; $R_1$ $C_2$-$C_8$-Alkanoyl, Halogen-$C_2$-$C_8$-alkanoyl, Phenyl- oder Naphthyl-$C_2$-$C_8$-alkanoyl, Benzoyl, Naphthoyl, 5- oder 6-gliedriges monocyclisches Monoaza-, Monooxa- oder Monothiaaroyl, $C_1$-$C_7$-Alkoxycarbonyl, Phenyl- oder Naphthyl-$C_1$-$C_7$-alkoxy-carbonyl, unsubstituiertes oder durch $C_1$-$C_7$-Alkyl mono- oder di-substituiertes Aminocarbonyl, $C_1$-$C_7$-Alkansulfonyl, Halogen-$C_1$-$C_7$-alkansulfonyl, Phenyl- oder Naphthyl-$C_1$-$C_7$-alkansulfonyl, $C_3$-$C_7$-Cycloalkansulfonyl oder Benzol- oder Naphthylsulfonyl bedeutet; $R_2$ $C_2$-$C_8$-Alkanoyl, Halogen-$C_2$-$C_8$-alkanoyl, Phenyl- oder Naphthyl-$C_2$-$C_8$-alkanoyl, Benzoyl, Naphthoyl, 5- oder 6-gliedriges monocyclisches Monoaza-, Monooxa- oder Monothiaaroyl, $C_1$-$C_7$-Alkoxycarbonyl, Phenyl- oder Naphthyl-$C_1$-$C_7$-alkoxy-carbonyl, unsubstituiertes oder durch $C_1$-$C_7$-Alkyl mono- oder di-substituiertes Aminocarbonyl, $C_1$-$C_7$-Alkansulfonyl, Halogen-$C_1$-$C_7$-alkansulfonyl, Phenyl- oder Naphthyl-$C_1$-$C_7$-alkansulfonyl, $C_3$-$C_7$-Cycloalkansulfonyl oder Benzol- oder Naphthylsulfonyl bedeutet, $R_3$ und $R_3'$ für eine gemeinsame Bindung stehen oder $R_3$ Wasserstoff oder $C_2$-$C_8$-Alkanoyl, Halogen-$C_2$-$C_8$-alkanoyl, Phenyl- oder Naphthyl-$C_2$-$C_8$-alkanoyl, Benzoyl, Naphthoyl, 5-oder 6-gliedriges monocyclisches Monoaza-, Monooxa- oder Monothiaaroyl, $C_1$-$C_7$-Alkoxycarbonyl, Phenyl- oder Naphthyl-$C_1$-$C_7$-alkoxy-carbonyl, unsubstituiertes oder durch $C_1$-$C_7$-Alkyl mono- oder di-substituiertes Aminocarbonyl, $C_1$-$C_7$-Alkansulfonyl, Halogen-$C_1$-$C_7$-alkansulfonyl, Phenyl- oder Naphthyl-$C_1$-$C_7$-alkansulfonyl, $C_3$-$C_7$-Cycloalkansulfonyl oder Benzol- oder Naphthylsulfonyl bedeutet und $R_3'$ Wasserstoff ist; $R_4$ Wasserstoff, $C_3$-$C_7$-Cycloalkyl, Phenyl, Biphenylyl oder Naphthyl bedeutet; $R_7$

Wasserstoff oder $C_1$-$C_7$-Alkyl bedeutet; wobei die jeweiligen aromatischen Reste unsubstituiert oder ein- oder mehrfach substituiert sind durch Substituenten ausgewählt aus der Gruppe bestehend aus Halogen, $C_1$-$C_7$-Alkyl, $C_1$-$C_7$-Alkoxy, Hydroxy, $C_2$-$C_8$-Alkanoyloxy, Trifluormethyl und Nitro.

Die Erfindung betrifft insbesondere Verbindungen der Formel I und ihre Salze, worin -$A_1$-$A_2$-, -$A_3$-$A_4$-, -$A_5$-$A_6$- und $R_5$ die angegebenen Bedeutungen haben; X für $>$ C($R_6$)- oder $>$ N- steht und $R_6$ Wasserstoff ist; alk $C_1$-$C_7$-Alkylen, $C_3$-$C_7$-Alkenylen oder $C_3$-$C_7$-Alkinylen bedeutet; $R_1$, $R_2$ und $R_3$ unabhängig voneinander $C_2$-$C_8$-Alkanoyl, Halogen-$C_2$-$C_8$-alkanoyl, Phenyl- oder Naphthyl-$C_2$-$C_8$-alkanoyl, Benzoyl, Naphthoyl, 5- oder 6-gliedriges monocyclisches Monoaza-, Monooxa- oder Monothiaaroyl, $C_1$-$C_7$-Alkoxycarbonyl, Phenyl- oder Naphthyl-$C_1$-$C_7$-alkoxy-carbonyl, unsubstituiertes oder durch $C_1$-$C_7$-Alkyl mono- oder di-substituiertes Aminocarbonyl, $C_1$-$C_7$-Alkansulfonyl, Halogen-$C_1$-$C_7$-alkansulfonyl, Phenyl- oder Naphthyl-$C_1$-$C_7$-alkansulfonyl, $C_3$-$C_7$-Cycloalkansulfonyl oder Benzol- oder Naphthylsulfonyl bedeuten und $R_1$ zusätzlich sowie $R_3'$ und $R_7$ Wasserstoff bedeuten; $R_4$ Wasserstoff, $C_3$-$C_7$-Cycloalkyl, Phenyl, Biphenylyl oder Naphthyl bedeutet; wobei die jeweiligen aromatischen Reste unsubstituiert oder ein- oder mehrfach substituiert sind durch Substituenten ausgewählt aus der Gruppe bestehend aus Halogen, $C_1$-$C_7$-Alkyl, $C_1$-$C_7$-Alkoxy, Hydroxy, $C_2$-$C_8$-Alkanoyloxy, Trifluormethyl und Nitro.

Die Erfindung betrifft insbesondere Verbindungen der Formel I und ihre Salze, worin -$A_1$-$A_2$-, -$A_3$-$A_4$-, -$A_5$-$A_6$- und $R_5$ die angegebenen Bedeutungen haben; X für $>$ C($R_6$)- oder $>$ N- steht und $R_6$ Wasserstoff ist; alk $C_1$-$C_7$-Alkylen, $C_3$-$C_7$-Alkenylen oder $C_3$-$C_7$-Alkinylen, insbesondere $C_1$-$C_4$-Alkylen, $C_3$-$C_4$-Alkenylen oder $C_3$-$C_4$-Alkinylen, bedeutet, wobei die Mehrfachbindungen in höherer als der α-Stellung zum Piperazinstickstoff lokalisiert sind; $R_1$ $C_2$-$C_8$-Alkanoyl, insbesondere $C_2$-$C_6$-Alkanoyl, Phenyl-$C_2$-$C_6$-alkanoyl, Benzoyl, $C_1$-$C_7$-Alkoxy-carbonyl, wie $C_1$-$C_4$-Alkoxycarbonyl, Phenyl-$C_1$-$C_4$-alkoxycarbonyl, $C_1$-$C_4$-Alkansulfonyl oder Benzolsulfonyl bedeutet; $R_2$ $C_2$-$C_8$-Alkanoyl, insbesondere $C_2$-$C_6$-Alkanoyl, Phenyl-$C_2$-$C_6$-alkanoyl, $C_1$-$C_7$-Alkoxy-carbonyl, wie $C_1$-$C_4$-Alkoxycarbonyl, Phenyl-$C_1$-$C_4$-alkoxycarbonyl, $C_1$-$C_4$-Alkansulfonyl oder Benzolsulfonyl bedeutet; $R_3$ und $R_3'$ gemeinsam für eine Bindung stehen oder $R_3$ Wasserstoff, $C_2$-$C_8$-Alkanoyl, insbesondere $C_2$-$C_6$-Alkanoyl, Phenyl-$C_2$-$C_6$-alkanoyl, Benzoyl, $C_1$-$C_7$-Alkoxy-carbonyl, wie $C_1$-$C_4$-Alkoxycarbonyl, Phenyl-$C_1$-$C_4$-alkoxycarbonyl, $C_1$-$C_4$-Alkansulfonyl oder Benzolsulfonyl bedeutet und $R_3'$ Wasserstoff ist; $R_4$ Wasserstoff, $C_3$-$C_7$-Cycloalkyl, Phenyl, Biphenylyl oder Naphthyl bedeutet; $R_7$ Wasserstoff oder $C_1$-$C_7$-Alkyl bedeutet; wobei die jeweiligen aromatischen Reste unsubstituiert oder ein- oder mehrfach substituiert sind durch Substituenten ausgewählt aus der Gruppe bestehend aus Halogen, $C_1$-$C_7$-Alkyl, $C_1$-$C_7$-Alkoxy, Hydroxy, $C_2$-$C_8$-Alkanoyloxy, Trifluormethyl und Nitro.

Die Erfindung betrifft insbesondere Verbindungen der Formel I und ihre Salze, worin -$A_1$-$A_2$-, -$A_3$-$A_4$-, -$A_5$-$A_6$- und $R_5$ die angegebenen Bedeutungen haben; X für $>$ C($R_6$)- oder $>$ N- steht und $R_6$ Wasserstoff ist; alk $C_1$-$C_7$-Alkylen, $C_3$-$C_7$-Alkenylen oder $C_3$-$C_7$-Alkinylen, insbesondere $C_1$-$C_4$-Alkylen, $C_3$-$C_4$-Alkenylen oder $C_3$-$C_4$-Alkinylen, bedeutet, wobei die Mehrfachbindungen in höherer als der α-Stellung zum Piperazinstickstoff lokalisiert sind; $R_1$, $R_2$ und $R_3$ unabhängig voneinander $C_2$-$C_8$-Alkanoyl, insbesondere $C_2$-$C_6$-Alkanoyl, Phenyl-$C_2$-$C_6$-alkanoyl, $C_1$-$C_7$-Alkoxy-carbonyl, wie $C_1$-$C_4$-Alkoxycarbonyl, Phenyl-$C_1$-$C_4$-alkoxycarbonyl, $C_1$-$C_4$-Alkansulfonyl oder Benzolsulfonyl bedeuten und $R_1$ zusätzlich sowie $R_3$ und $R_7$ Wasserstoff bedeuten; $R_4$ Wasserstoff, $C_3$-$C_7$-Cycloalkyl, Phenyl, Biphenylyl oder Naphthyl bedeutet; wobei die jeweiligen aromatischen Reste unsubstituiert oder ein- oder mehrfach substituiert sind durch Substituenten ausgewählt aus der Gruppe bestehend aus Halogen, $C_1$-$C_7$-Alkyl, $C_1$-$C_7$-Alkoxy, Hydroxy, $C_2$-$C_8$-Alkanoyloxy, Trifluormethyl und Nitro.

Die Erfindung betrifft insbesondere Verbindungen der Formel I und ihre Salze, worin -$A_1$-$A_2$-, -$A_3$-$A_4$-, -$A_5$-$A_6$- die angegebenen Bedeutungen haben; X für $>$ C($R_6$)-oder $>$ N- steht und $R_6$ Wasserstoff ist; alk $C_1$-$C_7$-Alkylen, $C_3$-$C_7$-Alkenylen oder $C_3$-$C_7$-Alkinylen, insbesondere $C_1$-$C_4$-Alkylen, $C_3$-$C_4$-Alkenylen oder $C_3$-$C_4$-Alkinylen, bedeutet, wobei die Mehrfachbindungen in höherer als der α-Stellung zum Piperazinstickstoff lokalisiert sind; $R_1$, $R_2$ und $R_3$ unabhängig voneinander $C_2$-$C_8$-Alkanoyl, insbesondere $C_2$-$C_6$-Alkanoyl, $C_1$-$C_4$-Alkoxy-carbonyl, $C_1$-$C_4$-Alkansulfonyl oder Benzolsulfonyl bedeuten, $R_1$ zusätzlich sowie $R_3'$ und $R_7$ Wasserstoff bedeuten; $R_4$ Wasserstoff, $C_3$-$C_7$-Cycloalkyl, Phenyl, Biphenylyl oder Naphthyl bedeutet; $R_5$ Acetyl ist; wobei die jeweiligen aromatischen Reste unsubstituiert oder ein- oder mehrfach substituiert sind durch Substituenten ausgewählt aus der Gruppe bestehend aus Halogen, $C_1$-$C_4$-Alkyl, $C_1$-$C_4$-Alkoxy, Hydroxy, $C_2$-$C_6$-Alkanoyloxy, Trifluormethyl und Nitro.

Die Erfindung betrifft insbesondere Verbindungen der Formel I und ihre Salze, worin -$A_1$-$A_2$-, -$A_3$-$A_4$-, -$A_5$-$A_6$- die angegebenen Bedeutungen haben; X für $>$ C($R_6$)-oder $>$ N- steht und $R_6$ Wasserstoff ist; alk $C_1$-$C_7$-Alkylen, insbesondere $C_1$-$C_4$-Alkylen, bedeutet; $R_1$ $C_2$-$C_8$-Alkanoyl, insbesondere $C_2$-$C_6$-Alkanoyl, bedeutet; $R_2$ $C_2$-$C_8$-Alkanoyl, insbesondere $C_2$-$C_6$-Alkanoyl, Benzoyl, $C_1$-$C_4$-Alkansulfonyl oder Benzolsulfonyl bedeutet, $R_3$ und $R_3'$ gemeinsam für eine Bindung stehen oder $R_3$ Wasserstoff, $C_2$-$C_8$-Alkanoyl, insbesondere $C_2$-$C_6$-Alkanoyl, Benzoyl, $C_1$-$C_4$-Alkansulfonyl oder Benzolsulfonyl bedeutet und $R_3'$ Wasserstoff ist; $R_4$ Wasserstoff, $C_3$-$C_7$-Cycloalkyl, Phenyl, Biphenylyl oder Naphthyl bedeutet, $R_5$ Acetyl ist;

$R_7$ Wasserstoff oder $C_1$-$C_4$-Alkyl ist; wobei die jeweiligen aromatischen Reste unsubstituiert oder ein- oder mehrfach substituiert sind durch Substituenten ausgewählt aus der Gruppe bestehend aus Halogen, $C_1$-$C_4$-Alkyl, $C_1$-$C_4$-Alkoxy, Hydroxy, $C_2$-$C_6$-Alkanoyloxy, Trifluormethyl und Nitro.

Die Erfindung betrifft insbesondere Verbindungen der Formel I und ihre Salze, worin -$A_1$-$A_2$-, -$A_3$-$A_4$- und -$A_5$-$A_6$- die angegebenen Bedeutungen haben; X für $>$ C($R_6$)- oder $>$ N- steht und $R_6$ Wasserstoff ist; alk $C_1$-$C_4$-Alkylen, wie Methylen oder 2-Methyl-1,3-propylen, oder $C_3$-$C_5$-Alkenylen, wie 1,4-But-2-enylen, bedeutet, wobei die Doppelbindung in höherer als der α-Stellung zum Piperazinstickstoff lokalisiert ist; $R_1$ Wasserstoff oder verzweigtes $C_3$-$C_6$-Alkanoyl, wie Pivaloyl, bedeutet; $R_2$ $C_2$-$C_6$-Alkanoyl, wie Acetyl, Propionyl oder Pivaloyl, $C_1$-$C_4$-Alkoxy-carbonyl, wie Methoxy- oder Ethoxy-carbonyl, $C_1$-$C_4$-Alkansulfonyl, wie Methansulfonyl, oder gegebenenfalls durch $C_1$-$C_4$-Alkyl oder Halogen mit Atomnummer bis und mit 35 substituiertes Benzolsulfonyl, wie p-Bromphenyl- oder p-Toluol-sulfonyl, bedeutet, $R_3$ $C_2$-$C_6$-Alkanoyl, wie Acetyl, Propionyl oder Pivaloyl, $C_1$-$C_4$-Alkoxycarbonyl, wie Methoxy- oder Ethoxycarbonyl, $C_1$-$C_4$-Alkansulfonyl, wie Methansulfonyl, oder gegebenenfalls durch $C_1$-$C_4$-Alkyl oder Halogen mit Atomnummer bis und mit 35 substituiertes Benzolsulfonyl, wie p-Bromphenyl- oder p-Toluolsulfonyl, bedeutet; $R_3'$ Wasserstoff ist; $R_4$ Wasserstoff, $C_3$-$C_7$-Cycloalkyl, wie Cyclohexyl, gegebenenfalls durch $C_1$-$C_4$-Alkyl substituiertes Phenyl, Biphenylyl oder Naphthyl, wie 2,4,6-Trimethylphenyl, 4-Biphenylyl oder 2-Naphthyl, bedeutet; $R_5$ Acetyl ist; $R_7$ Wasserstoff ist.

Die Erfindung betrifft insbesondere Verbindungen der Formel I und ihre Salze, worin -$A_1$-$A_2$-, -$A_3$-$A_4$- und -$A_5$-$A_6$- die angegebenen Bedeutungen haben; X für $>$ C($R_6$)- oder $>$ N- steht und $R_6$ Wasserstoff ist; alk $C_1$-$C_4$-Alkylen, wie Methylen, 2-Methyl-1,3-propylen oder 3-Methyl-1,4-butylen, bedeutet; $R_1$ Wasserstoff oder verzweigtes $C_3$-$C_6$-Alkanoyl, insbesondere Pivaloyl, bedeutet; $R_2$ $C_2$-$C_6$-Alkanoyl, wie Acetyl, Propionyl oder Pivaloyl, Benzoyl oder $C_1$-$C_4$-Alkyl-sulfonyl, wie Methansulfonyl, bedeutet; $R_3$ Wasserstoff, $C_2$-$C_6$-Alkanoyl, wie Acetyl, Propionyl oder Pivaloyl, Benzoyl oder $C_1$-$C_4$-Alkansulfonyl, wie Methansulfonyl, bedeutet und $R_3'$ Wasserstoff ist; $R_4$ Wasserstoff, $C_3$-$C_7$-Cycloalkyl, wie Cyclopentyl oder -hexyl, gegebenenfalls durch $C_1$-$C_4$-Alkyl substituiertes Phenyl, insbesondere 2,4,6-Trimethylphenyl, bedeutet; $R_5$ Acetyl ist; $R_7$ Wasserstoff oder $C_1$-$C_4$-Alkyl, wie Methyl, bedeutet.

Die Erfindung betrifft insbesondere Verbindungen der Formel I und ihre Salze, worin die Strukturelemente -$A_1$-$A_2$-, -$A_3$-$A_4$- und -$A_5$-$A_6$- die angegebenen Bedeutungen haben; in erster Linie jeweils Ethylen bedeuten; X für $>$ N- steht; $R_1$ verzweigtes $C_3$-$C_6$-Alkanoyl, insbesondere Pivaloyl, bedeutet; $R_2$ $C_2$-$C_5$-Alkanoyl, wie Acetyl, bedeutet; $R_3$ $C_2$-$C_5$-Alkanoyl, wie Acetyl, Propionyl oder Pivaloyl, bedeutet; $R_3'$ Wasserstoff ist; $R_5$ Acetyl ist und einerseits alk $C_1$-$C_4$-Alkylen, in erster Linie 2-Methyl-1,3-propylen, bedeutet und $R_4$ Wasserstoff bedeutet oder andererseits alk Methylen ist und $R_4$ 2,4,6-Trimethylphenyl bedeutet und $R_7$ jeweils Wasserstoff ist.

Die Erfindung betrifft in erster Linie Verbindungen der Formel I und ihre Salze, worin die Strukturelemente -$A_1$-$A_2$-, -$A_3$-$A_4$- und -$A_5$-$A_6$- jeweils Ethylen bedeuten; X für $>$ N- steht; $R_1$ verzweigtes $C_3$-$C_6$-Alkanoyl, insbesondere Pivaloyl, bedeutet; $R_2$ und $R_3$ jeweils $C_2$-$C_5$-Alkanoyl, wie Acetyl, Propionyl oder Pivaloyl, bedeuten und $R_3'$ Wasserstoff ist; oder $R_2$ $C_2$-$C_5$-Alkanoyl, wie Acetyl, Propionyl oder Pivaloyl, bedeutet, $R_3$ Wasserstoff oder $C_2$-$C_5$-Alkanoyl, wie Acetyl, Propionyl oder Pivaloyl, bedeutet und $R_3'$ Wasserstoff ist oder $R_3$ und $R_3'$ gemeinsam für eine Bindung stehen; $R_5$ Acetyl ist; alk-$R_4$ Isobutyl, Cyclopentylmethyl, Cyclohexylmethyl, Benzyl, 2,4,6-Trimethylbenzyl, bedeutet; $R_7$ Wasserstoff oder $C_1$-$C_4$-Alkyl, wie Methyl, ist.

Die Erfindung betrifft in erster Linie Verbindungen der Formel I und ihre Salze, worin die Strukturelemente -$A_1$-$A_2$-, -$A_3$-$A_4$- und -$A_5$-$A_6$- jeweils Ethylen bedeuten; X für $>$ N- steht; $R_1$ verzweigtes $C_3$-$C_6$-Alkanoyl, insbesondere Pivaloyl, bedeutet; $R_2$ und $R_3$ jeweils $C_2$-$C_5$-Alkanoyl, wie Acetyl, Propionyl oder Pivaloyl, bedeuten und $R_3'$ Wasserstoff ist; $R_5$ Acetyl ist; alk-$R_4$ Isobutyl, Cyclopentylmethyl, Cyclohexylmethyl, Benzyl, 2,4,6-Trimethylbenzyl, bedeutet; $R_7$ Wasserstoff, ferner $C_1$-$C_4$-Alkyl, wie Methyl, ist.

Die Erfindung betrifft in erster Linie Verbindungen der Formel I und ihre Salze, worin die Strukturelemente -$A_1$-$A_2$-, -$A_3$-$A_4$- und -$A_5$-$A_6$- jeweils Ethylen bedeuten; X für $>$ N- steht; $R_1$ verzweigtes $C_3$-$C_6$-Alkanoyl, insbesondere Pivaloyl, bedeutet; $R_2$ $C_2$-$C_5$-Alkanoyl, wie Acetyl, Propionyl oder Pivaloyl, bedeutet; $R_3$ und $R_3'$ gemeinsam für eine Bindung stehen; $R_5$ Acetyl ist; alk-$R_4$ Isobutyl, Cyclohexylmethyl, Benzyl, 2,4,6-Trimethylbenzyl, Naphthylmethyl oder Biphenylylmethyl, wie 4-Biphenylylmethyl, bedeutet; $R_7$ Wasserstoff oder $C_1$-$C_4$-Alkyl, wie Methyl, ist.

Die Erfindung betrifft in erster Linie Verbindungen der Formel I und ihre Salze, worin die Strukturelemente -$A_1$-$A_2$-, -$A_3$-$A_4$- und -$A_5$-$A_6$- jeweils Ethylen bedeuten; X für $>$ N- steht; $R_1$ verzweigtes $C_3$-$C_6$-Alkanoyl, insbesondere Pivaloyl, bedeutet; $R_2$ $C_2$-$C_5$-Alkanoyl, wie Acetyl, Propionyl oder Pivaloyl, bedeutet; $R_3$ und $R_3'$ jeweils Wasserstoff sind; $R_5$ Acetyl ist; alk-$R_4$ Isobutyl, Cyclohexylmethyl, Benzyl, 2,4,6-Trimethylbenzyl, Naphthylmethyl oder Biphenylylmethyl, wie 4-Biphenylylmethyl, bedeutet; $R_7$ Wasserstoff ist.

Die Erfindung betrifft in erster Linie Verbindungen der Formel I und ihre Salze, worin die Strukturelemente $-A_1-A_2-$, $-A_3-A_4-$ und $-A_5-A_6-$ jeweils Ethylen bedeuten; X für $>$N- steht; alk $C_1$-$C_4$-Alkylen, insbesondere Methylen, bedeutet; $R_1$ verzweigtes $C_3$-$C_5$-Alkanoyl, insbesondere Pivaloyl, bedeutet; $R_2$ $C_2$-$C_5$-Alkanoyl, insbesondere Acetyl, Propionyl oder Pivaloyl, bedeutet; $R_3$ und $R_3'$ jeweils Wasserstoff sind; $R_4$ 2,4,6-Tri-$C_1$-$C_4$-alkylphenyl, insbesondere 2,4,6-Trimethylphenyl, bedeutet; $R_5$ Acetyl bedeutet; $R_7$ Wasserstoff ist.

Die Erfindung betrifft in erster Linie Verbindungen der Formel I und ihre Salze, worin die Strukturelemente $-A_1-A_2-$, $-A_3-A_4-$ und $-A_5-A_6-$ jeweils Ethylen bedeuten; X für $>$N-steht, alk $C_1$-$C_4$-Alkylen, insbesondere Methylen, bedeutet; $R_1$ verzweigtes $C_3$-$C_5$-Alkanoyl, insbesondere Pivaloyl, bedeutet; $R_2$ $C_2$-$C_5$-Alkanoyl, insbesondere Acetyl, Propionyl oder Pivaloyl, ist; $R_3$ Wasserstoff oder $C_2$-$C_5$-Alkanoyl, insbesondere Acetyl, Propionyl oder Pivaloyl, bedeutet; $R_3'$ Wasserstoff ist; alk-$R_4$ 2,4,6-Tri-$C_1$-$C_4$-alkylphenyl-$C_1$-$C_4$-alkyl, insbesondere 2,4,6-Trimethylphenylmethyl, bedeutet; $R_5$ Acetyl bedeutet; $R_7$ Wasserstoff, ferner Methyl oder Ethyl ist.

Die Erfindung betrifft in erster Linie Verbindungen der Formel I und ihre Salze, worin die Strukturelemente $-A_1-A_2-$, $-A_3-A_4-$ und $-A_5-A_6-$ jeweils Ethylen bedeuten; X für $>$N-steht, alk $C_1$-$C_4$-Alkylen, insbesondere Methylen, bedeutet; $R_1$ verzweigtes $C_3$-$C_5$-Alkanoyl, insbesondere Pivaloyl, bedeutet; $R_2$ $C_2$-$C_5$-Alkanoyl, insbesondere Acetyl, Propionyl oder Pivaloyl, ist; $R_3$ und $R_3'$ gemeinsam für eine Bindung stehen; alk-$R_4$ 2,4,6-Tri-$C_1$-$C_4$-alkylphenyl-$C_1$-$C_4$-alkyl, insbesondere 2,4,6-Trimethylphenylmethyl, bedeutet; $R_5$ Acetyl bedeutet; $R_7$ Wasserstoff, ferner Methyl oder Ethyl, ist.

Die Erfindung betrifft in erster Linie Verbindungen der Formel I und ihre Salze, worin die Strukturelemente $-A_1-A_2-$, $-A_3-A_4-$ und $-A_5-A_6-$ jeweils Ethylen bedeuten; X für $>$N-steht; alk $C_1$-$C_4$-Alkylen, insbesondere Methylen, bedeutet; $R_1$ verzweigtes $C_3$-$C_5$-Alkanoyl, in erster Linie Pivaloyl, bedeutet; $R_2$ $C_2$-$C_5$-Alkanoyl, insbesondere Acetyl, Propionyl oder Pivaloyl, ist; $R_3$ $C_2$-$C_5$-Alkanoyl, insbesondere Acetyl, Propionyl oder Pivaloyl, bedeutet; $R_3'$ Wasserstoff ist; alk-$R_4$ 2,4,6-Tri-$C_1$-$C_4$-alkylphenyl-$C_1$-$C_4$-alkyl, insbesondere 2,4,6-Trimethylphenylmethyl, bedeutet; $R_5$ Acetyl bedeutet; $R_7$ Wasserstoff ist.

Die Erfindung betrifft insbesondere die in den Beispielen genannten neuen Verbindungen und ihre Herstellung.

Ebenfalls Gegenstand der Erfindung sind Verfahren zur Herstellung der erfindungsgemässen Verbindungen. Die Herstellung von Verbindungen der Formel I und ihrer Salze erfolgt in an sich bekannter Weise und ist z.B. dadurch gekennzeichnet, dass man

a) zur Herstellung von Verbindungen der Formel I und ihrer Salze, worin X für $>$N- steht, eine Verbindung der Formel

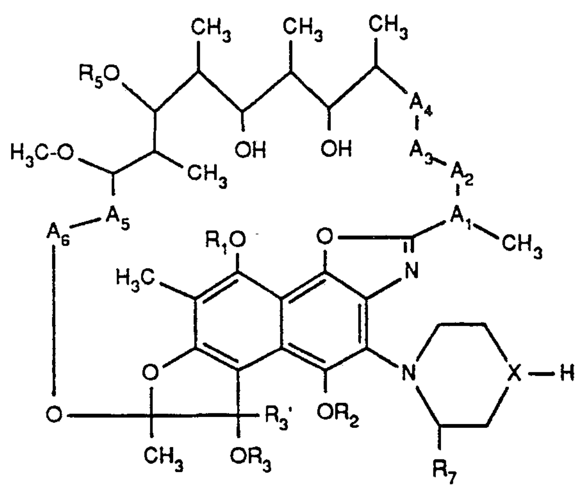

(IIa)

oder ein Salz davon, worin X für $>$N- steht, mit einer Verbindung der Formel

Z-alk-$R_4$ (IIb)

umsetzt, worin Z reaktionsfähiges verestertes Hydroxy bedeutet, oder

b) eine Verbindung der Formel

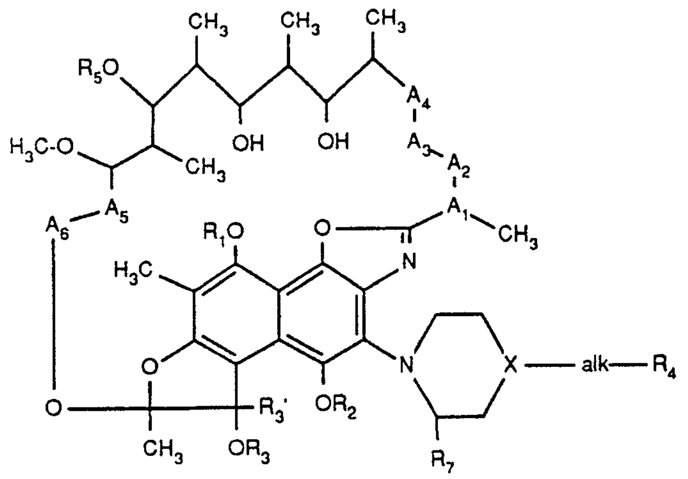

(III),

worin mindestens einer der Reste $R_1$,$R_2$ und/oder $R_3$ Wasserstoff bedeutet, acyliert, oder

c) eine Verbindung der Formel

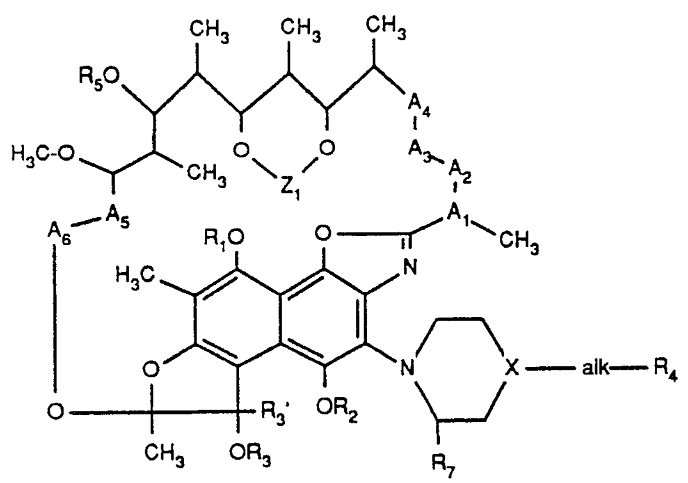

(IV),

worin $Z_1$ Alkyliden oder Cycloalkyliden bedeutet, hydrolysiert,

und gewünschtenfalls eine verfahrensgemäss oder auf andere Weise erhältliche Verbindung der Formel I oder ein Salz davon in eine andere erfindungsgemässe Verbindung oder ein Salz davon überführt, eine verfahrensgemäss erhältliche freie Verbindung der Formel I in ein Salz und/oder ein verfahrensgemäss erhältliches Salz in die freie Verbindung der Formel I oder in ein anderes Salz überführt und, wenn erwünscht ein verfahrensgemäss erhältliches Gemisch von Isomeren auftrennt.

Salze der Ausgangsmaterialien der Formeln IIa, III und IV, die eine acide phenolische Hydroxygruppe aufweisen, sind entsprechende Salze mit Basen der vorstehend aufgeführten Art, während Ausgangsverbindungen der Formel IIa, III oder IV, die ein oder zwei basische Zentren aufweisen, entsprechende Säureadditionssalze analog der Säureadditionssalze der Formel I bilden können.

Reaktionsfähiges verestertes Hydroxy, z.B. Z, ist insbesondere mit einer starken anorganischen Säure oder organischen Sulfonsäure verestertes Hydroxy, beispielsweise Halogen, wie Chlor, Brom oder Iod, Sulfonyloxy, wie Hydroxysulfonyloxy, Halogensulfonyloxy, z.B. Fluorsulfonyloxy, gegebenenfalls, z.B. durch Halogen, substituiertes $C_1$-$C_7$-Alkansulfonyloxy, z.B. Methan- oder Trifluormethansulfonyloxy, $C_5$-$C_7$-Cycloalkansulfonyloxy, z.B. Cyclohexansulfonyloxy, oder gegebenenfalls, z.B. durch $C_1$-$C_7$-Alkyl oder Halogen, substituiertes Benzolsulfonyloxy, z.B. p-Brombenzol- oder p-Toluolsulfonyloxy.

Alkyliden bedeutet beispielsweise $C_1$-$C_7$-Alkyliden, insbesondere $C_1$-$C_5$-Alkyliden, wie Methylen, Ethyliden, Isopropyliden, 1-Methyl-propyliden oder -butyliden, während Cycloalkyliden z.B. $C_3$-$C_7$-Cycloalkyliden, insbesondere Cyclopentyliden oder -hexyliden, bedeutet.

Die vor- und nachstehend in den Varianten beschriebenen Umsetzungen werden in an sich bekannter Weise durchgeführt, z.B. in Ab- oder üblicherweise in Anwesenheit eines geeigneten Lösungs- oder Verdünnungsmittels oder eines Gemisches derselben, wobei man je nach Bedarf unter Kühlen, bei

Raumtemperatur oder unter Erwärmen, z.B. in einem Temperaturbereich von etwa -80° bis zur Siedetemperatur des Reaktionsmediums, vorzugsweise von etwa -10° bis etwa +180° C, und, falls erforderlich, in einem geschlossenen Gefäss, unter Druck, in einer Inertgasatmosphäre und/oder unter wasserfreien Bedingungen arbeitet.

Variante a):

Z bedeutet vorzugsweise Halogen, wie Chlor, Brom oder Iod, ferner Sulfonyloxy, wie Methan- oder p-Toluolsulfonyloxy.

Die Umsetzung erfolgt in an sich bekannter Weise, vorteilhaft in Gegenwart einer Base.

Als Basen kommen vorzugsweise nicht nucleophile tertiäre Amine in Frage, beispielsweise Triniederalkylamine, basische Heterocyclen und carbocyclische Amine, wie Ethyl-diisopropylamin, Triethylamin, Pyridin, 1,5-Diaza- bicyclo[4.3.0]non-5-en (DBN) sowie 1,8-Diaza-bicyclo[5.4.0]undec-7-en (DBU).

Variante b):

Die Acylierung erfolgt in an sich bekannter Weise unter Verwendung eines geeigneten Acylierungsmittels. Als Acylierungsmittel kommt beispielsweise eine Verbindung der Formel Ac-$Z_2$ (IIIa) in Betracht, wobei Ac einen den Variablen $R_1$, $R_2$ bzw. $R_3$ entsprechenden Acylrest bedeutet und $Z_2$ Hydroxy oder insbesondere reaktionsfähig aktiviertes Hydroxy bedeutet. Entsprechendes Hydroxy kann beispielsweise durch starke Säuren, wie Halogenwasserstoff- oder Carbonsäure, z.B. durch Chlor-, Bromwasserstoffsäure, eine gegebenenfalls, z.B. durch Halogen, substituierte Alkancarbonsäure oder durch eine Säure der Formel Ac-OH, oder durch geeignete Aktivierungs- oder Kupplungsreagentien der nachstehend aufgeführten Art, insbesondere in situ, aktiviert sein. Ac-$Z_2$ kann ferner für einen aktivierten Ester stehen, wobei $Z_2$ insbesondere Cyanmethoxy, (4-)Nitrophenoxy oder Polyhalogen-, wie Pentachlor-, -phenoxy bedeutet. Als Aktivierungs- und Kupplungsreagentien können insbesondere Carbodiimide, z.B. N,N′-Di-$C_1$-$C_4$-alkyl- oder N,N′-Di-$C_5$-$C_7$-cycloalkyl-carbodiimid, wie Diisopropylcarbodiimid oder N,N′-Dicyclohexyl-carbodiimid, vorteilhaft unter Zusatz eines Aktivierungskatalysators, wie N-Hydroxysuccinimid oder gegebenenfalls, z.B. durch Halogen, $C_1$-$C_7$-Alkyl oder $C_1$-$C_7$-Alkoxy, substituiertes N-Hydroxy-benzotriazol oder N-Hydroxy-5-norbornen-2,3-dicarboxamid, ferner $C_1$-$C_4$-Alkylhalogenformiat, z.B. Isobutylchlorformiat, geeignete Carbonylverbindungen, z.B. N,N-Carbonyldiimidazol, geeignete 1,2-Oxazoliumverbindungen, z.B. 2-Ethyl-5-phenyl-1,2-oxazolium-3′-sulfonat oder 2-tert-Butyl-5-methyl-isoxazolium-perchlorat, geeignete Acylaminoverbindungen, z.B. 2-Ethoxy-1-ethoxycarbonyl- 1,2-dihydrochinolin, oder geeignete Phosphorylcyanamide bzw. -azide, z.B. Diethylphosphorylcyanamid oder Diphenylphosphorylazid, ferner Triphenylphosphin-disulfid oder 1-$C_1$-$C_4$-Alkyl-2-halogeno-pyridinium-halogenide, z.B. 1-Methyl-2-chlorpyridinium-iodid, eingesetzt werden.

$Z_2$ bedeutet bevorzugt Halogen, wie Chlor oder Brom, sowie Ac-O-.

Die erfindungsgemässe Acylierung wird vorzugsweise unter milden Bedingungen, beispielsweise bei Raumtemperatur oder leicht erhöhten Temperaturen, durchgeführt. Je nach Art des Ausgangsmaterials der Formel III ist die Menge an Acylierungsmittel so zu wählen, dass eine, zwei oder in zweiter Linie drei Acylgruppen eingeführt werden. Vorteilhaft kann das Acylierungsmittel als Lösungsmittel dienen. Der Reaktionsablauf wird zweckmässig mittels üblicher analytischer Methoden, insbesondere mit Dünnschichtchromatographie, verfolgt.

Falls die Acylierung mit einer Verbindung der Formel Ac-$Z_2$ durchgeführt wird, worin $Z_2$ Halogen bedeutet, wird vorteilhaft in Gegenwart eines säurebindenden Mittels, wie einer nichtacylierbaren Base, gearbeitet. Bei der Umsetzung mit einem Anhydrid, insbesondere einem symmetrischen Anhydrid, verwendet man insbesondere einen Ueberschuss an Ac-O-Ac.

In einer bevorzugten Ausführung des erfindungsgemässen Acylierungsverfahrens geht man von solchen Verbindungen der Formel III aus, worin $R_1$ Acyl ist, $R_5$ Acetyl bedeutet und $R_2$ und $R_3$ Wasserstoff bedeuten. Je nach Wahl der Reaktionsbedingungen kann man die Acylierung so steuern, dass lediglich das Sauerstoffatom an C-4 des Rifamycin-Ringsystems acyliert wird. Dabei acyliert man insbesondere bei Raumtemperatur mit dem jeweiligen Acylierungsmittel, das gleichzeitig als Lösungsmittel fungiert. Erhöht man die Temperatur bzw. setzt man beispielsweise eine Verbindung der Formel IIIa unter Verwendung einer Base ein, worin $Z_2$ Halogen bedeutet, wird insbesondere das O-Atom an C-4 sowie C-11 gleichzeitig acyliert.

Bedeutet $R_2$ Alkanoyl und $R_3$ Wasserstoff, kann das entsprechende 4-O-Acyl-Derivat im Gleichgewicht

mit dem entsprechenden 11-O-Acyl-Derivat stehen, die 4-O-Acyl-gruppe wandert also an die 11-Hydroxygruppe.

Zur Herstellung des Ausgangsmaterials der Formel III geht man beispielsweise von Rifamycin S oder SV aus und führt in an sich bekannter Weise, beispielsweise wie in WO 87/02361 beschrieben, in Position 3 die Seitenkette

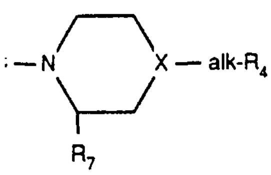

ein. Anschliessend wird das entsprechende SV-Derivat mittels eines Acylierungsmittels, wie Pivaloylchlorid, in die entsprechende 1,8-Di-O-acyl-Verbindung und danach durch längeres Erwärmen, z.B. auf 100 °C, in das entsprechende 8-O-Acyl-1-desoxy-15-desoxo-1,15-oxy-Derivat der Formel III übergeführt. Durch Hydrierung können in an sich bekannter Weise, z.B. wie nachstehend beschrieben, entsprechende Hydro-Verbindungen hergestellt werden.

Variante c):

$Z_1$ steht in erster Linie für $C_1$-$C_5$-Alkyliden, wie Isopropyliden. Die Hydrolyse des Ketals der Formel IV erfolgt in an sich bekannter Weise, vorteilhaft unter Verwendung einer Säure, wie einer anorganischen oder organischen Säure, wie Mineralsäure, z.B. eine Halogenwasserstoffsäure, Schwefelsäure oder eine Phosphorsäure, Sulfonsäure, z.B. Methan- oder p-Toluolsulfonsäure, oder eine Carbonsäure, z.B. Essigsäure..

Die Herstellung von Verbindungen der Formel IV erfolgt in an sich bekannter Weise. So geht man beispielsweise von einer Verbindung der Formel I, worin $R_2$ Wasserstoff ist und $R_3$ und $R_3'$ gemeinsam für eine Bindung stehen oder jeweils Wasserstoff bedeuten, aus und setzt diese in Gegenwart einer der aufgeführten Säuren mit einem $Z_1$ entsprechenden Aldehyd oder Keton bzw. einem Ketal davon, wie Di-$C_1$-$C_4$-alkyl- oder $C_2$-$C_4$-Alkylen-ketal, z.B. Acetondimethylketal, um. Verbindungen der Formel I wiederum, worin $-A_1$-$A_2$-, $-A_3$-$A_4$- und $-A_5$-$A_6$- jeweils Vinylen bedeuten und $R_3$ und $R_3'$ gemeinsam für eine Bindung stehen, sind beispielsweise in EP 314624 beschrieben. Zu entsprechenden Hydroderivaten ($-A_1$-$A_2$-, $-A_3$-$A_4$- und $-A_5$-$A_6$- z.B. bedeuten Ethylen) kann man beispielsweise unter Anwendung der nachstehend aufgeführten Hydrierungsverfahren gelangen.

Die Erfindung betrifft ebenfalls die nach den vorstehenden Verfahrensvarianten erhältlichen neuen Verbindungen.

Eine erfindungsgemäss oder auf andere Weise erhältliche Verbindung der Formel I oder Salz davon kann in an sich bekannter Weise in eine andere Verbindung der Formel übergeführt werden.

Verbindungen der Formel I, worin die Strukturelemente $-A_1$-$A_2$-, $A_3$-$A_4$- und $-A_5$-$A_6$-jeweils Vinylen oder $-A_1$-$A_2$- und $-A_3$-$A_4$- jeweils Ethylen und $-A_5$-$A_6$- Vinylen bedeuten, können durch Reduktion, z.B. durch katalytische Hydrierung, in die entsprechenden Tetrahydro- ($-A_1$-$A_2$- und $-A_3$-$A_4$- sind jeweils Ethylen und $-A_5$-$A_6$- ist Vinylen) oder die entsprechenden Hexahydroderivate ($-A_1$-$A_2$-, $A_3$-$A_4$- und $-A_5$-$A_6$- bedeuten jeweils Ethylen) übergeführt werden. So erfolgt die Hydrierung der Mehrfachbindungen in Gegenwart von Hydrierungskatalysatoren, wobei hierfür z.B. Edelmetalle bzw. deren Derivate, z.B. Oxide, geeignet sind, wie Nickel, Raney-Nickel, Palladium, Platinoxid, die gegebenenfalls auf Trägermaterialien, z.B. auf Kohle oder Calciumcarbonat, aufgezogen sein können. Die Hydrierung kann insbesondere bei Drucken von 1 bis etwa 100 at durchgeführt werden.

Verbindungen der Formel I, worin z.B. einer der Reste $R_1$, $R_2$ und $R_3$ Acyl bedeutet und mindestens einer der anderen Reste Wasserstoff ist, können in an sich bekannter Weise acyliert werden, beispielsweise analog der in Variante b) geschilderten Methodik, z.B. durch Umsetzung mit der entsprechenden Carbonsäure oder einem reaktionsfähigen Derivat davon. Derartige reaktionsfähige Derivate sind beispielsweise Anhydride, inklusive gemischte Anhydride, wie ein Säurehalogenid, z.B. -chlorid, oder Anhydride mit einem Ameisensäureester, aktivierte Carbonsäureester, wie Cyanmethyl-, (4-)Nitrophenyl-, Polyhalogenphenyl-, z.B. Pentachlorphenyl-, -ester. Die Umsetzung mit der Carbonsäure oder einem Salz davon erfolgt unter wasserabspaltenden Bedingungen, z.B. unter azeotroper Entfernung des Reaktionswassers, oder durch Behandeln mit einem geeigneten Kondensationsmittel, z.B. N,N'-Dicyclohexyl-carbodiimid. Die Umsetzung

mit einem reaktionsfähigen Säurederivat wird vorteilhaft in Gegenwart einer Base durchgeführt. Entsprechend kann durch Behandeln mit einem entsprechenden Acetylierungsmittel der Acetylrest $R_5$ in Verbindungen der Formel I, worin $R_5$ Wasserstoff ist, eingeführt werden.

Durch Behandeln mit starken Basen, wie Alkalimetallhydroxiden, können der Acetylrest $R_5$ und der Acylrest $R_1$, $R_2$ und/oder $R_3$ durch Wasserstoff ersetzt werden. Der Acylrest $R_1$ kann auch in Gegenwart des Acetylrests $R_5$ selektiv abgespalten werden, beispielsweise durch Behandeln mit einem Fluorid, wie Alkalimetall-, z.B. Natrium- oder Cäsiumfluorid, oder einem Ammoniumfluorid, z.B. Tetrabutylammoniumfluorid.

Salze von Verbindungen der Formel (I) können in an sich bekannter Weise hergestellt werden. So erhält man beispielsweise Säureadditionssalze von Verbindungen der Formel (I) durch Behandeln mit einer Säure oder einem geeigneten Ionenaustauscherreagenz. Salze können in üblicher Weise in die freien Verbindungen überführt werden, Säureadditionssalze z.B. durch Behandeln mit einem geeigneten basischen Mittel.

Je nach Verfahrensweise bzw. Reaktionsbedingungen können die erfindungsgemässen Verbindungen mit salzbildenden, insbesondere basischen Eigenschaften, in freier Form oder bevorzugt in Form von Salzen erhalten werden.

Infolge der engen Beziehung zwischen der neuen Verbindung in freier Form und in Form ihrer Salze sind im Vorausgegangenen und nachfolgend unter der freien Verbindung oder ihren Salzen sinn- und zweckgemäss gegebenenfalls auch die entsprechenden Salze bzw. die freie Verbindung zu verstehen.

Die neuen Verbindungen einschliesslich ihrer Salze von salzbildenden Verbindungen können auch in Form ihrer Hydrate erhalten werden oder andere zur Kristallisation verwendete Lösungsmittel einschliessen.

Die neuen Verbindungen können, je nach der Wahl der Ausgangsstoffe und Arbeitsweisen, in Form eines der möglichen Isomeren oder als Gemische derselben, z.B. je nach der Anzahl der asymmetrischen Kohlenstoffatome, als reine optische Isomere, wie Antipoden, oder als Isomerengemische, wie Racemate, Diastereoisomerengemische oder Racematgemische, vorliegen.

Erhaltene Racematgemische können auf Grund der physikalisch-chemischen Unterschiede der Bestandteile in bekannter Weise in die reinen Isomeren oder Racemate getrennt aufgetrennt werden, beispielsweise durch fraktio- nierte Kristallisation. Erhaltene Racemate lassen sich ferner nach bekannten Methoden in die optischen Antipoden zerlegen, beispielsweise durch Umkristallisation aus einem optisch aktiven Lösungsmittel, Chromatographie an chiralen Adsorbentien, mit Hilfe von geeigneten Mikroorganismen, durch Spaltung mit spezifischen, immobilisierten Enzymen, über die Bildung von Einschlussverbindungen, z.B. unter Verwendung chiraler Kronenether, wobei nur ein Enantiomeres komplexiert wird, oder durch Ueberführung in diastereomere Salze, z.B. durch Umsetzung eines basischen Endstoffracemats mit einer optisch aktiven Säure, wie Carbonsäure, z.B. Wein- oder Aepfelsäure, oder Sulfonsäure, z.B. Camphersulfonsäure, und Trennung des auf diese Weise erhaltenen Diastereomeren- gemisches, z.B. auf Grund ihrer verschiedenen Löslichkeiten, in die Diastereomeren, aus denen das gewünschte Enantiomere durch Einwirkung geeigneter Mittel freigesetzt werden kann. Vorteilhaft isoliert man das wirksamere Enantiomere.

Die Aufarbeitung des Reaktionsproduktes aus dem verfahrensgemäss erhältlichen Reaktionsgemisch erfolgt in an sich bekannter Weise, z.B. durch Verdünnen mit Wasser, und/oder gegebenenfalls durch Neutralisieren oder leichtes Ansäuern (bis etwa pH = 3) mit einer wässrigen Säure, wie einer anorganischen oder organischen Säure, z.B. einer Mineralsäure oder, vorteilhafterweise, Zitronensäure, und Zugabe eines mit Wasser nichtmischbaren Lösungsmittels, wie eines chlorierten Kohlenwasserstoffs, z.B. Chloroform oder Methylenchlorid, wobei das Reaktionsprodukt in die organische Phase übergeht, aus welcher es in üblicher Weise, z.B. durch Trocknen, Eindampfen des Lösungsmittels und Kristallisation und/oder Chromatographie des Rückstandes oder andere übliche Reinigungsmethoden in gereinigter Form erhalten werden kann. Ergibt die obige Reaktion beispielsweise ein Gemisch von acylierten Verbindungen, kann dieses in an sich bekannter Weise, z.B. mittels fraktionierter Kristallisation, Chromatographie, etc. in die gewünschten individuellen Acyl-Verbindungen aufgetrennt werden.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Derivates bzw. Salzes und/oder seiner Racemate bzw. Antipoden verwendet oder insbeson- dere unter den Reaktionsbedingungen bildet.

Beim Verfahren der vorliegenden Erfindung werden vorzugsweise solche Ausgangsstoffe verwendet, welche zu den eingangs als besonders wertvoll geschilderten Verbindungen führen. Neue Ausgangsstoffe, die speziell für die Herstellung der erfindungsgemässen Verbindungen entwickelt wurden, insbesondere neue Verbindungen der Formel III, ihre Verwendung und Verfahren zu ihrer Herstellung bilden ebenfalls einen Gegenstand der Erfindung, wobei die Variablen $A_1$-$A_2$, $A_3$-$A_4$, $A_5$-$A_6$, $R_1$, $R_2$, $R_3$, $R_3'$, $R_4$, $R_5$, $R_6$, $R_7$,

X und alk die für die jeweils bevorzugten Verbindungsgruppen der Formel I angegebenen Bedeutungen haben.

Die vorliegende Erfindung umfasst auch die Verwendung der Verbindungen der Formel I und ihrer Salze allein oder zusammen mit Hilfsstoffen, sowie auch in Kombination mit anderen Wirkstoffen, als Mittel zur therapeutischen, und zwar sowohl kurativen wie auch präventiven Behandlung von Krankheiten oder krankhaften Zuständen, die z.B. durch einen erhöhten Gehalt an Cholesterin und/oder Triglyceriden im Blut, insbesondere im Blutserum, angezeigt oder verursacht werden. Die erfindungsgemässen Wirkstoffe werden in therapeutisch wirksamen Mengen, vorzugsweise in Form von pharmazeutischen Zusammensetzungen zusammen mit konventionellen pharmazeutischen Trägermaterialien und/oder Hilfsstoffen an den behandlungsbedürftigen Warmblüter, in erster Linie Menschen, verabreicht. Dabei werden z.B. an Warmblüter je nach Spezies, Körpergewicht, Alter und individuellem Zustand, tägliche Dosen entsprechend etwa 1 bis etwa 100, insbesondere etwa 3 bis etwa 50 mg pro kg Körpergewicht, die in schweren Fällen überschritten werden können, verabreicht. Sinngemäss umfasst die Erfindung auch die entsprechende Methode zur medizinischen Behandlung.

Die Erfindung betrifft gleichfalls pharmazeutische Präparate, die die erfindungsgemässen Verbindungen oder pharmazeutisch verwendbare Salze derselben als Wirkstoffe enthalten, sowie Verfahren zu ihrer Herstellung.

Bei den erfindungsgemässen pharmazeutischen Präparaten, welche die erfindungsgemässe Verbindung oder pharmazeutisch verwendbare Salze davon enthalten, handelt es sich um solche zur enteralen, wie oralen ferner rektalen, und parenteralen Verabreichung an Warmblüter(n), wobei der pharmakologische Wirkstoff allein oder zusammen mit einem pharmazeutisch anwendbaren Trägermaterial enthalten ist. Die tägliche Dosierung des Wirkstoffs hängt von dem Alter und dem individuellen Zustand sowie von der Applikationsweise ab.

Die neuen pharmazeutischen Präparate enthalten z.B. von etwa 10 % bis etwa 80 %, vorzugsweise von etwa 20 % bis etwa 60 %, des Wirkstoffs. Erfindungsgemässe pharmazeutische Präparate zur enteralen bzw. parenteralen Verabreichung sind z.B. solche in Dosiseinheitsformen, wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister, unter Verwendung z.B. von Mais-, Weizen-, Reis- oder Kartoffelstärke, Gelatine, Tragakanth, Methylcellu- lose und/oder Polyvinylpyrrolidon, wenn erwünscht, Sprengmittel, wie die obengenannten Stärken, ferner Carboxmethylstärke, quervernetzes Polyvinylpyrrolidon, Agar, Alginsäure oder ein Salz davon, wie Natriumalginat, Hilfsmittel sind in erster Linie Fliess-, Fliessregulier- und Schmiermittel, z.B. Kieselsäure, Talk, Stearinsäure oder Salze davon, wie Magnesium- oder Calciumstearat, und/oder Polyethylenglykol. Dragée-Kerne werden mit geeigneten, gegebenenfalls Magensaft-resistenten Ueberzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemische oder, zur Herstellung von Magensaft-resistenten Ueberzügen, Lösungen von geeigneten Cellulosepräparaten, wie Acetylcellulosephthalat oder Hydroxypropylmethylcellulose- phthalat, verwendet. Den Tabletten oder Dragée-Ueberzügen können Farb- stoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls Stabilisatoren, enthalten. In weichen Kapseln ist der Wirkstoff vorzugsweise in geeigneten Flüssigkeiten, wie fetten Oelen, Paraffinöl oder flüssigen Polyethylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositorienmasse eignen sich z.B. natürliche oder synthetische Triglyceride, Paraffinkohlenwasserstoffe, Polyethylenglykole oder höhere Alkanole. Ferner können auch Gelatine- Rektalkapseln verwendet werden, die eine Kombination des Wirkstoffs mit einem Grundmassenstoff enthalten. Als Grundmassenstoffe kommen z.B. flüssige Triglyceride, Polyethylenglykole oder Paraffinkohlenwasser- stoffe in Frage.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z.B. Sesamöl, oder synthetische Fettsäureester, z.B. Ethyloleat oder Triglyceride, verwendet oder wässrige Injektionssuspensionen, welche viskositätserhöhende Stoffe, z.B. Natriumcarboxymethylcellulose, Sorbit und/oder Dextran, und gegebenenfalls auch Stabilisatoren enthalten.

Die Dosierung des Wirkstoffs hängt von der Warmblüter-Spezis, dem Alter und dem individuellen Zustand sowie der Applikationsweise ab. Im Normalfall ist für einen etwa 75 kg schweren Warmblüter bei oraler Applikation eine ungefähre Tagesdosis von etwa 150 mg bis etwa 1500 mg, vorteilhaft in mehreren gleichen Teildosen, zu veranschlagen.

Die nachfolgenden Beispiele illustrieren die oben beschriebene Erfindung; sie sollen jedoch diese in ihrem Umfang in keiner Weise einschränken. Temperaturen sind in Celsiusgraden angegeben.

Die chemische Bezeichnung der Grundgerüste, von denen sich die Rifamycin-Derivate der vorliegenden Erfindung ableiten, lautet wie folgt:
1-Desoxy-15-desoxo- 1,15-oxy-rifamycin (A) [$R_3$ und $R_3'$ bedeuten eine gemeinsame Bindung] und
11-Hydroxy-11,15-desoxo-1-desoxy-1,15-oxy-rifamycin (B) [$R_3$ und $R_3'$ bedeuten jeweils Wasserstoff] lassen sich folgendermassen darstellen:

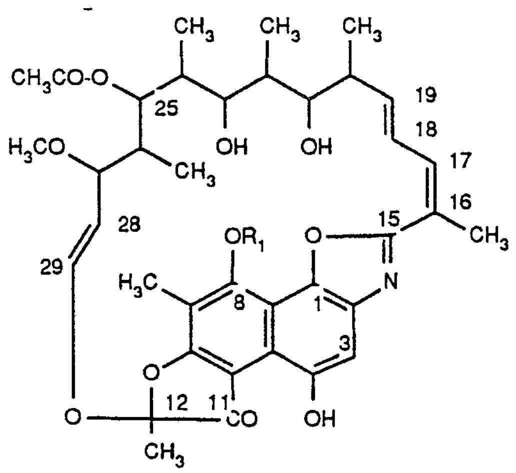

(A)

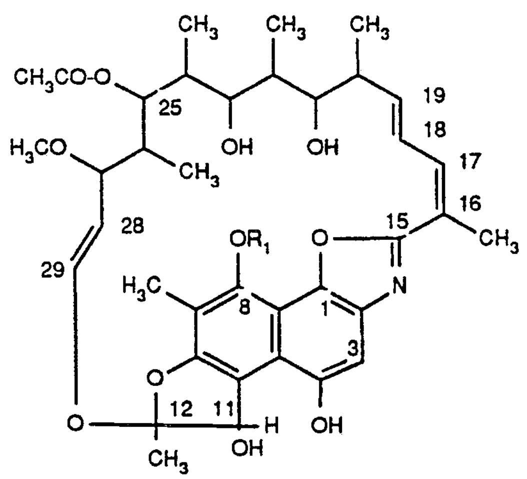

(B)

Beispiel 1:

2 g 16,17,18,19,28,29-Hexahydro-8-O-pivaloyl-11-hydroxy- 11,15-desoxo-1-desoxy-1,15-oxy-3-[4-(2,4,6-trimethylbenzyl)-piperazin-1-yl]-rifamycin werden in 25 ml Essigsäureanhydrid gelöst und gerührt. Wenn dünnschichtchromatographisch (an Kieselgel; Hexan/Essigsäureethylester 2:1) kein Ausgangsmaterial mehr nachweisbar ist, wird das überschüssige Essigsäureanhydrid mit Methanol- und Wasserzusatz zersetzt und das Reaktionsprodukt mit Essigsäureethylester aufgenommen. Nach dem Trocknen über $Na_2SO_4$ und Eindampfen des Essigesterextraktes bleibt ein farbloser Rückstand, der aus Diethylether/Pentan und Pentan kristallisiert. Man erhält so das 16,17,18,19,28,29-Hexahydro-4-O-acetyl-8-O-pivaloyl-11-hydroxy-11,15-desoxo-1-desoxy-1,15-oxy-3-[4-(2,4,6-trimethylbenzyl)-piperazin-1-yl]-rifamycin der Formel I, worin die Strukturelemente $-A_1-A_2-$, $-A_3-A_4-$ und $-A_5-A_6-$ jeweils Ethylen bedeuten, X für >N- steht, alk Methylen bedeutet, $R_1$ Pivaloyl bedeutet, $R_2$ Acetyl bedeutet, $R_3$, $R_3'$ und $R_7$ jeweils Wasserstoff bedeuten, $R_4$ 2,4,6-Trimethylphenyl bedeutet und $R_5$ Acetyl ist, in farblosen Kristallen, die bei 205-208° unter Zersetzung schmelzen. $C_{58}H_{83}N_3O_{13}$; MG: 1029 (gefunden: MS, FAB)

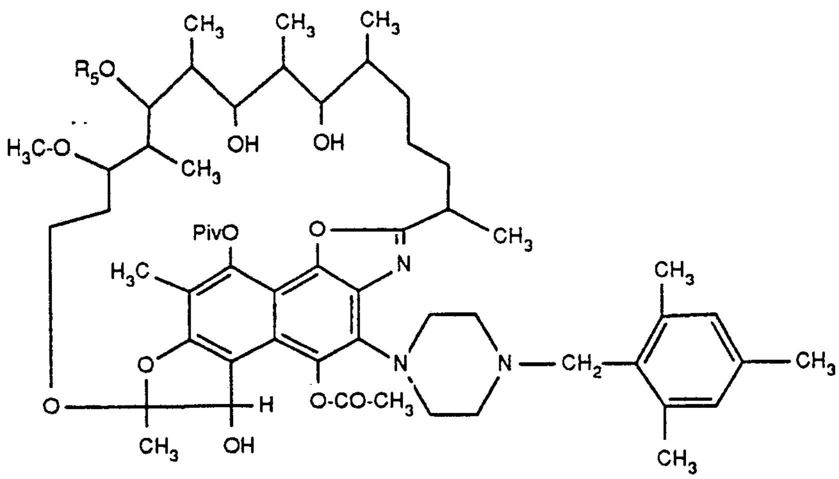

Das Ausgangsmaterial kann wie folgt hergestellt werden:

a) Ein Gemisch aus 5 g 3-[4-(2,4,6-Trimethylbenzyl)-piperazin-1-yl]-rifamycin SV [WO 87/02361], 50 ml trockenem Pyridin und 4,5 ml Pivaloylchlorid wird während 30 Minuten auf 50° gehalten. Anschliessend verdampft man das Lösungsmittel im Vakuum. Der ölige Rückstand wird in Essigester gelöst und mit 2N-Salzsäure, mit Pufferlösung pH = 7 und mit Kochsalzlösung gewaschen. Nach dem Trocknen über Natriumsulfat und Eindampfen wird der gelbe Rückstand aus Ether/Hexan kristallisiert. Man erhält so das 1,8-Di-O-pivaloyl-3-[4-(2,4,6-trimethylbenzyl)-piperazin-1-yl]-rifamycin SV vom Schmelzpunkt 203-204°. $C_{61}H_{83}N_3O_{16}$; MG: 1081 (gefunden, MS).

b) 30 g 1,8-Di-O-pivaloyl-3-[4-(2,4,6-trimethylbenzyl)-piperazin-1-yl]- rifamycin SV werden in der Wärme in 1000 ml 2-Methoxyethanol gelöst und unter Stickstoff während 5 Stunden unter Rückfluss gekocht. Dann dampft man das Lösungsmittel im Vakuum ein und kristallisiert den Rückstand zweimal aus Methanol. Man erhält das 8-O-Pivaloyl-1-desoxy-15-desoxo-1,15-oxy-3-[4-(2,4,6-trimethylbenzyl)-piperazin-1-yl]-rifamycin der Formel I, worin die Strukturelemente $-A_1-A_2-$, $A_3-A_4-$ und $-A_5-A_6-$ jeweils Vinylen bedeuten, X für >N- steht, alk Methylen bedeutet, $R_1$ Pivaloyl bedeutet, $R_2$ und $R_7$ jeweils Wasserstoff bedeuten, $R_3$ und $R_3'$ gemeinsam für eine Bindung stehen, $R_4$ 2,4,6-Trimethylphenyl bedeutet und $R_5$ Acetyl ist. Schmelzpunkt 160-165°. $C_{56}H_{73}N_3O_{12}$; M = 979, gefunden (MS): 979; $^1$H-NMR (360 MHz, $CDCl_3$, TMS): 1,49 (s, 9H, Pivaloyl an O-8).

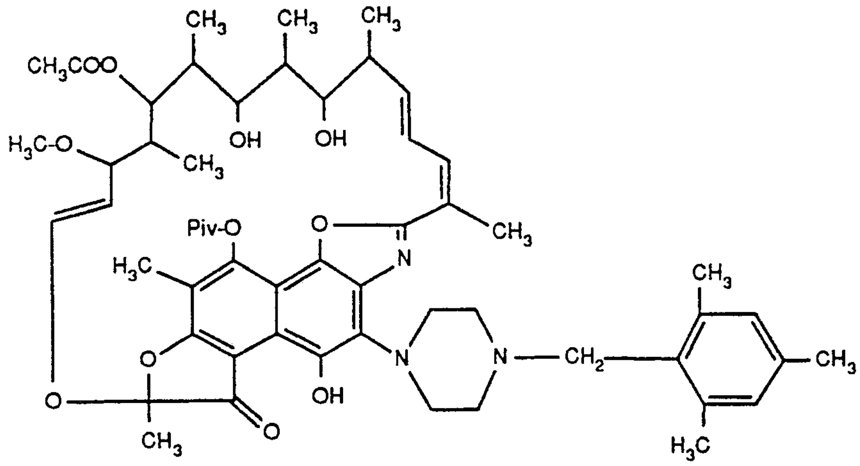

c) 5 g des Reaktionsprodukts von b) werden in 500 ml Ethanol gelöst und in Gegenwart von 0,5 g $PtO_2$ und 0,7 g Schwefelsäure bei Raumtemperatur und Normaldruck bis zur Aufnahme von 4 Aequivalenten Wasserstoff (~ 460 ml) hydriert. Man entfernt den Katalysator durch Filtration, neutralisiert das Filtrat mittels wässriger Natriumbicarbonatlösung, setzt gesättigte Kochsalzlösung zu und extrahiert das Hydrierungsprodukt durch wiederholtes Ausschütteln mit Essigester. Nach dem Trocknen und Eindampfen des Essigesterextrakts wird der Rückstand in Ether gelöst. Nach einigem Stehen kristallisiert das 16,17,18,19,28,29-Hexahydro-8-O-pivaloyl-11-hydroxy-11,15-desoxo-1-desoxy-1,15-oxy-3-[4-(2,4,6-trimethylbenzyl)-piperazin-1-yl]-rifamycin der Formel I, worin die Strukturelemente $-A_1-A_2-$, $A_3-A_4-$ und $-A_5-A_6-$ jeweils Ethylen bedeuten, X für $\geq$ N- steht, alk Methylen bedeutet, $R_1$ Pivaloyl bedeutet, $R_2$, $R_3$,$R_3'$ und $R_7$ jeweils Wasserstoff bedeuten, $R_4$ 2,4,6-Trimethylphenyl bedeutet und $R_5$ Acetyl ist. Die schwach braunroten Kristalle, die nach Umkristallisieren aus Ether farblose Plättchen bilden, schmelzen bei 226-227° (Zersetzung). $C_{56}H_{81}N_3O_{12}$; MG: 987 (gefunden; MS,FD).

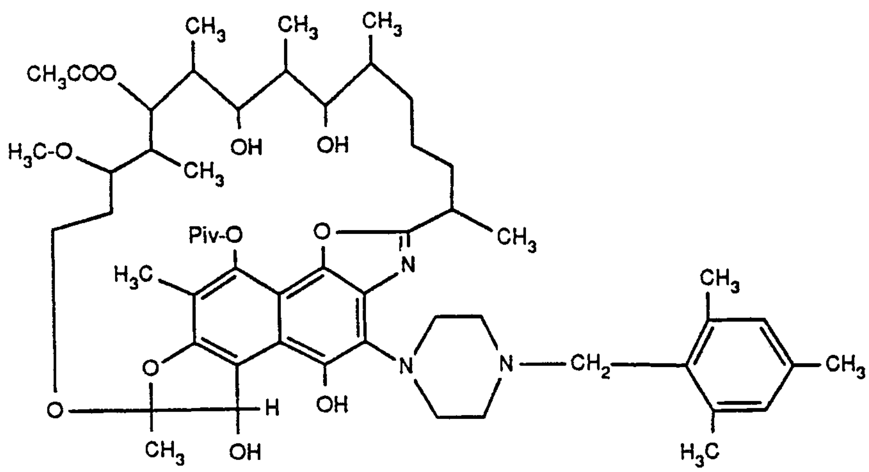

Beispiel 2:

In analoger Weise, z.B. wie in Beispiel 1 beschrieben, erhält man ausgehend von 2 g 16,17,18,19,28,29-Hexahydro-11-hydroxy-1-desoxy-11,15-desoxo-1,15-oxy-3-[4-(2,4,6-trimethylbenzyl)-piperazin-1-yl]-rifamycin und Propionsäureanhydrid das 16,17,18,19,28,29-Hexahydro-4-O-propionyl-8-O-pivaloyl-11-hydroxy-1-desoxy-11,15-desoxo-1,15-oxy-3-[4-(2,4,6-trimethylbenzyl)-piperazin-1-yl]-rifamycin der Formel I, worin die Strukturelemente $-A_1-A_2-$, $-A_3-A_4-$ und $-A_5-A_6-$ jeweils Ethylen bedeuten, X für $\geq$ N- steht, alk Methylen bedeutet, $R_1$ Pivaloyl bedeutet, $R_2$ Propionyl bedeutet, $R_3$, $R_3'$ und $R_7$ jeweils Wasserstoff bedeuten, $R_4$ 2,4,6-Trimethylphenyl bedeutet und $R_5$ Acetyl ist. Es bildet aus Hexan plattenartige, farblose Kristalle, die bei 152° schmelzen. $C_{59}H_{85}N_3O_{13}$; MG: 1043 (gefunden: MS, FAB und DCI);

$^1$H-NMR (360 MHz, DMSO-$d_6$, 80°): 5,45 (d, 1H, H-11); 1,25 (t) und 2,73 (q -Propionyl).

Beispiel 3:

Eine Lösung von 3 g 16,17,18,19,28,29-Hexahydro-11-hydroxy- 1-desoxy-11,15-desoxo-1,15-oxy-3-[4-(2,4,6-trimethylbenzyl)-piperazin-1-yl]-rifamycin in 50 ml Pivalinsäureanhydrid wird solange auf 100° erhitzt, bis alles Ausgangsmaterial umgesetzt ist. Die Kontrolle erfolgt durch Dünnschichtchromatographie an Kieselgel mit dem Elutionsmittel Essigsäureethylester/Hexan (1:2). Das Reaktionsgemisch wird im Hochvakuum eingedampft und der farblose Rückstand in Pentan gelöst. Nach Entfernen geringer Mengen unlöslichen Materials kristallisiert das 16,17,18,19,28,29-Hexahydro-4-O,8-O-di-pivaloyl-11-hydroxy-1-desoxy-11,15-desoxo-1,15oxy-3-[4-(2,4,6-trimethyl benzyl)-piperazin-1-yl]-rifamycin der Formel I, worin die Strukturelemente -$A_1$-$A_2$-, $A_3$-$A_4$- und $A_5$-$A_6$ jeweils Ethylen bedeuten, X für $>$ N- steht, alk Methylen bedeutet, $R_1$ und $R_2$ Pivaloyl bedeuten, $R_3$, $R_3'$ und $R_7$ Wasserstoff bedeuten, $R_4$ 2,4,6-Trimethylphenyl bedeutet und $R_5$ Acetyl ist, in farblosen, sechseckigen Kristallplatten vom Smp. 178-179°. $C_{61}H_{89}N_3O_{13}$; MG; 1071 (gefunden: MS, DCI):

Beispiel 4:

0,8 g (0,8 Mol) von 16,17,18,19,28,29-Hexahydro-8-O-pivaloyl-11-hydroxy-1-desoxy-11,15-desoxo-1,15-oxy-3-(4-isobutyl-1-piperazinyl)-rifamycin werden in 5 ml Acetanhydrid gelöst und in Stickstoffatmosphäre eine Stunde auf 50° C erwärmt. Es wird nochmals zwei Stunden bei Raumtemperatur gerührt. Ueberschüssiges Acetanhydrid wird im Vakuum entfernt und der Rückstand durch Blitzchromatographie (Methylenchlorid/Methanol: 95/5) gereinigt. Das erhaltene rötliche Oel wird aus Methanol/Wasser kristallisiert. Man erhält so das 16,17,18,19,28,29-Hexahydro-4-O-acetyl-8-O-pivaloyl-11-acetyloxy-1-desoxy-11,15-desoxo-1,15-oxy-3-(4-isobutyl-1-piperazinyl)-rifamycin der Formel I, worin -$A_1$-$A_2$-, -$A_3$-$A_4$- und -$A_5$-$A_6$- jeweils Ethylen bedeuten, X für $>$ N- steht, $R_1$ Pivaloyl bedeutet, $R_2$, $R_3$ und $R_5$ jeweils Acetyl bedeuten, alk 2-Methyl-1,3-propylen bedeutet und $R_3'$, $R_4$ und $R_7$ jeweils Wasserstoff bedeuten.

Schmelzpunkt: 142-143°.

NMR:
HO-(21 oder 23): 4,25 ppm;
HO-(23 oder 21): 3,80 ppm.
Me-($OOCCH_3$ bei 4): 2,2 ppm,s
Me-(14): 2,1 ppm, s Me-($COCH_3$): 2,08 ppm,s
IR: 1643 $cm^{-1}$ ($COCH_3$)
1735 $cm^{-1}$ ($OOCCH_3$ bei 25)
1760 $cm^{-1}$ ($OOCCH_3$ bei 4)
MS:996(M+1), 100

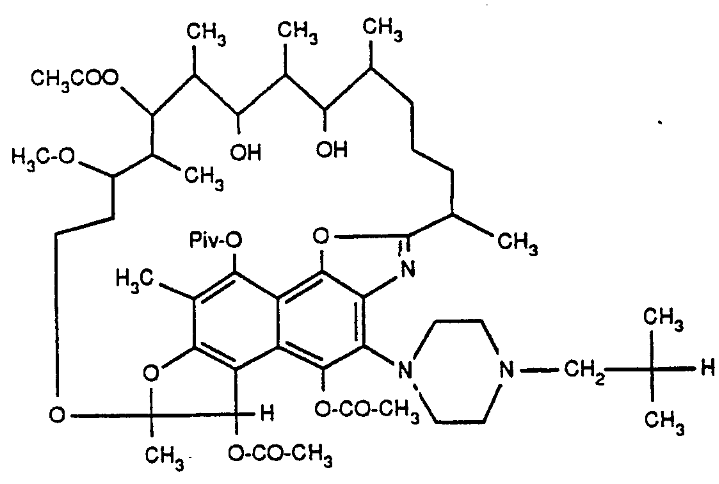

Das Ausgangsmaterial kann wie folgt hergestellt werden:

a) In analoger Weise wie in Beispiel 1 b) beschrieben erhält man aus 1,8-Di-O-pivaloyl-3-(4-isobutyl-1-piperazinyl)-rifamycin SV das 8-O-Pivaloyl- 1-desoxy-15-desoxo-1,15-oxy-3-(4-isobutyl-1-piperazinyl)-rifamycin, entsprechend der Formel I, worin die Strukturelemente $-A_1-A_2-$, $-A_3-A_4-$ und $-A_5-A_6-$ jeweils Vinylen bedeuten, X für $>$N- steht, alk 2-Methyl-1,3-propylen bedeutet, $R_1$ Pivaloyl ist, $R_2$, $R_4$ und $R_7$ jeweils Wasserstoff bedeuten, $R_3$und $R_3'$ gemeinsam für eine Bindung stehen und $R_5$ Acetyl ist, vom Schmelzpunkt 187-188°C (kristallisiert aus Methanol/Wasser).
$C_{50}H_{69}N_3O_{12}$; MG: 903 (gefunden, MS,FD). $^1$H-NMR (360 MHz, $CDCl_3$): Signale der Isobutylgruppe bei 0,92(6H, $(CH_3)_2CH$) 2,16 (d, 2H, $CH_2N$).

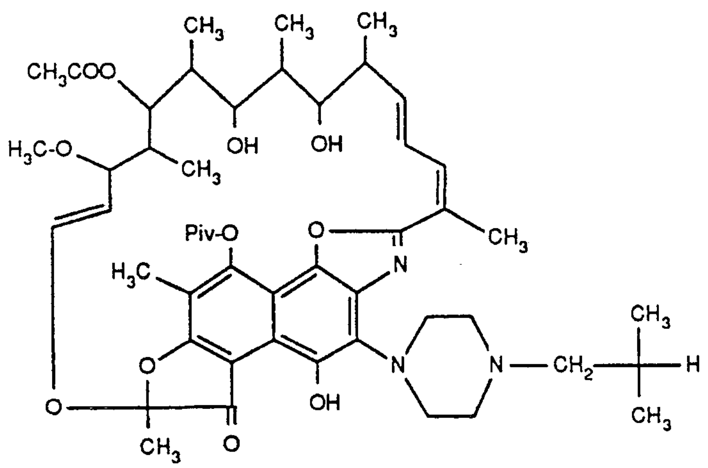

b) In analoger Weise wie im Beispiel 1 c) beschrieben erhält man durch Hydrierung mit $PtO_2$ in Ethanol unter Zusatz von Schwefelsäure das 16,17,18,19,28,29-Hexahydro-8-O-pivaloyl-11-hydroxy-1-desoxy-11,15-desoxo-1,15-oxy-3-(4-isobutyl-1-piperazinyl)-rifamycin entsprechend der Formel I, worin die Strukturelemente $-A_1-A_2-$, $-A_3-A_4-$ und $-A_5-A_6-$ jeweils Ethylen bedeuten, X für $>$ N- steht, alk 2-Methyl-1,3-propylen bedeutet, $R_1$ Pivaloyl bedeutet, $R_2$, $R_3$, $R_3'$, $R_4$ und $R_7$ jeweils Wasserstoff bedeuten und $R_5$ Acetyl bedeutet. Die schwach rötlich gefärbten Kristalle bilden nach dem Umkristallisieren aus Hexan farblose, quadratische Plättchen, die zwischen 150 und 160° schmelzen. $C_{50}H_{77}N_3O_{12}$; MG: 911 (gefunden; MS,FD).

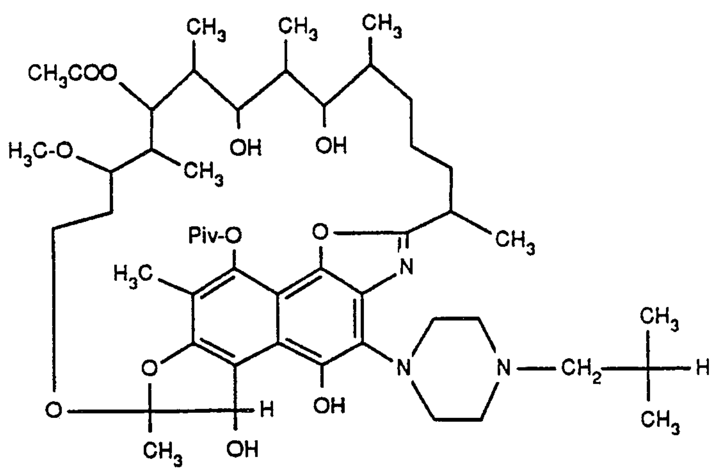

Beispiel 5:

In analoger Weise, z.B. wie in Beispiel 1 beschrieben, erhält man aus 16,17,18,19,28,29-Hexahydro-8-O-pivaloyl-11-hydroxy-1-desoxy-11,15-desoxo-1,15-oxy-3-[4-(2,4,6-trimethylbenzyl)-piperazin-1-yl]-rifamycin und Pyrokohlensäurediethylester das 16,17,18,19,28,29-Hexahydro-4-O-ethoxycarbonyl-8-O-pivaloyl-11-hydroxy-1-desoxy-11,15-desoxo-1,15-oxy-3-[4-(2,4,6-trimethylbenzyl)-piperazin-1-yl]-rifamycin der Formel I,

worin die Strukturelemente $-A_1-A_2-$, $-A_3-A_4-$ und $-A_5-A_6-$ jeweils Ethylen bedeuten, X für $\geq$ N- steht, alk Methylen bedeutet, $R_1$ Pivaloyl bedeutet, $R_2$ Ethoxycarbonyl ist, $R_4$ 2,4,6-Trimethylphenyl bedeutet, $R_5$ Acetyl bedeutet und $R_3$, $R_3'$ und $R_7$ jeweils Wasserstoff bedeuten. Durch Chromatographie an Kieselgel mit dem Elutionsmittel Hexan/Essigester (3:1) wird das Reaktionsprodukt von einer geringen Menge rasch wandernder Verunreinigungen befreit.

$C_{59}H_{85}N_3O_{14}$; MG: 1059 (gefunden: MS, DCI).

$^1$H-NMR (360 MHz, DMSO-$d_6$, 80°, ppm): 1,36 (t, etwa 3H) und 4,28 (q, etwa 2H): $COOCH_2CH_3$; 5,50 (s, 1H): H-11.

Beispiel 6:

In analoger Weise, z.B. wie in Beispiel 1 beschrieben, erhält man ausgehend von 16,17,18,19,28,29-Hexahydro-8-O-pivaloyl-11-hydroxy-1-desoxy-11,15-desoxo-1,15-oxy-3-[4-(2,4,6-trimethylbenzyl)-piperazin-1-yl]-rifamycin und Benzoesäureanhy drid das 16,17,18,19,28,29-Hexahydro-4-O-benzoyl-8-O-pivaloyl-11-hydroxy-1-desoxy-11,15-desoxo-1,15-oxy-3-[4-(2,4,6-trimethylbenzyl)-piperazin-1-yl]-rifamycin. Durch Chromatographie an Kieselgel mit dem Elutionsmittel Hexan/Essigester (3:1) wird das Reaktionsprodukt von einer geringen Menge rasch wandernder Verunreinigungen befreit.

$C_{63}H_{85}N_3O_{12}$; MG: 1091 (gefunden: MS DCI).

$^1$H-NMR (360 MHz, DMSO-$d_6$, 80°, ppm): 7,5-7,6 (m, etwa 3H) und etwa 8,5 (m, 2H): 5- arom. H der Benzoylgruppe; etwa 5,38 (unscharf, etwa 1 H): H-11.

Beispiel 7:

In analoger Weise, z.B. wie in Beispiel 4 beschrieben, erhält man ausgehend von 16,17,18,19,28,29-Hexahydro-8-O-pivaloyl-11-hydroxy-1-desoxy-11,15-desoxo-1,15-oxy-3-[4-(2,4,6-trimethylbenzyl)-piperazin-1-yl]-rifamycin und Benzoesäureanhydrid das 4-O-Benzoyl-8-O-pivaloyl-11-benzoyloxy-1-desoxy-11, 15-desoxo-1,15-oxy-3-[4-(2,4,6-trimethylbenzyl)-piperazin-1-yl]-rifamycin. $C_{70}H_{89}N_3O_{14}$; MG: 1196 (gefunden: MS, DCI).

Beispiel 8:

Man versetzt eine Lösung von 1,7 g 16,17,18,19-Tetrahydro-4-O-acetyl-8-O-pivaloyl-11-acetyloxy-1-desoxy-11,15-desoxo-1,15-oxy-3-[4-(2,4,6-trimethylbenzyl)-piperazin-1-yl]-rifamycin-21,23-acetonid in 50 ml Tetrahydrofuran mit 50 ml 2 N wässriger Schwefelsäure und lässt das Gemisch bei 25° 2 Stunden stehen. Danach setzt man wässrige NaCl-Lösung zu, stellt auf pH 7,5-8 ein und nimmt das Reaktionsgemisch in Ether auf. Nach Trocknen und Eindampfen des Lösungsmittels wird das Reaktionsgemisch an Kieselgel mit dem Elutionsmittel Petrolether/Essigester 3:1 chromatographisch getrennt. Man eluiert zunächst das Ausgangsmaterial und schliesslich als Hauptprodukt das cremefarbene 16,17,18,19-Tetrahydro-4-O-acetyl-8-O-pivaloyl-11-acetyloxy-1-desoxy-11,15-desoxo-1,15-oxy-3-[4-(2,4,6-trimethylbenzyl)-piperazin-1-yl]-rifamycin mit dem $R_f$ Wert von 0,23. MG: 1069 (gefunden: MS, DCI); $C_{60}H_{83}N_3O_{14}$.

Das Ausgangsmaterial kann beispielsweise wie folgt hergestellt werden:

Eine Lösung von 2 g 8-O-Pivaloyl-1-desoxy-15-desoxo-1,15-oxy-3-[4-(2,4,6-trimethylbenzyl)-piperazin-1-yl]-rifamycin hydriert man in 100 ml Ethanol in Gegenwart von 0,2 g Palladium auf Kohle (10%) bei 25° und Normaldruck bis zum Ende der Wasserstoffaufnahme. Anschliessend filtriert man den Katalysator ab und dampft das Lösungsmittel im Vakuum ein. Der dunkelrote Rückstand wird an 200 g Kieselgel mit dem Elutionsmittel Petrolether/Essigester (3:1) chromatographiert. Das Eluat der Hauptbande wird gesammelt und eingedampft. Der Rückstand besteht aus epimerenreinem 16,17,18,19-Tetrahydro-8-O-pivaloyl-1-desoxy-15-desoxo-1,15-oxy-3-[4-(2,4,6-trimethylbenzyl)-piperazinl-yl]-rifamycin. MG: 983 (gefunden: MS).

Eine Lösung von 7,95 g 16,17,18,19-Tetrahydro-8-O-pivaloyl-1-desoxy-15-desoxo-1,15-oxy-3-[4-(2,4,6-trimethylbenzyl)-piperazin-1-yl]-rifamycin in 75 ml Acetondimethylketal wird mit 2,3 g p-Toluolsulfonsäure versetzt und bei Raumtemperatur vier Stunden stehengelassen. Anschliessend neutralisiert man mit überschüssiger Natriumbicarbonatlösung, verdünnt mit Wasser und nimmt das Reaktionsprodukt mit Ether auf. Nach Trocknen über $Na_2SO_4$ und Eindampfen der Etherlösung wird der Rückstand durch 800 g Kieselgel mit dem Lösungsmittel Petrolether/Essigester 3:1 chromatographiert. Man erhält in der rasch wandernden Hauptbande das 16,17,18,19-Tetrahydro-8-O-pivaloyl-1-desoxy-15-desoxo-1,15-oxy-3-[4-(2,4,6-trimethylbenzyl)-piperazin-1-yl]-rifamycin-21,23-acetonid als tiefrot gefärbtes Material. MG: 1023 (gefunden: MS, DCI); $C_{59}H_{81}N_3O_{12}$.

Man versetzt eine 5%-ige methanolische Lösung des 16,17,18,19-Tetrahydro-8--pivaloyl-1-desoxy-15-desoxo-1,15-oxy-3-[4-(2,4,6-trimethylbenzyl)-piperazin-1-yl]-rifamycin-21,23-acetonid bei 25° solange tropfenweise mit 1%-iger methanolischer Natriumborhydridlösung, bis die rote Farbe des Ausgangsmaterials verschwunden ist. Danach setzt man dem Reaktionsgemisch gesättigte Kochsalzlösung zu und nimmt das gebildete Produkt in Ether auf. Nach Waschen der Etherlösung mit Pufferlösung von pH 7 und mit Kochsalzlösung und Trocknen mit Natriumsulfat dampft man im Vakuum ein und erhält als Rückstand das 16,17,18,19-Tetrahydro-8-O-pivaloyl-11-hydroxy-1-desoxy-11,15-desoxo-1,15-oxy-3-[4-(2,4,6-trimethylbenzyl)-piperazin-1-yl]-rifamycin-21,23-acetonid als cremefarbenen Schaum.

Eine Lösung von 1,8 g dieses Hexahydroderivates in 10 ml Pyridin wird mit 2 ml Essigsäureanhydrid und 0,2 g 4-Dimethylaminopyridin versetzt und bei Raumtemperatur 1 Stunde stehengelassen. Dann setzt man Wasser und Kochsalz zu, nimmt das Reaktionsprodukt in Ether/Essigester auf und wäscht die organische Phase mehrmals mit Zitronensäurelösung. Nach weiterm Waschen der organischen Phase mit Natriumbicarbonatlösung und gesättigte Kochsalzlösung wird mit Natriumsulfat getrocknet und eingedampft. Man erhält einen farblosen Rückstand von 16,17,18,19-Tetrahydro-4-O-acetyl-8-O-pivaloyl-11-acetyloxy-1-desoxy-11,15-desoxo-1,15-oxy-3-[4-(2,4,6-trimethylbenzyl)-piperazin-1-yl]-rifamycin-21,23-acetonid. MG: 1109 (gefunden: MS); $C_{63}H_{87}N_3O_{14}$.

Beispiel 9:

0,5 g 16,17,18,19,28,29-Hexahydro-8-O-pivaloyl-11-hydroxy-1-desoxy-11,15-desoxo-1,15-oxy-3-(4-isobutyl-piperazin-1-yl)-rifamycin werden in 10 ml Acetanhydrid gelöst. Dazu werden 0,5 g Natriumacetat gegeben und die Lösung wird bei Raumtemperatur 24 Stunden und dann bei 50° 5 Stunden gerührt. Die Lösung wird über Celite gefiltert und das Acetanhydrid im Vakuum verdampft. Der Rückstand wird durch Chromatographie über $SiO_2$ (Chromatotron, 4 mm; Elutionsmittel: $CH_2Cl_2/CH_3OH$: 97/3) und durch Ausfällen, wobei der Rückstand in 3 ml $CH_3OH$ gelöst und unter Rühren mit bis 30 ml einer 1:1-Lösung von $CH_3OH$/Wasser versetzt wird, gereinigt. Nach Filtrieren und Trocknen erhält man 16,17,18,19,28,29-Hexahydro-4-O-acetyl-8-O-pivaloyl-11-acetyloxy-1-desoxy-11,15-desoxo-1,15-oxy-3-(4-isobutyl-piperazin-1-yl)-rifamycin als beiges Pulver, Smp. 142 - 143°.

Das Ausgangsmaterial kann beispielsweise wie folgt hergestellt werden:

1 g (1,08 mmol) 8-O-pivaloyl-1-desoxy-15-desoxo-1,15-oxy-3-(4-isobutyl-piperazin-1-yl)-rifamycin wird in 50 ml absolutem Ethanol gelöst. Dazu werden 200 mg hydriertes $PtO_2$ gegeben und das System mehrfach im Vakuum mit Wasserstoff gereinigt. Die Reaktion wird bei Raumtemperatur und bei 1 atm 14-18 Stunden durchgeführt. Die Farbe der Lösung ändert sich von tiefem rot zu klarem Gelb. Das System wird anschliessend im Vakuum und unter Stickstoff gereinigt und mit 500 mg Ascorbinsäure versetzt. Dann wird der Katalysator über Celite abfiltriert und die Lösung eingedampft. Der gelbe Rückstand wird dann in 30 ml Wasser gelöst und 3 mal mit 20 ml Essigester extrahiert. Die organische Phase wird mit 30 ml Wasser gewaschen, direkt mit $MgSO_4$ getrocknet, filtriert und eingedampft. Der Rückstand wird dann in 100 ml kochendes Hexan suspendiert, filtriert, bei 0° kristallisiert und getrocknet. Man erhält so das 16,17,18,19,28,29-Hexahydro-8-O-pivaloyl-11-hydroxy-1-desoxy-11,15-desoxo-1,15-oxy-3-(4-isobutyl-piperazin-1-yl)-rifamycin. 200 mg Ascorbinsäure in 10 ml Methanol werden zu 400 mg 16,17,18,19,28,29-Hexahydro-8-O-pivaloyl-11-hydroxy-1-desoxy-11,15-desoxo-1,15-oxy-3-(4-isobutyl-piperazin-1-yl)-rifamycin gegeben. Die Lösung wird 15 Minuten gerührt und eingedampft. Der klare Rückstand wird in 10 ml Wasser gelöst, tropfenweise mit 15 ml einer gesättigten NaCl-Lösung und dann mit 2,5 g NaCl versetzt.Die Lösung wird 30 Minuten gerührt und 3 mal mit 10 ml Essigester extrahiert. Die organische Phase wird mit 10 ml einer gesättigten NaCl-Lösung gewaschen, mit $MgSO_4$ getrocknet, über Celite filtriert und eingedampft. Der Rückstand kristallisiert aus Essigester/Hexan 3/2. Man erhält so das Hydrochlorid in beigen Kristallen, Smp. 175-177°.

Beispiel 10:

1,0 g 16,17,18,19,28,29-Hexahydro-8-O-pivaloyl-11-hydroxy-1-desoxy-11,15-desoxo-1,15-oxy-3-(4-isobutyl-piperazin-1-yl)-rifamycin, erhalten z.B. gemäss Beispiel 1, werden in 20 ml Propionsäureanhydrid gelöst. Dann wird 1,0 g Natriumacetat zugefügt und die Lösung unter Stickstoff vier Stunden bei Raumtemperatur und eine Stunde bei 50° gerührt. Die Lösung wird über Celite filtriert. Das Propionsäureanhydrid wird im Vakuum abgedampft. Der Rückstand wird in 10 ml Essigester gelöst. Die organische Phase wird mit 5 ml 1 N $NaHCO_3$, 5 ml gesättigter NaCl-Lösung gewaschen, über $MgSO_4$ getrocknet, filtriert and im Vakuum eingedampft. Der Rückstand wird chromatographisch ($SiO_2$, Chromatotron, 4 mm, Hexan/Essigester: 6/1 bis 1:1) und durch Ausfällen gereinigt, wobei der Rückstand in 2 ml Methanol gelöst und mit bis zu 50 ml einer Methanol/Wasser-Lösung 1/2 versetzt, gereinigt. Man erhält nach Filtrieren und Trocknen 16,17,18,19,28,29-Hexahydro-4-O-propionyl-8-O-pivaloyl-11-propionyloxy-1-desoxy-11,15-desoxo-1,15-oxy-3-(4-isobutyl-piperazin-1-yl)-rifamycin als beiges Pulver, Smp.129-131°.

Beispiel 11:

0,76 g rohes 16,17,18,19,28,29-Hexahydro-8-O-pivaloyl-11-hydroxy-1-desoxy-11,15-desoxo-1,15-oxy-3-(4-cyclohexylmethyl-piperazin-1-yl)-rifamycin werden in 15 ml Propionsäureanhydrid gelöst. Die Lösung wird vier Stunden bei 40° unter Stickstoff gerührt. Das Propionsäureanhydrid wird im Vakuum abgedampft. Der Rückstand wird in 15 ml Essigester gelöst. Die organische Phase wird mit 10 ml 1 N $NaHCO_3$, mit gesättigter NaCl-Lösung gewaschen, über $MgSO_4$ getrocknet, filtriert und im Vakuum eingedampft. Der Rückstand wird chromatographisch ($SiO_2$, 100 g, Flash-Chromatographie, Hexan/Aceton: 3:1) und durch Ausfällen gereinigt, wobei der Rückstand in 3 ml Methanol gelöst und mit bis zu 30 ml einer gerührten 1:1 Methanol/Wasser-Lösung versetzt wird. Nach Filtrieren und Trocknen erhält man 16,17,18,19,28,29-Hexahydro-4-O-propionyl-8-O-pivaloyl-11-propionyloxy-1-desoxy-11,15-desoxo-1,15-oxy-3-(4-cyclohexylmethyl-piperazin-1-yl)-rifamycin vom Smp. 139 -141°.

Das Ausgangsmaterial kann beispielsweise wie folgt hergestellt werden:

1 g 8-O-Pivaloyl-1-desoxy-15-desoxo-1,15-oxy-3-(4-cyclohexylmethyl-piperazin-1-yl)-rifamycin werden in 50 ml absolutem Ethanol gelöst. 200 mg hydriertes $PtO_2$ werden zugefügt, und das System wird mehrmals im Vakuum mit Wasserstoff gereinigt. Die Reaktion wird bei Raumtemperatur und 1 atm über 14-18 Stunden durchgeführt. Die Farbe der Lösung ändert sich von tiefem rot bis klarem gelb. Das System wird mehrmal im Vakuum mit Stickstoff gereinigt. Der Katalysator wird über Celite abfiltriert und die Lösung eingedampft. Der Rückstand wird in 5 ml Methanol gelöst und unter Stickstoff bei 0° gerührt. Nach wenigen Minuten kristallisiert das Produkt, wird über Papier filtriert und getrocknet. Man erhält so 16,17,18,19,28,29-Hexahydro-8-O-pivaloyl-11-hydroxy-1-desoxy-11,15-desoxo-1,15-oxy-3-(4-cyclohexylmethyl-piperazin-1-yl)-rifamycin als weisses Pulver.

Beispiel 12:

0,5 g rohes 16,17,18,19,28,29-Hexahydro-8-O-pivaloyl-11-hydroxy-1-desoxy-11,15-desoxo-1,15-oxy-3-(4-cyclohexylmethyl-piperazin-1-yl)-rifamycin werden in 10 ml Acetanhydrid gelöst. Die Lösung wird 4 Stunden bei 40° unter Stickstoff gerührt. Das Acetanhydrid wird im Vakuum abgedampft. Der Rückstand wird in 10 ml Essigester gelöst. Die organische Phase wird mit 5 ml $NaHCO_3$, mit 5 ml gesättigter NaCl-Lösung gewaschen, über $MgSO_4$ getrocknet, filtriert und im Vakuum eingedampft. Der Rückstand wird chromatographisch ($SiO_2$, 50 g, Flash-Chromatographie, Hexan/Essigester: 1:1) und durch Ausfällen gereinigt, wobei der Rückstand in 3 ml Methanol gelöst und mit bis zu 30 ml einer gerührten 1:1 Methanol/Wasser-Lösung versetzt wird. Nach Filtrieren und Trocknen erhält man 16,17,18,19,28,29-Hexahydro-4-O-acetyl-8-O-pivaloyl-11-acetyloxy-1-desoxy-11,15-desoxo-1,15-oxy-3-(4-cyclohexylmethyl-piperazin-1-yl)-rifamycin vom Smp. 147 -149° als weisses Pulver.

Beispiel 13:

Eine Lösung von 10 g 8-O-Pivaloyl-1-desoxy-15-desoxo-1,15-oxy-3-[4-(2,4,6-trimethylbenzyl)-piperazin-1-yl]-rifamycin in 100 ml Essigester und 100 ml Acetanhydrid wird mit 2 ml Essigsäure und 2,4 g $PtO_2$ versetzt und bei Raumtemperatur und 49 psi hydriert. Nach 53 Stunden wird der Katalysator abfiltriert und der Rückstand im Vakuum eingedampft. Das Rohprodukt wird durch Flash-Chromatographie über Kieselgel mit dem Elutionsmittel Essigester/Hexan 1:3 bis 1:1 gereinigt. Das amorphe gereinigte Produkt wird aus Methanol/Wasser kristallisiert. Man erhält so das 16,17,18,19,28,29-Hexahydro-4-O-acetyl-8-O-pivaloyl-11-acetyloxy-1-desoxy-11, 15-desoxo-1,15-oxy-3-[4-(2,4,6-trimethylbenzyl)-piperazin-1-yl]-rifamycin, Smp. 150° (Zersetzung). MG: 1072,45 (gefunden: MS).

Beispiel 14:

Eine Lösung von 800 mg 16,17,18,19,28,29-Hexahydro-8-O-pivaloyl-11-hydroxy-1-desoxy-11,15-desoxo-1,15-oxy-3-[4-(2,4,6-trimethylbenzyl)-piperazin-1-yl]-rifamycin in 20 ml Methylenchlorid wird auf 0° gekühlt und mit 0,12 ml Triethylamin versetzt. Dann wird eine Lösung von 0,06 ml Methansulfonylchlorid in 3 ml Methylenchlorid tropfenweise unter Rühren zugetropft. Es wird 15 Stunden bei Raumtemperatur gerührt, dann das Lösungsmittel im Vakuum entfernt,der Rückstand in Essigester gelöst und mit gesättigter Natriumbicarbonat- und Kochsalzlösung extrahiert und über $MgSO_4$ getrocknet. Das erhaltene 16,17,18,19,28,29-Hexahydro-4-O-methansulfonyl-8-O-pivaloyl-11-hydroxy-1-desoxy-11,15-desoxo-1,15-oxy-3-[4-(2,4,6-trimethylbenzyl)-piperazin-1-yl]-rifamycin wird durch Flash-Chromatographie über Kieselgel unter Verwendung des Elutionsmittels Essigester/Hexan 1:2 gereinigt, Smp. 146-150°. MG: 1066 (gefunden: MS FAB).

Das Ausgangsmaterial kann beispielsweise wie folgt hergestellt werden:

5 g 8-O-Pivaloyl-1-desoxy-15-desoxo-1,15-oxy-3-[4-(2,4,6-trimethylbenzyl)-piperazin-1-yl]-rifamycin werden in 500 ml Ethanol gelöst und bei Raumtemperatur und Normaldruck in Gegenwart von g $PtO_2$ und 0,7 g Schwefelsäure bis zur Aufnahme von 4 Aequivalenten (460 ml) Wasserstoff hydriert. Man entfernt den Katalysator durch Filtration, neutralisiert mittels wässriger Natriumbicarbonatlösung, setzt gesättigte Kochsalzlösung zu und extrahiert das Hydrierungsprodukt durch wiederholtes Ausschütteln mit Essigester. Nach einigem Stehen kristallisiert das 16, 17,18,19,28,29-Hexahydro-8-O-pivaloyl-11-hydroxy-1-desoxy-11,15-desoxo-1,15-oxy-3-[4-(2,4,6-trimethylbenzyl)-piperazin-1-yl]-rifamycin in schwach braunroten Kristallen, die nach Umkristallisieren aus Ether farblose Plättchen bilden und bei 226-227° (Zersetzung) schmelzen.

Beispiel 15:

Eine Lösung von 400 mg 8-O-Pivaloyl-1-desoxy-15-desoxo-1,15-oxy-3-[2-methyl-4-(2,4,6-trimethylbenzyl)-piperazin-1-yl]-rifamycin (Isomeres A) in 8 ml Essigester, 8 ml Acetanhydrid und 1,6 ml Essigsäure wird bei 50 psi und Raumtemperatur 4,5 Stunden über $PtO_2$ hydriert. Der Katalysator wird abfiltriert und das Filtrat im Vakuum konzentriert. Der erhaltene Rückstand wird durch Flash-Chromatographie über Silicagel mit dem Elutionsmittel Hexan/Essigester 3:1 gereinigt. Man erhält so das 16,17,18,19,28,29-Hexahydro-4-O-acetyl-8-O-pivaloyl-11-hydroxy-1-desoxy-11,15-desoxo-1,15-oxy-3-[2-methyl-4-(2,4,6-trimethylbenzyl)-piperazin-1-yl]-rifamycin (Isomeres A) vom Smp. 146-149°.

Das Ausgangsmaterial kann beispielsweise wie folgt hergestellt werden:

Eine Lösung von 1,8-Di-O-pivaloyl-3-[2-methyl-4-(2,4,6-trimethylbenzyl)-pipera zin-1-yl]-rifamycin in 8 ml 2-Methoxyethanol wird unter Stickstoff unter Rückfluss erhitzt. Nach 5-stündigem Erhitzen wird das Lösungsmittel im Vakuum entfernt. Das Rohprodukt wird durch Flash-Chromatographie an Silikagel gereinigt und mit Ethylacetat/Hexan 1:4 bis 1:3 eluiert. Man erhält so das 8-O-Pivaloyl-1-desoxy-15-desoxo-1,15-oxy-3-[2-methyl-4-(2,4,6-trimethylbenzyl)-piperazin-1-yl]-rifamycin.

Isomeres A: (im Hinblick auf die Methylgruppe am Piperazinring) Smp. 140° (Zersetzung); NMR (300 MHz,$CD_3OD$): 6,80 (s, 2H).

Isomeres B: (im Hinblick auf die Methylgruppe am Piperazinring) Smp. 140° (Zersetzung); NMR (300 MHz,$CD_3OD$): 6,85 (s, 2H).

Beispiel 16:

Eine Lösung von 400 mg 8-O-Pivaloyl-1-desoxy-15-desoxo-1,15-oxy-3-[2-methyl-4-(2,4,6-trimethylbenzyl)-piperazin-1-yl]-rifamycin (Isomeres B, siehe Beispiel 15) in 8 ml Essigester, 8 ml Acetanhydrid und 1,6 ml Essigsäure wird 4 Stunden bei Raumtemperatur und 45 psi über 80 mg $PtO_2$ hydriert. Der Katalysator wird abfiltriert, das Filtrat im Vakuum eingedampft und der Rückstand durch Flash-Chromatographie unter Verwendung von Hexan/Essigester 3:1 bis 2:1 als Elutionsmittel gereinigt. Man erhält so 16,17,18,19,28,29-Hexahydro-4-O-acetyl-8-O-pivaloyl-11-hydroxy-1-desoxy-11,15-desoxo-1,15-oxy-3-[2-methyl-4-(2,4,6-trimethylbenzyl)-piperazin-1-yl]-rifamycin (Isomeres B) vom Smp. 147-150°.

Beispiel 17:

In analoger Weise, beispielsweise wie in Beispiel 13 beschrieben, kann man ausgehend von 8-O-Pivaloyl-1-desoxy-15-desoxo-1,15-oxy-3-(4-cyclopentylmethyl-1-piperazinyl)-rifamycin das 16,17,18,19,28,29-Hexahydro-4-O-acetyl-8-O-pivaloyl-11-hydroxy-1-desoxy-11,15-desoxo-1,15-oxy-3-(4-cyclopentylmethyl-1-piperazinyl)-rifamycin vom Smp. 148-152° herstellen.

Das Ausgangsmaterial 8-O-Pivaloyl-1-desoxy-15-desoxo-1,15-oxy-3-(4-cyclopentylmethyl-1-piperazinyl)-rifamycin kann in analoger Weise, beispielsweise wie in Beispiel 1 beschrieben, hergestellt werden.

Beispiel 18:

In analoger Weise, beispielsweise wie in Beispiel 13 beschrieben, kann man das 16,17,18,19,28,29-Hexahydro-4-O-acetyl-8-O-pivaloyl-11-hydroxy-1-desoxy-11,15-desoxo-1,15-oxy-3-[4-(2-cyclopentylethyl)-piperazin-1-yl]-rifamycin vom Smp. 130-132° herstellen.

Das Ausgangsmaterial kann in analoger Weise, beispielsweise wie in Beispiel 1 beschreiben, hergestellt werden.

Beispiel 19:

In analoger Weise, beispielsweise wie in Beispiel 13 beschrieben, kann man das 16,17,18,19,28,29-Hexahydro-4-O-acetyl-8-O-pivaloyl-11-acetyloxy-1-desoxy-11,15-desoxo-1,15-oxy-3-(4-neopentyl-piperazin-1-yl)-rifamycin vom Smp. 123-126° herstellen.

Das Ausgangsmaterial kann in analoger Weise, beispielsweise wie in Beispiel 1 beschreiben, hergestellt werden.

Beispiel 20:

In analoger Weise, beispielsweise wie in Beispiel 15 beschrieben, kann man das 16,17,18,19,28,29-Hexahydro-4-O-acetyl-8-O-pivaloyl-11-acetyloxy-1-desoxy-11,15-desoxo-1,15-oxy-3-(2-methyl-4-cyclohexylmethyl-piperazin-1-yl)-rifamycin (Isomeres A) vom Smp. 156-158° und das 16,17,18,19,28,29-Hexahydro-4-O-acetyl-8-O-pivaloyl-11-acetyloxy-1-desoxy-11,15-desoxo-1,15-oxy-3-(2-methyl-4-cyclohexylmethyl-piperazin-1-yl)-rifamycin (Isomeres B) vom Smp. 152-156° herstellen.

Beispiel 21:

Ein Gemisch von 18 ml Propionsäureanhydrid und 3 g 8-O-Pivaloyl-1-desoxy-15-desoxo-1,15-oxy-3-[4-(2,4,6-trimethylbenzyl)-piperazin-1-yl]-rifamycin wird 20 Minuten auf 140° erhitzt. Das überschüssige Propionsäureanhydrid wird im Vakuum entfernt und das Rohprodukt durch Flash-Chromatographie gereinigt, wobei als Eluiermittel Hexan:Essigester 4:1 verwendet wird. Man erhält so das 4-O-Propionyl-8-O-pivaloyl-1-desoxy-15-desoxo-1,15-oxy-3-[4-(2,4,6-trimethylbenzyl)-piperazin-1-yl]-rifamycin vom Smp. 136-139°.

In analoger Weise kann man das 4-O-Acetyl-8-O-pivaloyl-1-desoxy-15-desoxo-1,15-oxy-3-[4-(2,4,6-trimethylbenzyl)-piperazin-1-yl]-rifamycin vom Smp. 156-160° herstellen.

Beispiel 22:

Eine Lösung von 10 ml Acetanhydrid und 1 g 8-O-Pivaloyl-1-desoxy-15-desoxo-1,15-oxy-3-[2-methyl-4-(2,4,6-trimethylbenzyl)-piperazin-1-yl]-rifamycin (Isomeres B) wird 20 Minuten auf 140° erhitzt. Das überschüssige Acetanhydrid wird im Vakuum entfernt und das Rohprodukt durch Flash-Chromatographie gereinigt, wobei als Eluiermittel Hexan/Essigester (von 3:1 über 2:1 bis 1:1) verwendet wird. Man erhält so das 4-O-Acetyl-8-O-pivaloyl-1-desoxy-15-desoxo-1,15-oxy-3-[2-methyl-4-(2,4,6-trimethylbenzyl)-piperazin-1-yl]-rifamycin vom Smp. 148-152°.

In analoger Weise kann man ausgehend von dem entsprechenden A-Isomeren das entsprechende Isomere 4-O-Acetyl-8-O-pivaloyl-1-desoxy-15-desoxo-1,15-oxy-3-[2-methyl- 4-(2,4,6-mmethylbenzyl)-piperazin-1-yl]-rifamycin vom Smp. 145-148° herstellen.

Beispiel 23:

Kapseln, enthaltend 250 mg Wirkstoff, z.B. die Verbindung der Formel I, worin die Strukturelemente $-A_1-A_2-$, $A_3-A_4-$ und $-A_5-A_6-$ jeweils Ethylen bedeuten, X für $>$ N- steht, alk Methylen bedeutet, $R_1$ Pivaloyl bedeutet, $R_2$ Acetyl bedeutet, $R_3$, $R_3'$ und $R_7$ jeweils Wasserstoff bedeuten, $R_4$ 2,4,6-Trimethylphenyl bedeutet und $R_5$ Acetyl ist, können wie folgt hergestellt werden:

| Zusammensetzung (für 1000 Kapseln): | |
|---|---|
| Wirkstoff | 250,0g |
| Maisstärke | 50,0g |
| Polyvinylpyrrolidon | 15,0g |
| Magnesiumstearat | 5,0g |
| Ethanol | q.s. |

Der Wirkstoff und die Maisstärke werden vermischt und mit einer Lösung des Polyvinylpyrrolidons in 50 g Ethanol befeuchtet. Die feuchte Masse wird durch ein Sieb mit einer Maschenweite von 3 mm gepresst und bei 45° getrocknet. Das trockene Granulat wird durch ein Sieb mit einer Maschen- weite von 1 mm gesiebt und mit 5 g Magnesiumstearat vermischt. Die Mischung wird in Portionen von 0,320 g in Steckkapseln der Grösse 0 abgefüllt.

In analoger Weise kann man auch die übrigen, gemäss den vorstehenden Beispielen hergestellten Verbindungen als Wirkstoffkomponente verwenden.

**Ansprüche**

1. Verbindungen der Formel

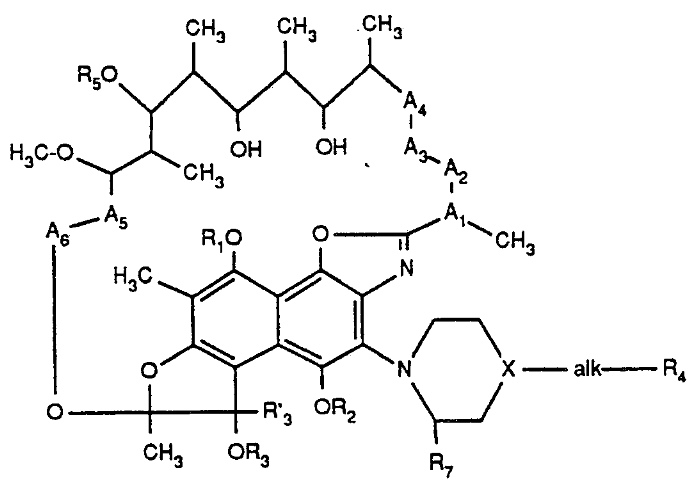

(I),

und ihre Salze, worin die Strukturelemente $-A_1-A_2-$, $-A_3-A_4-$ und $-A_5-A_6-$ jeweils Ethylen oder Vinylen oder die Elemente $-A_1-A_2-$ und $-A_3-A_4-$ jeweils Ethylen und $-A_5-A_6-$Vinylen bedeuten; X für $>$ $C(R_6)$- oder $>$ N- steht und $R_6$ Wasserstoff oder Alkyl bedeutet; alk einen aliphatischen Kohlenwasserstoffrest bedeutet; $R_1$ Wasserstoff oder Acyl bedeutet; $R_2$ Acyl bedeutet und $R_3'$ und $R_3'$ für eine gemeinsame Bindung stehen oder $R_3$ Wasserstoff oder Acyl bedeutet und $R_3'$ Wasserstoff ist; Wasserstoff, Cycloalkyl oder Aryl bedeutet; $R_5$ Wasserstoff oder Acetyl bedeutet; $R_7$ Wasserstoff oder Alkyl bedeutet.

2. Verbindungen gemäss Anspruch 1 der Formel I und ihre Salze, worin die Strukturelemente $-A_1-A_2-$, $-A_3-A_4-$ und $-A_5-A_6-$ jeweils Ethylen oder Vinylen oder die Elemente $-A_1-A_2-$ und $-A_3-A_4-$ jeweils Ethylen und $-A_5-A_6-$ Vinylen bedeuten; x für $>$ $C(R_6)$- oder $>$ N- und $R_6$ Wasserstoff ist; alk einen aliphatischen Kohlenwasserstoffrest bedeutet; $R_1$ Wasserstoff oder Acyl bedeutet; $R_2$ und $R_3$ unabhängig voneinander Acyl bedeuten; $R_3'$ Wasserstoff bedeutet; $R_4$ Wasserstoff, Cycloalkyl oder Aryl bedeutet; $R_5$

Wasserstoff oder Acetyl bedeutet; $R_7$ Wasserstoff bedeutet.

3. Verbindungen gemäss Anspruch 1 der Formel I und ihre Salze, worin $-A_1-A_2-$, $-A_3-A_4-$, $-A_5-A_6-$ und $R_5$ die angegebenen Bedeutungen haben;. X für $\geq$ C($R_6$)- oder $\geq$ N- steht und $R_6$ Wasserstoff oder $C_1$-$C_7$-Alkyl bedeutet; alk $C_1$-$C_7$-Alkylen, $C_3$-$C_7$-Alkenylen oder $C_3$-$C_7$-Alkinylen bedeutet; $R_1$ $C_2$-$C_8$-Alkanoyl, Halogen-$C_2$-$C_8$-alkanoyl, Phenyl- oder Naphthyl-$C_2$-$C_8$-alkanoyl, Benzoyl, Naphthoyl, 5- oder 6-gliedriges monocyclisches Monoaza-, Monooxa- oder Monothiaaroyl, $C_1$-$C_7$-Alkoxycarbonyl, Phenyl- oder Naphthyl-$C_1$-$C_7$-alkoxy-carbonyl, unsubstituiertes oder durch $C_1$-$C_7$-Alkyl mono- oder di-substituiertes Aminocarbonyl, $C_1$-$C_7$-Alkansulfonyl, Halogen-$C_1$-$C_7$-alkansulfonyl, Phenyl- oder Naphthyl-$C_1$-$C_7$-alkansulfonyl, $C_3$-$C_7$-Cycloalkansulfonyl oder Benzol- oder Naphthylsulfonyl bedeutet; $R_2$ $C_2$-$C_8$-Alkanoyl, Halogen-$C_2$-$C_8$-alkanoyl, Phenyl- oder Naphthyl-$C_2$-$C_8$-alkanoyl, Benzoyl, Naphthoyl, 5- oder 6-gliedriges monocyclisches Monoaza-, Monooxa- oder Monothiaaroyl, $C_1$-$C_7$-Alkoxycarbonyl, Phenyl- oder Naphthyl-$C_1$-$C_7$-alkoxy-carbonyl, unsubstituiertes oder durch $C_1$-$C_7$-Alkyl mono- oder di-substituiertes Aminocarbonyl, $C_1$-$C_7$-Alkansulfonyl, Halogen-$C_1$-$C_7$-alkansulfonyl, Phenyl- oder Naphthyl-$C_1$-$C_7$-alkansulfonyl, $C_3$-$C_7$-Cycloalkansulfonyl oder Benzol- oder Naphthylsulfonyl bedeutet, $R_3$ und $R_3'$ für eine gemeinsame Bindung stehen oder $R_3$ Wasserstoff oder $C_2$-$C_8$-Alkanoyl, Halogen-$C_2$-$C_8$-alkanoyl, Phenyl- oder Naphthyl-$C_2$-$C_8$-alkanoyl, Benzoyl, Naphthoyl, 5-oder 6-gliedriges monocyclisches Monoaza-, Monooxa- oder Monothiaaroyl, $C_1$-$C_7$-Alkoxycarbonyl, Phenyl- oder Naphthyl-$C_1$-$C_7$-alkoxy-carbonyl, unsubstituiertes oder durch $C_1$-$C_7$-Alkyl mono- oder di-substituiertes Aminocarbonyl, $C_1$-$C_7$-Alkansulfonyl, Halogen-$C_1$-$C_7$-alkansulfonyl, Phenyl- oder Naphthyl-$C_1$-$C_7$-alkansulfonyl, $C_3$-$C_7$-Cycloalkansulfonyl oder Benzol- oder Naphthylsulfonyl bedeutet und $R_3'$ Wasserstoff ist; $R_4$ Wasserstoff, $C_3$-$C_7$-Cycloalkyl, Phenyl, Biphenylyl oder Naphthyl bedeutet; $R_7$ Wasserstoff oder $C_1$-$C_7$-Alkyl bedeutet; wobei die jeweiligen aromatischen Reste unsubstituiert oder ein- oder mehrfach substituiert sind durch Substituenten ausgewählt aus der Gruppe bestehend aus Halogen, $C_1$-$C_7$-Alkyl, $C_1$-$C_7$-Alkoxy, Hydroxy, $C_2$-$C_8$-Alkanoyloxy, Trifluormethyl und Nitro.

4. Verbindungen gemäss Anspruch 1 der Formel I und ihre Salze, worin $-A_1-A_2-$, $-A_3-A_4-$, $-A_5-A_6-$ und $R_5$ die angegebenen Bedeutungen haben; X für $\geq$ C($R_6$)- oder $\geq$ N- steht und $R_6$ Wasserstoff ist; alk $C_1$-$C_7$-Alkylen, $C_3$-$C_7$-Alkenylen oder $C_3$-$C_7$-Alkinylen bedeutet; $R_1$, $R_2$ und $R_3$ unabhängig voneinander $C_2$-$C_8$-Alkanoyl, Halogen-$C_2$-$C_8$-alkanoyl, Phenyl- oder Naphthyl-$C_2$-$C_8$-alkanoyl, Benzoyl, Naphthoyl, 5-oder 6-gliedriges monocyclisches Monoaza-, Monooxa- oder Monothiaaroyl, $C_1$-$C_7$-Alk oxycarbonyl, Phenyl- oder Naphthyl-$C_1$-$C_7$-alkoxy-carbonyl, unsubstituiertes oder durch $C_1$-$C_7$-Alkyl mono- oder di-substituiertes Aminocarbonyl, $C_1$-$C_7$-Alkansulfonyl, Halogen-$C_1$-$C_7$-alkansulfonyl, Phenyl- oder Naphthyl-$C_1$-$C_7$-alkansulfonyl, $C_3$-$C_7$-Cycloalkansulfonyl oder Benzol- oder Naphthylsulfonyl bedeuten und $R_1$ zusätzlich sowie $R_3'$ und $R_7$ Wasserstoff bedeuten; $R_4$ Wasserstoff, $C_3$-$C_7$-Cycloalkyl, Phenyl, Biphenylyl oder Naphthyl bedeutet; wobei die jeweiligen aromatischen Reste unsubstituiert oder ein- oder mehrfach substituiert sind durch Substituenten ausgewählt aus der Gruppe bestehend aus Halogen, $C_1$-$C_7$-Alkyl, $C_1$-$C_7$-Alkoxy, Hydroxy, $C_2$-$C_8$-Alkanoyloxy, Trifluormethyl und Nitro.

5. Verbindungen gemäss Anspruch 1 der Formel I und ihre Salze, worin $-A_1-A_2-$, $-A_3-A_4-$, $-A_5-A_6-$ und $R_5$ die angegebenen Bedeutungen haben; X für $\geq$ C($R_6$)- oder $\geq$ N- steht und $R_6$ Wasserstoff ist; alk $C_1$-$C_7$-Alkylen, $C_3$-$C_7$-Alkenylen oder $C_3$-$C_7$-Alkinylen, insbesondere $C_1$-$C_4$-Alkylen, $C_3$-$C_4$-Alkenylen oder $C_3$-$C_4$-Alkinylen, bedeutet, wobei die Mehrfachbindungen in höherer als der $\alpha$-Stellung zum Piperazinstickstoff lokalisiert sind; $R_1$ $C_2$-$C_8$-Alkanoyl, insbesondere $C_2$-$C_6$-Alkanoyl, Phenyl-$C_2$-$C_6$-alkanoyl, Benzoyl, $C_1$-$C_7$-Alkoxy-carbonyl, wie $C_1$-$C_4$-Alkoxycarbonyl, Phenyl-$C_1$-$C_4$-alkoxycarbonyl, $C_1$-$C_4$-Alkansulfonyl oder Benzolsulfonyl bedeuten; $R_2$ $C_2$-$C_8$-Alkanoyl, insbesondere $C_2$-$C_6$-Alkanoyl, Phenyl-$C_2$-$C_6$-alkanoyl, $C_1$-$C_7$-Alkoxy-carbonyl, wie $C_1$-$C_4$-Alkoxycarbonyl, Phenyl-$C_1$-$C_4$-alkoxycarbonyl, $C_1$-$C_4$-Alkansulfonyl oder Benzolsulfonyl bedeutet; $R_3$ und $R_3'$ gemeinsam für eine Bindung stehen oder $R_3$ Wasserstoff, $C_2$-$C_8$-Alkanoyl, insbesondere $C_2$-$C_6$-Alkanoyl, Phenyl-$C_2$-$C_6$-alkanoyl, Benzoyl, $C_1$-$C_7$-Alkoxy-carbonyl, wie $C_1$-$C_4$-Alkoxycarbonyl, Phenyl-$C_1$-$C_4$-alkoxycarbonyl, $C_1$-$C_4$-Alkansulfonyl oder Benzolsulfonyl bedeutet und $R_3'$ Wasserstoff ist; $R_4$ Wasserstoff, $C_3$-$C_7$-Cycloalkyl, Phenyl, Biphenylyl oder Naphthyl bedeutet; $R_7$ Wasserstoff oder $C_1$-$C_7$-Alkyl bedeutet; wobei die jeweiligen aromatischen Reste unsubstituiert oder ein- oder mehrfach substituiert sind durch Substituenten ausgewählt aus der Gruppe bestehend aus Halogen, $C_1$-$C_7$-Alkyl, $C_1$-$C_7$-Alkoxy, Hydroxy, $C_2$-$C_8$-Alkanoyloxy, Trifluormethyl und Nitro.

6. Verbindungen gemäss Anspruch 1 der Formel I und ihre Salze, worin $-A_1-A_2-$, $-A_3-A_4-$, $-A_5-A_6-$ und $R_5$ die angegebenen Bedeutungen haben; X für $\geq$ C($R_6$)- oder $\geq$ N- steht und $R_6$ Wasserstoff ist; alk $C_1$-$C_7$-Alkylen, $C_3$-$C_7$-Alkenylen oder $C_3$-$C_7$-Alkinylen, insbesondere $C_1$-$C_4$-Alkylen, $C_3$-$C_4$-Alkenylen oder $C_3$-$C_4$-Alkinylen, bedeutet, wobei die Mehrfachbindungen in höherer als der $\alpha$-Stellung zum Piperazinstickstoff lokalisiert sind; $R_1$, $R_2$ und $R_3$ unabhängig voneinander $C_2$-$C_8$-Alkanoyl, insbesondere $C_2$-$C_6$-Alkanoyl, Phenyl-$C_2$-$C_6$-alkanoyl, $C_1$-$C_7$-Alkoxy-carbonyl, wie $C_1$-$C_4$-Alkoxycarbonyl, Phenyl-$C_1$-$C_4$-alkoxycarbonyl, $C_1$-$C_4$-Alkansulfonyl oder Benzolsulfonyl bedeuten und $R_1$ zusätzlich sowie $R_3$ und $R_7$ Wasserstoff bedeuten; $R_4$ Wasserstoff, $C_3$-$C_7$-Cycloalkyl, Phenyl, Biphenylyl oder Naphthyl bedeutet; wobei die

jeweiligen aromatischen Reste unsubstituiert oder ein- oder mehrfach substituiert sind durch Substituenten ausgewählt aus der Gruppe bestehend aus Halogen, $C_1$-$C_7$-Alkyl, $C_1$-$C_7$-Alkoxy, Hydroxy, $C_2$-$C_8$-Alkanoyloxy, Trifluormethyl und Nitro.

7. Verbindungen gemäss Anspruch 1 der Formel I und ihre Salze, worin -$A_1$-$A_2$-, -$A_3$-$A_4$-, -$A_5$-$A_6$- die angegebenen Bedeutungen haben; X für $\geq$ C($R_6$)- oder $\geq$ N-steht und $R_6$ Wasserstoff ist; alk $C_1$-$C_7$-Alkylen, $C_3$-$C_7$-Alkenylen oder $C_3$-$C_7$-Alkinylen, insbesondere $C_1$-$C_4$-Alkylen, $C_3$-$C_4$-Alkenylen oder $C_3$-$C_4$-Alkinylen, bedeutet, wobei die. Mehrfachbindungen in höherer als der $\alpha$-Stellung zum Piperazinstickstoff lokalisiert sind; $R_1$, $R_2$ und $R_3$ unabhängig voneinander $C_2$-$C_8$-Alkanoyl, insbesondere $C_2$-$C_6$-Alkanoyl, $C_1$-$C_4$-Alkoxy-carbonyl, $C_1$-$C_4$-Alkansulfonyl oder Benzolsulfonyl bedeuten, $R_1$ zusätzlich sowie $R_3'$ und $R_7$ Wasserstoff bedeuten; $R_4$ Wasserstoff, $C_3$-$C_7$-Cycloalkyl, Phenyl, Biphenylyl oder Naphthyl bedeutet; $R_5$ Acetyl ist; wobei die jeweiligen aromatischen Reste unsubstituiert oder ein- oder mehrfach substituiert sind durch Substituenten ausgewählt aus der Gruppe bestehend aus Halogen, $C_1$-$C_4$-Alkyl, $C_1$-$C_4$-Alkoxy, Hydroxy, $C_2$-$C_6$-Alkanoyloxy, Trifluormethyl und Nitro.

8. Verbindungen gemäss Anspruch 1 der Formel I und ihre Salze, worin -$A_1$-$A_2$-, -$A_3$-$A_4$-, -$A_5$-$A_6$- die angegebenen Bedeutungen haben; X für $\geq$ C($R_6$)- oder $\geq$ N-steht und $R_6$ Wasserstoff ist; alk $C_1$-$C_7$-Alkylen, insbesondere $C_1$-$C_4$-Alkylen, bedeutet; $R_1$ $C_2$-$C_8$-Alkanoyl, insbesondere $C_2$-$C_6$-Alkanoyl, bedeutet; $R_2$ $C_2$-$C_8$-Alkanoyl, insbesondere $C_2$-$C_6$-Alkanoyl, Benzoyl, $C_1$-$C_4$-Alkansulfonyl oder Benzolsulfonyl bedeutet, $R_3$ und $R_3'$ gemeinsam für eine Bindung stehen oder $R_3$ Wasserstoff, $C_2$-$C_8$-Alkanoyl, insbesondere $C_2$-$C_6$-Alkanoyl, Benzoyl, $C_1$-$C_4$-Alkansulfonyl oder Benzolsulfonyl bedeutet und $R_3'$ Wasserstoff ist; $R_4$ Wasserstoff, $C_3$-$C_7$-Cycloalkyl, Phenyl, Biphenylyl oder Naphthyl bedeutet, $R_5$ Acetyl ist; $R_7$ Wasserstoff oder $C_1$-$C_4$-Alkyl ist; wobei die jeweiligen aromatischen Reste unsubstituiert oder ein- oder mehrfach substituiert sind durch Substituenten ausgewählt aus der Gruppe bestehend aus Halogen, $C_1$-$C_4$-Alkyl, -$C_1$-$C_4$-Alkoxy, Hydroxy, $C_2$-$C_6$-Alkanoyloxy, Trifluormethyl und Nitro.

9. Verbindungen gemäss Anspruch 1 der Formel I und ihre Salze, worin -$A_1$-$A_2$-, -$A_3$-$A_4$-und -$A_5$-$A_6$- die angegebenen Bedeutungen haben; X für $\geq$ C($R_6$)- oder $\geq$ N-steht und $R_6$ Wasserstoff ist; alk $C_1$-$C_4$-Alkylen, wie Methylen oder 2-Methyl-1,3-propylen, oder $C_3$-$C_5$-Alkenylen, wie 1,4-But-2-enylen, bedeutet, wobei die Doppelbindung in höherer als der $\alpha$-Stellung zum Piperazinstickstoff lokalisiert ist; $R_1$ Wasserstoff oder verzweigtes $C_3$-$C_6$-Alkanoyl, wie Pivaloyl, bedeutet; $R_2$ $C_2$-$C_6$-Alkanoyl, wie Acetyl, Propionyl oder Pivaloyl, $C_1$-$C_4$-Alkoxy-carbonyl, wie Methoxy- oder Ethoxy-carbonyl, $C_1$-$C_4$-Alkansulfonyl, wie Methansulfonyl, oder gegebenenfalls durch $C_1$-$C_4$-Alkyl oder Halogen mit Atomnummer bis und mit 35 substituiertes Benzolsulfonyl, wie p-Bromphenyl- oder p-Toluol-sulfonyl, bedeutet, $R_3$ $C_2$-$C_6$-Alkanoyl, wie Acetyl, Propionyl oder Pivaloyl, $C_1$-$C_4$-Alkoxycarbonyl, wie Methoxy- oder Ethoxycarbonyl, $C_1$-$C_4$-Alkansulfonyl, wie Methansulfonyl, oder gegebenenfalls durch $C_1$-$C_4$-Alkyl oder Halogen mit Atomnummer bis und mit 35 substituiertes Benzolsulfonyl, wie p-Bromphenyl- oder p-Toluolsulfonyl, bedeutet; $R_3'$ Wasserstoff ist; $R_4$ Wasserstoff, $C_3$-$C_7$-Cycloalkyl, wie Cyclohexyl, gegebenenfalls durch $C_1$-$C_4$-Alkyl substituiertes Phenyl, Biphenylyl oder Naphthyl, wie 2,4,6-Trimethylphenyl, 4-Biphenylyl oder 2-Naphthyl, bedeutet; $R_5$ Acetyl ist; $R_7$ Wasserstoff ist.

10. Verbindungen gemäss Anspruch 1 der Formel I und ihre Salze, worin -$A_1$-$A_2$-, -$A_3$-$A_4$- und -$A_5$-$A_6$- die angegebenen Bedeutungen haben; X für $\geq$ C($R_6$)- oder $\geq$ N- steht und $R_6$ Wasserstoff ist; alk $C_1$-$C_4$-Alkylen, wie Methylen, 2-Methyl-1,3-propylen oder 3-Methyl-1,4-butylen, bedeutet; $R_1$ Wasserstoff oder verzweigtes $C_3$-$C_6$-Alkanoyl, insbesondere Pivaloyl, bedeutet; $R_2$ $C_2$-$C_6$-Alkanoyl, wie Acetyl, Propionyl oder Pivaloyl, Benzoyl oder $C_1$-$C_4$-Alkyl-sulfonyl, wie Methansulfonyl, bedeutet; $R_3$ Wasserstoff, $C_2$-$C_6$-Alkanoyl, wie Acetyl, Propionyl oder Pivaloyl, Benzoyl oder $C_1$-$C_4$-Alkansulfonyl, wie Methansulfonyl, bedeutet und $R_3'$ Wasserstoff ist; $R_4$ Wasserstoff, $C_3$-$C_7$-Cycloalkyl, wie Cyclopentyl oder -hexyl, gegebenenfalls durch $C_1$-$C_4$-Alkyl substituiertes Phenyl, insbesondere 2,4,6-Trimethylphenyl, bedeutet; $R_5$ Acetyl ist; $R_7$ Wasserstoff oder $C_1$-$C_4$-Alkyl, wie Methyl, bedeutet.

11. Verbindungen gemäss Anspruch 1 der Formel I und ihre Salze, worin die Strukturelemente -$A_1$-$A_2$-, -$A_3$-$A_4$- und -$A_5$-$A_6$- die angegebenen Bedeutungen haben; in erster Linie jeweils Ethylen bedeuten; X für $\geq$ N- steht; $R_1$ verzweigtes $C_3$-$C_6$-Alka noyl, insbesondere Pivaloyl, bedeutet; $R_2$ $C_2$-$C_5$-Alkanoyl, wie Acetyl, bedeutet; $R_3$ $C_2$-$C_5$-Alkanoyl, wie Acetyl, Propionyl oder Pivaloyl, bedeutet; $R_3'$ Wasserstoff ist; $R_5$ Acetyl ist und einerseits alk $C_1$-$C_4$-Alkylen, in erster Linie 2-Methyl-1,3-propylen, bedeutet und $R_4$ Wasserstoff bedeutet oder andererseits alk Methylen ist und $R_4$ 2,4,6-Trimethylphenyl bedeutet und $R_7$ jeweils Wasserstoff ist.

12. Verbindungen gemäss Anspruch 1 der Formel I und ihre Salze, worin die Strukturelemente -$A_1$-$A_2$-, -$A_3$-$A_4$- und -$A_5$-$A_6$- jeweils Ethylen bedeuten; X für $\geq$ N- steht; $R_1$ verzweigtes $C_3$-$C_6$-Alkanoyl, insbesondere Pivaloyl, bedeutet; $R_2$ und $R_3$ jeweils $C_2$-$C_5$-Alkanoyl, wie Acetyl, Propionyl oder Pivaloyl, bedeuten und $R_3'$ Wasserstoff ist; oder $R_2$ $C_2$-$C_5$-Alkanoyl, wie Acetyl, Propionyl oder Pivaloyl, bedeutet, $R_3$ Wasserstoff oder $C_2$-$C_5$-Alkanoyl, wie Acetyl, Propionyl oder Pivaloyl, bedeutet und $R_3'$ Wasserstoff ist

oder $R_3$ und $R_3'$ gemeinsam für eine Bindung stehen; $R_5$ Acetyl ist; alk-$R_4$ Isobutyl, Cyclopentylmethyl, Cyclohexylmethyl, Benzyl, 2,4,6-Trimethylbenzyl, bedeutet; $R_7$ Wasserstoff oder $C_1$-$C_4$-Alkyl, wie Methyl, ist.

13. Verbindungen gemäss Anspruch 1 der Formel I und ihre Salze, worin die Strukturelemente -$A_1$-$A_2$-, -$A_3$-$A_4$- und -$A_5$-$A_6$- jeweils Ethylen bedeuten; X für $>$N- steht; $R_1$ verzweigtes $C_3$-$C_6$-Alkanoyl, insbesondere Pivaloyl, bedeutet; $R_2$ und $R_3$ jeweils $C_2$-$C_5$-Alkanoyl, wie Acetyl, Propionyl oder Pivaloyl, bedeuten und $R_3'$ Wasserstoff ist; $R_5$ Acetyl ist; alk-$R_4$ Isobutyl, Cyclopentylmethyl, Cyclohexylmethyl, Benzyl, 2,4,6-Trimethylbenzyl, bedeutet; $R_7$ Wasserstoff, ferner $C_1$-$C_4$-Alkyl, wie Methyl, ist.

14. Verbindungen gemäss Anspruch 1 der Formel I und ihre Salze, worin die Strukturelemente -$A_1$-$A_2$-, -$A_3$-$A_4$- und -$A_5$-$A_6$- jeweils Ethylen bedeuten; X für $>$N- steht; $R_1$ verzweigtes $C_3$-$C_6$-Alkanoyl, insbesondere Pivaloyl, bedeutet; $R_2$ $C_2$-$C_5$-Alkanoyl, wie Acetyl, Propionyl oder Pivaloyl, bedeutet; $R_3$ und $R_3'$ gemeinsam für eine Bindung stehen; $R_5$ Acetyl ist; alk-$R_4$ Isobutyl, Cyclohexylmethyl, Benzyl, 2,4,6-Trimethylbenzyl, Naphthylmethyl oder Biphenylylmethyl, wie 4-Biphenylylmethyl, bedeutet; $R_7$ Wasserstoff oder $C_1$-$C_4$-Alkyl, wie Methyl, ist.

15. Verbindungen gemäss Anspruch 1 der Formel I und ihre Salze, worin die Strukturelemente -$A_1$-$A_2$-, -$A_3$-$A_4$- und -$A_5$-$A_6$- jeweils Ethylen bedeuten; X für $>$N- steht; $R_1$ verzweigtes $C_3$-$C_6$-Alkanoyl, insbesondere Pivaloyl, bedeutet; $R_2$ $C_2$-$C_5$-Alkanoyl, wie Acetyl, Propionyl oder Pivaloyl, bedeutet; $R_3$ und $R_3'$ jeweils Wasserstoff sind; $R_5$ Acetyl ist; alk-$R_4$ Isobutyl, Cyclohexylmethyl, Benzyl, 2,4,6-Trimethylbenzyl, Naphthylmethyl oder Biphenylylmethyl, wie 4-Biphenylylmethyl, bedeutet; $R_7$ Wasserstoff ist.

16. Verbindungen gemäss Anspruch 1 der Formel I und ihre Salze, worin die Strukturelemente -$A_1$-$A_2$-, -$A_3$-$A_4$- und -$A_5$-$A_6$- jeweils Ethylen bedeuten; X für $>$N- steht; alk $C_1$-$C_4$-Alkylen, insbesondere Methylen, bedeutet; $R_1$ verzweigtes $C_3$-$C_5$-Alkanoyl, insbesondere Pivaloyl, bedeutet; $R_2$ $C_2$-$C_5$-Alkanoyl, insbesondere Acetyl, Propionyl oder Pivaloyl, bedeutet; $R_3$ und $R_3'$ jeweils Wasserstoff sind; $R_4$ 2,4,6-Tri-$C_1$-$C_4$-alkylphenyl, insbesondere 2,4,6-Trimethylphenyl, bedeutet; $R_5$ Acetyl bedeutet; $R_7$ Wasserstoff ist.

17. Verbindungen gemäss Anspruch 1 der Formel I und ihre Salze, worin die Strukturelemente -$A_1$-$A_2$-, -$A_3$-$A_4$- und -$A_5$-$A_6$- jeweils Ethylen bedeuten; X für $>$N- steht, alk $C_1$-$C_4$-Alkylen, insbesondere Methylen, bedeutet; $R_1$ verzweigtes $C_3$-$C_5$-Alkanoyl, insbesondere Pivaloyl, bedeutet; $R_2$ $C_2$-$C_5$-Alkanoyl, insbesondere Acetyl, Propionyl oder Pivaloyl, ist; $R_3$ Wasserstoff oder $C_2$-$C_5$-Alkanoyl, insbesondere Acetyl, Propionyl oder Pivaloyl, bedeutet; $R_3'$ Wasserstoff ist; alk-$R_4$ 2,4,6-Tri-$C_1$-$C_4$-alkylphenyl-$C_1$-$C_4$-alkyl, insbesondere 2,4,6-Trimethylphenylmethyl, bedeutet; $R_5$ Acetyl bedeutet; $R_7$ Wasserstoff, ferner Methyl oder Ethyl ist.

18. Verbindungen gemäss Anspruch 1 der Formel I und ihre Salze, worin die Strukturelemente -$A_1$-$A_2$-, -$A_3$-$A_4$- und -$A_5$-$A_6$- jeweils Ethylen bedeuten; X für $>$N- steht, alk $C_1$-$C_4$-Alkylen, insbesondere Methylen, bedeutet; $R_1$ verzweigtes $C_3$-$C_5$-Alkanoyl, insbesondere Pivaloyl, bedeutet; $R_2$ $C_2$-$C_5$-Alkanoyl, insbesondere Acetyl, Propionyl oder Pivaloyl, ist; $R_3$ und $R_3'$ gemeinsam für eine Bindung stehen; alk-$R_4$ 2,4,6-Tri-$C_1$-$C_4$-alkylphenyl-$C_1$-$C_4$-alkyl, insbesondere 2,4,6-Trimethylphenylmethyl, bedeutet; $R_5$ Acetyl bedeutet; $R_7$ Wasserstoff, ferner Methyl oder Ethyl ist.

19. Verbindungen gemäss Anspruch 1 der Formel I und ihre Salze, worin die Strukturelemente -$A_1$-$A_2$-, -$A_3$-$A_4$- und -$A_5$-$A_6$- jeweils Ethylen bedeuten; X für $>$N- steht; alk $C_1$-$C_4$-Alkylen, insbesondere Methylen, bedeutet; $R_1$ verzweigtes $C_3$-$C_5$-Alkanoyl, in erster Linie Pivaloyl, bedeutet; $R_2$ $C_2$-$C_5$-Alkanoyl, insbesondere Acetyl, Propionyl oder Pivaloyl, ist; $R_3$ $C_2$-$C_5$-Alkanoyl, insbesondere Acetyl, Propionyl oder Pivaloyl, bedeutet; $R_3'$ Wasserstoff ist; alk-$R_4$ 2,4,6-Tri-$C_1$-$C_4$-alkylphenyl-$C_1$-$C_4$-alkyl, insbesondere 2,4,6-Trimethylphenylmethyl, bedeutet; $R_5$ Acetyl bedeutet; $R_7$ Wasserstoff ist.

20. 16,17,18,19,28,29-Hexahydro-4-O-propionyl-8-O-pivaloyl-11-hydroxy-1-desoxy-11,15-desoxo-1,15-oxy-3-[4-(2,4,6-trimethylbenzyl)-piperazin-1-yl]-rifamycin oder ein Salz davon.

21. 16,17,18,19-Tetrahydro-4-O-acetyl-8-O-pivaloyl-11-acetyloxy-1-desoxy-11,15-desoxo-1,15-oxy-3-[4-(2,4,6-trimethylbenzyl)-piperazin-1-yl]-rifamycin oder ein Salz davon.

22. 16,17,18,19,28,29-Hexahydro-4-O-acetyl-8-O-pivaloyl-11-acetyloxy-1-desoxy-11,15-desoxo-1,15-oxy-3-[4-(2,4,6-trimethylbenzyl)-piperazin-1-yl]-rifamycin oder ein Salz davon.

23. 16,17,18,19,28,29-Hexahydro-4-O-acetyl-8-O-pivaloyl-11-acetyloxy-1-desoxy-11,15-desoxo-1,15-oxy-3-(4-cyclohexylmethyl-piperazin-1-yl)-rifamycin oder ein Salz davon.

24. 16,17,18,19,28,29-Hexahydro-4-O-acetyl-8-O-pivaloyl-11-hydroxy-1-desoxy-11,15-desoxo-1,15-oxy-3-[2-methyl-4-(2,4,6-trimethylbenzyl)-piperazin-1-yl]-rifamycin oder ein Salz davon.

25. Verbindung gemäss einem der Ansprüche 1 bis 24 in Form eines pharmazeutisch verwendbaren Salzes.

26. Verbindung gemäss einem der Ansprüche 1 bis 25 zur Anwendung in einem Verfahren zur therapeutischen Behandlung des menschlichen oder tierischen Körpers.

27. Pharmazeutische Präparate enthaltend eine Verbindung gemäss einem der Ansprüche 1 bis 24

oder ein pharmazeutisch verwendbares Salz davon mit geeigneten Hilfs- und Zusatzstoffen.

28. Verwendung einer Verbindung gemäss einem der Ansprüche 1 bis 25 zur Herstellung von pharmazeutischen Präparaten zur Behandlung von Hypolipidaemie und Arteriosklerose.

28. Verfahren zur Herstellung von Verbindungen gemäss einem der Ansprüche 1 bis 25 der Formel I und ihrer Salze, dadurch gekennzeichnet, dass man

a) zur Herstellung von Verbindungen der Formel I und ihrer Salze, worin X für $>$ N- steht, eine Verbindung der Formel

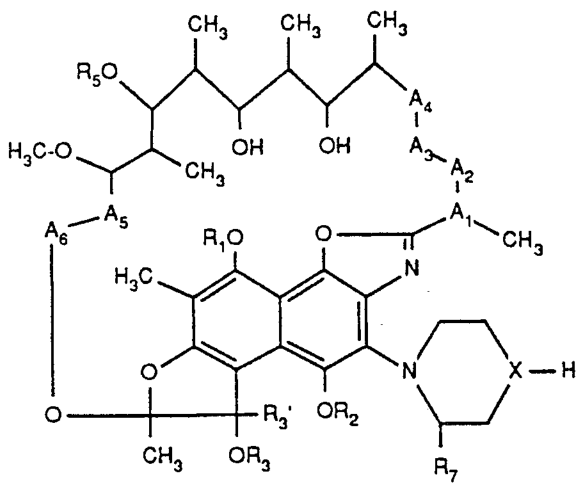

(IIa)

oder ein Salz davon, worin X für $>$ N- steht, mit einer Verbindung der Formel

Z-alk-$R_4$ (IIb)

umsetzt, worin Z reaktionsfähiges verestertes Hydroxy bedeutet, oder

b) eine Verbindung der Formel

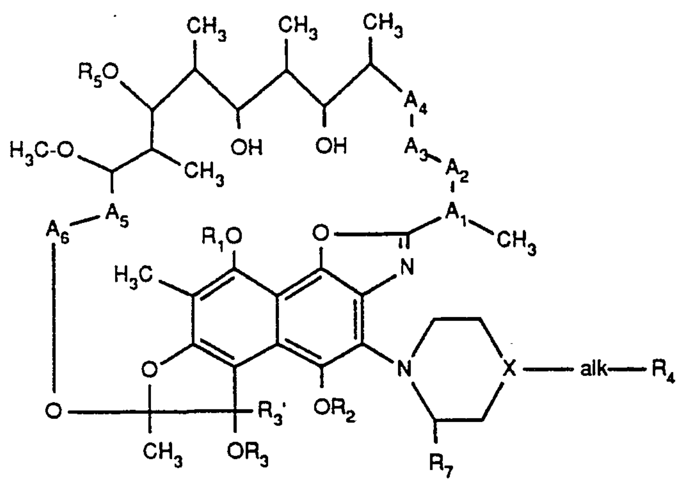

(III),

worin mindestens einer der Reste $R_1$, $R_2$ und/oder $R_3$ Wasserstoff bedeutet, acyliert, oder

c) eine Verbindung der Formel

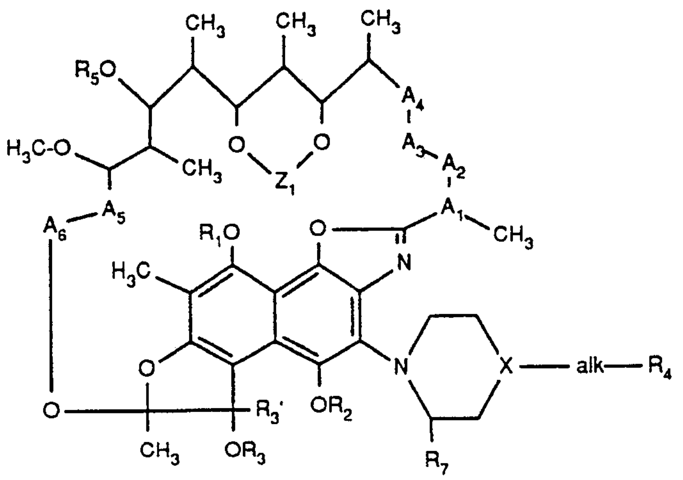

(IV),

worin $Z_1$ Alkyliden oder Cycloalkyliden bedeutet, hydrolysiert, und gewünschtenfalls eine verfahrensgemäss oder auf andere Weise erhältliche Verbindung der Formel I oder ein Salz davon in eine andere erfindungsgemässe Verbindung oder ein Salz davon überführt, eine verfahrensgemäss erhältliche freie Verbindung der Formel I in ein Salz und/oder ein verfahrensgemäss erhältliches Salz in die freie Verbindung der Formel I oder in ein anderes Salz überführt und, wenn erwünscht ein verfahrensgemäss erhältliches Gemisch von Isomeren auftrennt.

29. Die nach dem Verfahren gemäss Anspruch 28 erhältichen Verbindungen.

Patentansprüche für die folgende Vertragsstaaten: ES,GR

1. Verfahren zur Herstellung von Verbindungen der Formel

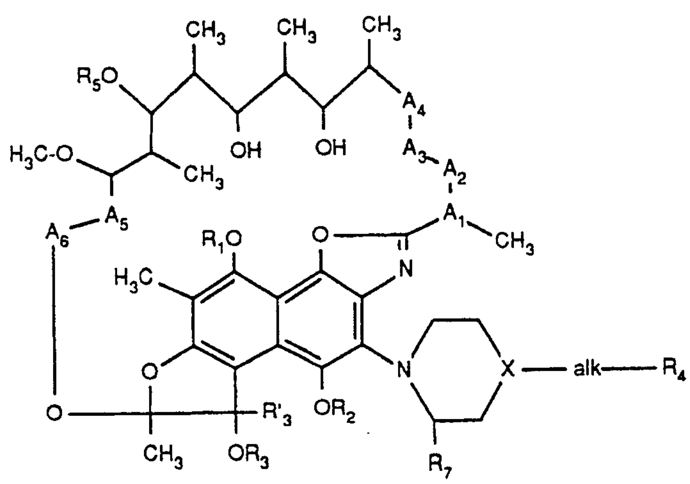

(I),

und ihrer Salze, worin die Strukturelemente $-A_1-A_2-$, $-A_3-A_4-$ und $-A_5-A_6-$ jeweils Ethylen oder Vinylen oder die Elemente $-A_1-A_2-$ und $-A_3-A_4-$ jeweils Ethylen und $-A_5-A_6-$Vinylen bedeuten; X für $>$ C($R_6$)- oder $>$ N- steht und $R_6$ Wasserstoff oder Alkyl bedeutet; alk einen aliphatischen Kohlenwasserstoffrest bedeutet; $R_1$ Wasserstoff oder Acyl bedeutet; $R_2$ Acyl bedeutet und $R_3$ und $R_3'$ für eine gemeinsame Bindung stehen oder $R_3$ Wasserstoff oder Acyl bedeutet und $R_3'$ Wasserstoff ist; $R_4$ Wasserstoff, Cycloalkyl oder Aryl bedeutet; $R_5$ Wasserstoff oder Acetyl bedeutet; $R_7$ Wasserstoff oder Alkyl bedeutet, dadurch gekennzeichnet, dass man

a) zur Herstellung von Verbindungen der Formel I und ihrer Salze, worin X für $>$ N- steht, eine Verbindung der Formel

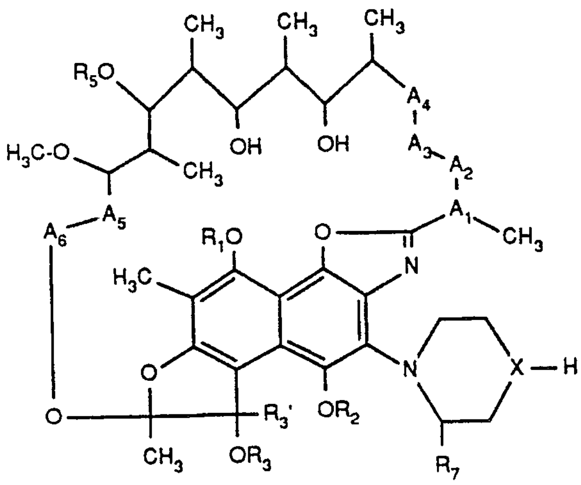

(IIa)

oder ein Salz davon, worin X für $>$ N- steht, mit einer Verbindung der Formel
Z-alk-$R_4$ (IIb)
umsetzt, worin Z reaktionsfähiges verestertes Hydroxy bedeutet, oder

b) eine Verbindung der Formel

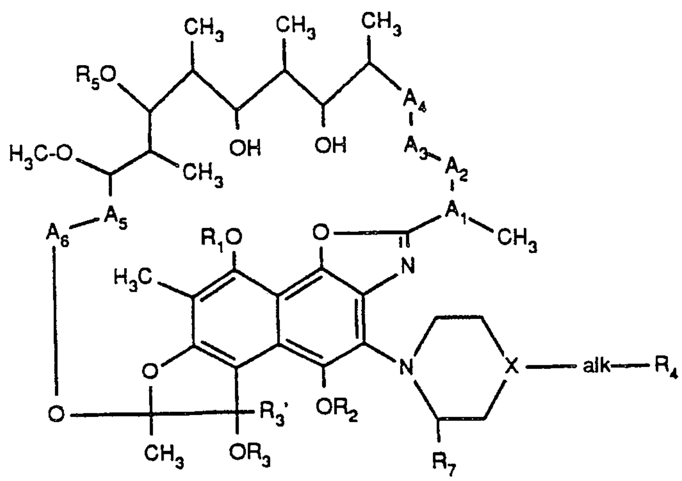

(III),

worin mindestens einer der Reste $R_1$,$R_2$ und/oder $R_3$ Wasserstoff bedeutet, acyliert, oder

c) eine Verbindung der Formel

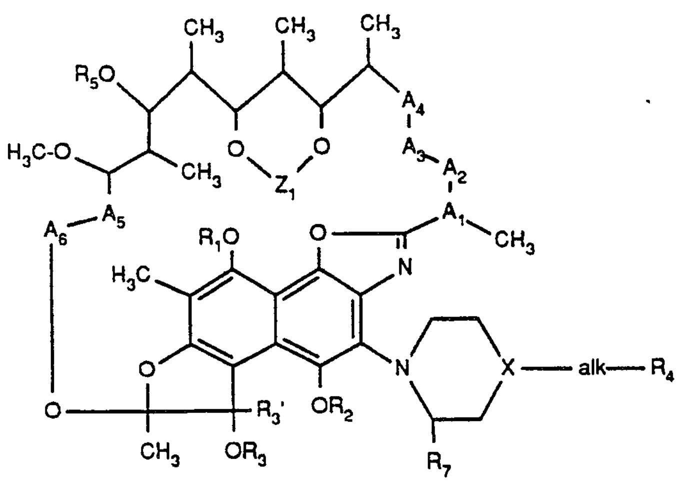

(IV),

worin $Z_1$ Alkyliden oder Cycloalkyliden bedeutet, hydrolysiert, und gewünschtenfalls eine verfahrensgemäss

oder auf andere Weise erhältliche Verbindung der Formel I oder ein Salz davon in eine andere erfindungsgemässe Verbindung oder ein Salz davon überführt, eine verfahrensgemäss erhältliche freie Verbindung der Formel I in ein Salz und/oder ein verfahrensgemäss erhältliches Salz in die freie Verbindung der Formel I oder in ein anderes Salz überführt und, wenn erwünscht ein verfahrensgemäss erhältliches Gemisch von Isomeren auftrennt.

2. Verfahren gemäss Anspruch 1 zur Herstellung von Verbindungen der Formel I und ihrer Salze, worin die Strukturelemente $-A_1-A_2-$, $-A_3-A_4-$ und $-A_5-A_6-$ jeweils Ethylen oder Vinylen oder die Elemente $-A_1-A_2-$ und $-A_3-A_4-$ jeweils Ethylen und $-A_5-A_6-$ Vinylen bedeuten; X für $>$ $C(R_6)$- oder $>$ N- steht und $R_6$ Wasserstoff ist; alk einen aliphatischen Kohlenwasserstoffrest bedeutet; $R_1$ Wasserstoff oder Acyl bedeutet; $R_2$ und $R_3$ unabhängig voneinander Acyl bedeuten; $R_3'$ Wasserstoff bedeutet; $R_4$ Wasserstoff, Cycloalkyl oder Aryl bedeutet; $R_5$ Wasserstoff oder Acetyl bedeutet; $R_7$ Wasserstoff bedeutet.

3. Verfahren gemäss Anspruch 1 zur Herstellung von Verbindungen der Formel I und ihrer Salze, worin $-A_1-A_2-$, $-A_3-A_4-$, $-A_5-A_6-$ und $R_5$ die angegebenen Bedeutungen haben; X für $>$ $C(R_6)$- oder $>$ N-steht und $R_6$ Wasserstoff oder $C_1$-$C_7$-Alkyl bedeutet; alk $C_1$-$C_7$-Alkylen, $C_3$-$C_7$-Alkenylen oder $C_3$-$C_7$-Alkinylen bedeutet; $R_1$ $C_2$-$C_8$-Alkanoyl, Halogen-$C_2$-$C_8$-alkanoyl, Phenyl- oder Naphthyl-$C_2$-$C_8$-alkanoyl, Benzoyl, Naphthoyl, 5-oder 6-gliedriges monocyclisches Monoaza-, Monooxa- oder Monothiaaroyl, $C_1$-$C_7$-Alkoxycarbonyl, Phenyl- oder Naphthyl-$C_1$-$C_7$-alkoxy-carbonyl, unsubstituiertes oder durch $C_1$-$C_7$-Alkyl mono- oder di-substituiertes Aminocarbonyl, $C_1$-$C_7$-Alkansulfonyl, Halo gen-$C_1$-$C_7$-alkansulfonyl, Phenyl- oder Naphthyl-$C_1$-$C_7$-alkansulfonyl, $C_3$-$C_7$-Cycloalkansulfonyl oder Benzol- oder Naphthylsulfonyl bedeutet; $R_2$ $C_2$-$C_8$-Alkanoyl, Halogen-$C_2$-$C_8$-alkanoyl, Phenyl- oder Naphthyl-$C_2$-$C_8$-alkanoyl, Benzoyl, Naphthoyl, 5-oder 6-gliedriges monocyclisches Monoaza-, Monooxa- oder Monothiaaroyl, $C_1$-$C_7$-Alkoxycarbonyl, Phenyl-oder Naphthyl-$C_1$-$C_7$-alkoxy-carbonyl, unsubstituiertes oder durch $C_1$-$C_7$-Alkyl mono- oder di-substituiertes Aminocarbonyl, $C_1$-$C_7$-Alkansulfonyl, Halogen-$C_1$-$C_7$-alkansulfonyl, Phenyl- oder Naphthyl-$C_1$-$C_7$-alkansulfonyl, $C_3$-$C_7$-Cycloalkansulfonyl oder Benzol- oder Naphthylsulfonyl bedeutet, $R_3$ und $R_3'$ für eine gemeinsame Bindung stehen oder $R_3$ Wasserstoff oder $C_2$-$C_8$-Alkanoyl, Halogen-$C_2$-$C_8$-alkanoyl, Phenyl- oder Naphthyl-$C_2$-$C_8$-alkanoyl, Benzoyl, Naphthoyl, 5-oder 6-gliedriges monocyclisches Monoaza-, Monooxa-oder Monothiaaroyl, $C_1$-$C_7$-Alkoxycarbonyl, Phenyl- oder Naphthyl-$C_1$-$C_7$-alkoxy-carbonyl, unsubstituiertes oder durch $C_1$-$C_7$-Alkyl mono- oder di-substituiertes Aminocarbonyl, $C_1$-$C_7$-Alkansulfonyl, Halogen-$C_1$-$C_7$-alkansulfonyl, Phenyl- oder Naphthyl-$C_1$-$C_7$-alkansulfonyl, $C_3$-$C_7$-Cycloalkansulfonyl oder Benzol- oder Naphthylsulfonyl bedeutet und $R_3'$ Wasserstoff ist; $R_4$ Wasserstoff, $C_3$-$C_7$-Cycloalkyl, Phenyl, Biphenylyl oder Naphthyl bedeutet; $R_7$ Wasserstoff oder $C_1$-$C_7$-Alkyl bedeutet; wobei die jeweiligen aromatischen Reste unsubstituiert oder ein- oder mehrfach substituiert sind durch Substituenten ausgewählt aus der Gruppe bestehend aus Halogen, $C_1$-$C_7$-Alkyl, $C_1$-$C_7$-Alkoxy, Hydroxy, $C_2$-$C_8$-Alkanoyloxy, Trifluormethyl und Nitro.

4. Verfahren gemäss Anspruch 1 zur Herstellung von Verbindungen der Formel I und ihrer Salze, worin $-A_1-A_2-$, $-A_3-A_4-$, $-A_5-A_6-$ und $R_5$ die angegebenen Bedeutungen haben; X für $>$ $C(R_6)$- oder $>$ N-steht und $R_6$ Wasserstoff ist; alk $C_1$-$C_7$-Alkylen, $C_3$-$C_7$-Alkenylen oder $C_3$-$C_7$-Alkinylen bedeutet; $R_1$, $R_2$ und $R_3$ unabhängig voneinander $C_2$-$C_8$-Alkanoyl, Halogen-$C_2$-$C_8$-alkanoyl, Phenyl- oder Naphthyl-$C_2$-$C_8$-alkanoyl, Benzoyl, Naphthoyl, 5- oder 6-gliedriges monocyclisches Monoaza-, Monooxa- oder Monothiaaroyl, $C_1$-$C_7$-Alk- oxycarbonyl, Phenyl- oder Naphthyl-$C_1$-$C_7$-alkoxy-carbonyl, unsubstituiertes oder durch $C_1$-$C_7$-Alkyl mono- oder di-substituiertes Aminocarbonyl, $C_1$-$C_7$-Alkansulfonyl, Halogen-$C_1$-$C_7$-alkansulfonyl, Phenyl- oder Naphthyl-$C_1$-$C_7$-alkansulfonyl, $C_3$-$C_7$-Cycloalkansulfonyl oder Benzol- oder Naphthylsulfonyl bedeuten und $R_1$ zusätzlich sowie $R_3'$ und $R_7$ Wasserstoff bedeuten; $R_4$ Wasserstoff, $C_3$-$C_7$-Cycloalkyl, Phenyl, Biphenylyl oder Naphthyl bedeutet; wobei die jeweiligen aromatischen Reste unsubstituiert oder ein- oder mehrfach substituiert sind durch Substituenten ausgewählt aus der Gruppe bestehend aus Halogen, $C_1$-$C_7$-Alkyl, $C_1$-$C_7$-Alkoxy, Hydroxy, $C_2$-$C_8$-Al kanoyloxy, Trifluormethyl und Nitro.

5. Verfahren gemäss Anspruch 1 zur Herstellung von Verbindungen der Formel I und ihrer Salze, worin $-A_1-A_2-$, $-A_3-A_4-$, $-A_5-A_6-$ und $R_5$ die angegebenen Bedeutungen haben; X für $>$ $C(R_6)$- oder $>$ N-steht und $R_6$ Wasserstoff ist; alk $C_1$-$C_7$-Alkylen, $C_3$-$C_7$-Alkenylen oder $C_3$-$C_7$-Alkinylen, insbesondere $C_1$-$C_4$-Alkylen, $C_3$-$C_4$-Alkenylen oder $C_3$-$C_4$-Alkinylen, bedeutet, wobei die Mehrfachbindungen in höherer als der $\alpha$-Stellung zum Piperazinstickstoff lokalisiert sind; $R_1$ $C_2$-$C_8$-Alkanoyl, insbesondere $C_2$-$C_6$-Alkanoyl, Phenyl-$C_2$-$C_6$-alkanoyl, Benzoyl, $C_1$-$C_7$-Alkoxy-carbonyl, wie $C_1$-$C_4$-Alkoxycarbonyl, Phenyl-$C_1$-$C_4$-alkoxycarbonyl, $C_1$-$C_4$-Alkansulfonyl oder Benzolsulfonyl bedeuten; $R_2$ $C_2$-$C_8$-Alkanoyl, insbesondere $C_2$-$C_6$-Alkanoyl, Phenyl-$C_2$-$C_6$-alkanoyl, $C_1$-$C_7$-Alkoxy-carbonyl, wie $C_1$-$C_4$-Alkoxycarbonyl, Phenyl-$C_1$-$C_4$-alkoxycarbonyl, $C_1$-$C_4$-Alkansulfonyl oder Benzolsulfonyl bedeutet; $R_3$ und $R_3'$ gemeinsam für eine Bindung stehen oder $R_3$ Wasserstoff, $C_2$-$C_8$-Alkanoyl, insbesondere $C_2$-$C_6$-Alkanoyl, Phenyl-$C_2$-$C_6$-alkanoyl, Benzoyl, $C_1$-$C_7$-Alkoxy-carbonyl, wie $C_1$-$C_4$-Alkoxycarbonyl, Phenyl-$C_1$-$C_4$-alkoxycarbonyl, $C_1$-$C_4$-Alkansulfonyl oder Benzolsulfonyl bedeutet und $R_3'$ Wasserstoff ist; $R_4$ Wasserstoff, $C_3$-$C_7$-Cycloalkyl, Phenyl, Biphenylyl

oder Naphthyl bedeutet; $R_7$ Wasserstoff oder $C_1$-$C_7$-Alkyl bedeutet; wobei die jeweiligen aromatischen Reste unsubstituiert oder ein- oder mehrfach substituiert sind durch Substituenten ausgewählt aus der Gruppe bestehend aus Halogen, $C_1$-$C_7$-Alkyl, $C_1$-$C_7$-Alkoxy, Hydroxy, $C_2$-$C_8$-Alkanoyloxy, Trifluormethyl und Nitro.

6. Verfahren gemäss Anspruch 1 zur Herstellung von Verbindungen der Formel I und ihrer Salze, worin -$A_1$-$A_2$-, -$A_3$-$A_4$-, -$A_5$-$A_6$- und $R_5$ die angegebenen Bedeutungen haben; X für $>$ C($R_6$)- oder $>$ N-steht und $R_6$ Wasserstoff ist; alk $C_1$-$C_7$-Alkylen, $C_3$-$C_7$-Alkenylen oder $C_3$-$C_7$-Alkinylen, insbesondere $C_1$-$C_4$-Alkylen, $C_3$-$C_4$-Alkenylen oder $C_3$-$C_4$-Alkinylen, bedeutet, wobei die Mehrfachbindungen in höherer als der $\alpha$-Stellung zum Piperazinstickstoff lokalisiert sind; $R_1$, $R_2$ und $R_3$ unabhängig voneinander $C_2$-$C_8$-Alkanoyl, insbesondere $C_2$-$C_6$-Alkanoyl, Phenyl-$C_2$-$C_6$-alkanoyl, $C_1$-$C_7$-Alkoxy-carbonyl, wie $C_1$-$C_4$-Alkoxycarbonyl, Phenyl-$C_1$-$C_4$-alkoxycarbonyl, $C_1$-$C_4$-Alkansulfonyl oder Benzolsulfonyl bedeuten und $R_1$ zusätzlich sowie $R_3$ und $R_7$ Wasserstoff bedeuten; $R_4$ Wasserstoff, $C_3$-$C_7$-Cycloalkyl, Phenyl, Biphenylyl oder Naphthyl bedeutet; wobei die jeweiligen aromatischen Reste unsubstituiert oder ein- oder mehrfach substituiert sind durch Substituenten ausgewählt aus der Gruppe bestehend aus Halogen, $C_1$-$C_7$-Alkyl, $C_1$-$C_7$-Alkoxy, Hydroxy, $C_2$-$C_8$-Alkanoyloxy, Trifluormethyl und Nitro.

7. Verfahren gemäss Anspruch 1 zur Herstellung von Verbindungen der Formel I und ihrer Salze, worin -$A_1$-$A_2$-, -$A_3$-$A_4$-, -$A_5$-$A_6$- die angegebenen Bedeutungen haben; X für $>$ C($R_6$)- oder $>$ N- steht und $R_6$ Wasserstoff ist; alk $C_1$-$C_7$-Alkylen, $C_3$-$C_7$-Alkenylen oder $C_3$-$C_7$-Alkinylen, insbesondere $C_1$-$C_4$-Alkylen, $C_3$-$C_4$-Alkenylen oder $C_3$-$C_4$-Alkinylen, bedeutet, wobei die Mehrfachbindungen in höherer als der $\alpha$-Stellung zum Piperazinstickstoff lokalisiert sind; $R_1$, $R_2$ und $R_3$ unabhängig voneinander $C_2$-$C_8$-Alkanoyl, insbesondere $C_2$-$C_6$-Alkanoyl, $C_1$-$C_4$-Alkoxy-carbonyl, $C_1$-$C_4$-Alkansulfonyl oder Benzolsulfonyl bedeuten, $R_1$ zusätzlich sowie $R_3'$ und $R_7$ Wasserstoff bedeuten; $R_4$ Wasserstoff, $C_3$-$C_7$-Cycloalkyl, Phenyl, Biphenylyl oder Naphthyl bedeutet; $R_5$ Acetyl ist; wobei die jeweiligen aromatischen Reste unsubstituiert oder ein- oder mehrfach substituiert sind durch Substituenten ausgewählt aus der Gruppe bestehend aus Halogen, $C_1$-$C_4$-Alkyl, $C_1$-$C_4$-Alkoxy, Hydroxy, $C_2$-$C_6$-Alkanoyloxy, Trifluormethyl und Nitro.

8. Verfahren gemäss Anspruch 1 zur Herstellung von Verbindungen der Formel I und ihrer Salze, worin -$A_1$-$A_2$-, -$A_3$-$A_4$-, -$A_5$-$A_6$- die angegebenen Bedeutungen haben; X für $>$ C($R_6$)- oder $>$ N- steht und $R_6$ Wasserstoff ist; alk $C_1$-$C_7$-Alkylen, insbesondere $C_1$-$C_4$-Alkylen, bedeutet; $R_1$ $C_2$-$C_8$-Alkanoyl, insbesondere $C_2$-$C_6$-Alkanoyl, bedeutet; $R_2$ $C_2$-$C_8$-Alkanoyl, insbesondere $C_2$-$C_6$-Alkanoyl, Benzoyl, $C_1$-$C_4$-Alkansulfonyl oder Benzolsulfonyl bedeutet, $R_3$ und $R_3'$ gemeinsam für eine Bindung stehen oder $R_3$ Wasserstoff, $C_2$-$C_8$-Alkanoyl, insbesondere $C_2$-$C_6$-Alkanoyl, Benzoyl, $C_1$-$C_4$-Alkansulfonyl oder Benzolsulfonyl bedeutet und $R_3'$ Wasserstoff ist; $R_4$ Wasserstoff, $C_3$-$C_7$-Cycloalkyl, Phenyl, Biphenylyl oder Naphthyl bedeutet, $R_5$ Acetyl ist; $R_7$ Wasserstoff oder $C_1$-$C_4$-Alkyl ist; wobei die jeweiligen aromatischen Reste unsubstituiert oder ein- oder mehrfach substituiert sind durch Substituenten ausgewählt aus der Gruppe bestehend aus Halogen, $C_1$-$C_4$-Alkyl, $C_1$-$C_4$-Alkoxy, Hydroxy, $C_2$-$C_6$-Alkanoyloxy, Trifluormethyl und Nitro.

9. Verfahren gemäss Anspruch 1 zur Herstellung von Verbindungen der Formel I und ihrer Salze, worin -$A_1$-$A_2$-, -$A_3$-$A_4$- und -$A_5$-$A_6$- die angegebenen Bedeutungen haben; X für $>$ C($R_6$)- oder $>$ N- steht und $R_6$ Wasserstoff ist; alk $C_1$-$C_4$-Alkylen, wie Methylen oder 2-Methyl-1,3-propylen, oder $C_3$-$C_5$-Alkenylen, wie 1,4-But-2-enylen, bedeutet, wobei die Doppelbindung in höherer als der $\alpha$-Stellung zum Piperazinstickstoff lokalisiert ist; $R_1$ Wasserstoff oder verzweigtes $C_3$-$C_6$-Alkanoyl, wie Pivaloyl, bedeutet; $R_2$ $C_2$-$C_6$-Alkanoyl, wie Acetyl, Propionyl oder Pivaloyl, $C_1$-$C_4$-Alkoxy-carbonyl, wie Methoxy- oder Ethoxy-carbonyl, $C_1$-$C_4$-Alkansulfonyl, wie Methansulfonyl, oder gegebenenfalls durch $C_1$-$C_4$-Alkyl oder Halogen mit Atomnummer bis und mit 35 substituiertes Benzolsulfonyl, wie p-Bromphenyl- oder p-Toluol-sulfonyl, bedeutet, $R_3$ $C_2$-$C_6$-Alkanoyl, wie Acetyl, Propionyl oder Pivaloyl, $C_1$-$C_4$-Alkoxycarbonyl, wie Methoxy- oder Ethoxycarbonyl, $C_1$-$C_4$-Alkansulfonyl, wie Methansulfonyl, oder gegebenenfalls durch $C_1$-$C_4$-Alkyl oder Halogen mit Atomnummer bis und mit 35 substituiertes Benzolsulfonyl, wie p-Bromphenyl- oder p-Toluolsulfonyl, bedeutet; $R_3'$ Wasserstoff ist; $R_4$ Wasserstoff, $C_3$-$C_7$-Cycloalkyl, wie Cyclohexyl, gegebenenfalls durch $C_1$-$C_4$-Alkyl substituiertes Phenyl, Biphenylyl oder Naphthyl, wie 2,4,6-Trimethylphenyl, 4-Biphenylyl oder 2-Naphthyl, bedeutet; $R_5$ Acetyl ist; $R_7$ Wasserstoff ist.

10. Verfahren gemäss Anspruch 1 zur Herstellung von Verbindungen der Formel I und ihrer Salze, worin -$A_1$-$A_2$-, -$A_3$-$A_4$- und -$A_5$-$A_6$- die angegebenen Bedeutungen haben; X für $>$ C($R_6$)- oder $>$ N-steht und $R_6$ Wasserstoff ist; alk $C_1$-$C_4$-Alkylen, wie Methylen, 2-Methyl-1,3-propylen oder 3-Methyl-1,4-butylen, bedeutet; $R_1$ Wasserstoff oder verzweigtes $C_3$-$C_6$-Alkanoyl, insbesondere Pivaloyl, bedeutet; $R_2$ $C_2$-$C_6$-Alkanoyl, wie Acetyl, Propionyl oder Pivaloyl, Benzoyl oder $C_1$-$C_4$-Alkyl-sulfonyl, wie Methansulfonyl, bedeutet; $R_3$ Wasserstoff, $C_2$-$C_6$-Alkanoyl, wie Acetyl, Propionyl oder Pivaloyl, Benzoyl oder $C_1$-$C_4$-Alkansulfonyl, wie Methansulfonyl, bedeutet und $R_3'$ Wasserstoff ist; $R_4$ Wasserstoff, $C_3$-$C_7$-Cycloalkyl, wie Cyclopentyl oder -hexyl, gegebenenfalls durch $C_1$-$C_4$-Alkyl substituiertes Phenyl, insbesondere 2,4,6-Trimethylphenyl, bedeutet; $R_5$ Acetyl ist; $R_7$ Wasserstoff oder $C_1$-$C_4$-Alkyl, wie Methyl, bedeutet.

11. Verfahren gemäss Anspruch 1 zur Herstellung von Verbindungen der Formel I und ihrer Salze, worin die Strukturelemente $-A_1-A_2-$, $-A_3-A_4-$ und $-A_5-A_6-$ die angegebenen Bedeutungen haben; in erster Linie jeweils Ethylen bedeuten; X für $\geq$ N- steht; $R_1$ verzweigtes $C_3$-$C_6$-Alkanoyl, insbesondere Pivaloyl, bedeutet; $R_2$ $C_2$-$C_5$-Alkanoyl, wie Acetyl, bedeutet; $R_3$ $C_2$-$C_5$-Alkanoyl, wie Acetyl, Propionyl oder Pivaloyl, bedeutet; $R_3'$ Wasserstoff ist; $R_5$ Acetyl ist und einerseits alk $C_1$-$C_4$-Alkylen, in erster Linie 2-Methyl-1,3-propylen, bedeutet und $R_4$ Wasserstoff bedeutet oder andererseits alk Methylen ist und $R_4$ 2,4,6-Trimethylphenyl bedeutet und $R_7$ jeweils Wasserstoff ist.

12. Verfahren gemäss Anspruch 1 zur Herstellung von Verbindungen der Formel I und ihrer Salze, worin die Strukturelemente $-A_1-A_2-$, $-A_3-A_4-$ und $-A_5-A_6-$ jeweils Ethylen bedeuten; X für $\geq$ N- steht; $R_1$ verzweigtes $C_3$-$C_6$-Alkanoyl, insbesondere Pivaloyl, bedeutet; $R_2$ und $R_3$ jeweils $C_2$-$C_5$-Alkanoyl, wie Acetyl, Propionyl oder Pivaloyl, bedeuten und $R_3'$ Wasserstoff ist; oder $R_2$ $C_2$-$C_5$-Alkanoyl, wie Acetyl, Propionyl oder Pivaloyl, bedeutet, $R_3$ Wasserstoff oder $C_2$-$C_5$-Alkanoyl, wie Acetyl, Propionyl oder Pivaloyl, bedeutet und $R_3'$ Wasserstoff ist oder $R_3$ und $R_3'$ gemeinsam für eine Bindung stehen; $R_5$ Acetyl ist; alk-$R_4$ Isobutyl, Cyclopentylmethyl, Cyclohexylmethyl, Benzyl, 2,4,6-Trimethylbenzyl, bedeutet; $R_7$ Wasserstoff oder $C_1$-$C_4$-Alkyl, wie Methyl, ist.

13. Verfahren gemäss Anspruch 1 zur Herstellung von Verbindungen der Formel I und ihrer Salze, worin die Strukturelemente $-A_1-A_2-$, $-A_3-A_4-$ und $-A_5-A_6-$ jeweils Ethylen bedeuten; X für $\geq$ N- steht; $R_1$ verzweigtes $C_3$-$C_6$-Alkanoyl, insbesondere Pivaloyl, bedeutet; $R_2$ und $R_3$ jeweils $C_2$-$C_5$-Alkanoyl, wie Acetyl, Propionyl oder Pivaloyl, bedeuten und $R_3'$ Wasserstoff ist; $R_5$ Acetyl ist; alk-$R_4$ Isobutyl, Cyclopentylmethyl, Cyclohexylmethyl, Benzyl, 2,4,6-Trimethylbenzyl, bedeutet; $R_7$ Wasserstoff, ferner $C_1$-$C_4$-Alkyl, wie Methyl, ist.

14. Verfahren gemäss Anspruch 1 zur Herstellung von Verbindungen der Formel I und ihrer Salze, worin die Strukturelemente $-A_1-A_2-$, $-A_3-A_4-$ und $-A_5-A_6-$ jeweils Ethylen bedeuten; X für $\geq$ N- steht; $R_1$ verzweigtes $C_3$-$C_6$-Alkanoyl, insbesondere Pivaloyl, bedeutet; $R_2$ $C_2$-$C_5$-Alkanoyl, wie Acetyl, Propionyl oder Pivaloyl, bedeutet; $R_3$ und $R_3'$ gemeinsam für eine Bindung stehen; $R_5$ Acetyl ist; alk-$R_4$ Isobutyl, Cyclohexylmethyl, Benzyl, 2,4,6-Trimethylbenzyl, Naphthylmethyl oder Biphenylylmethyl, wie 4-Biphenylylmethyl, bedeutet; $R_7$ Wasserstoff oder $C_1$-$C_4$-Alkyl, wie Methyl, ist.

15. Verfahren gemäss Anspruch 1 zur Herstellung von Verbindungen der Formel I und ihrer Salze, worin die Strukturelemente $-A_1-A_2-$, $-A_3-A_4-$ und $-A_5-A_6-$ jeweils Ethylen bedeuten; X für $\geq$ N- steht; $R_1$ verzweigtes $C_3$-$C_6$-Alkanoyl, insbesondere Pivaloyl, bedeutet; $R_2$ $C_2$-$C_5$-Alkanoyl, wie Acetyl, Propionyl oder Pivaloyl, bedeutet; $R_3$ und $R_3'$ jeweils Wasserstoff sind; $R_5$ Acetyl ist; alk-$R_4$ Isobutyl, Cyclohexylmethyl, Benzyl, 2,4,6-Trimethylbenzyl, Naphthylmethyl oder Biphenylylmethyl, wie 4-Biphenylylmethyl, bedeutet; $R_7$ Wasserstoff ist.

16. Verfahren gemäss Anspruch 1 zur Herstellung von Verbindungen der Formel I und ihrer Salze, worin die Strukturelemente $-A_1-A_2-$, $-A_3-A_4-$ und $-A_5-A_6-$ jeweils Ethylen bedeuten; X für $\geq$ N- steht; alk $C_1$-$C_4$-Alkylen, insbesondere Methylen, bedeutet; $R_1$ verzweigtes $C_3$-$C_5$-Alkanoyl, insbesondere Pivaloyl, bedeutet; $R_2$ $C_2$-$C_5$-Alkanoyl, insbesondere Acetyl, Propionyl oder Pivaloyl, bedeutet; $R_3$ und $R_3'$ jeweils Wasserstoff sind; $R_4$ 2,4,6-Tri-$C_1$-$C_4$-alkylphenyl, insbesondere 2,4,6-Trimethylphenyl, bedeutet; $R_5$ Acetyl bedeutet; $R_7$ Wasserstoff ist.

17. Verfahren gemäss Anspruch 1 zur Herstellung von Verbindungen der Formel I und ihrer Salze, worin die Strukturelemente $-A_1-A_2-$, $-A_3-A_4-$ und $-A_5-A_6-$ jeweils Ethylen bedeuten; X für $\geq$ N- steht, alk $C_1$-$C_4$-Alkylen, insbesondere Methylen, bedeutet; $R_1$ verzweigtes $C_3$-$C_5$-Alkanoyl, insbesondere Pivaloyl, bedeutet; $R_2$ $C_2$-$C_5$-Alkanoyl, insbesondere Acetyl, Propionyl oder Pivaloyl, ist; $R_3$ Wasserstoff oder $C_2$-$C_5$-Alkanoyl, insbesondere Acetyl, Propionyl oder Pivaloyl, bedeutet; $R_3'$ Wasserstoff ist; alk-$R_4$ 2,4,6-Tri-$C_1$-$C_4$-alkylphenyl-$C_1$-$C_4$-alkyl, insbesondere 2,4,6-Trimethylphenylmethyl, bedeutet; $R_5$ Acetyl bedeutet; $R_7$ Wasserstoff, ferner Methyl oder Ethyl ist.

18. Verbindungen gemäss Anspruch 1 der Formel I und ihre Salze, worin die Strukturelemente $-A_1-A_2-$, $-A_3-A_4-$ und $-A_5-A_6-$ jeweils Ethylen bedeuten; X für $\geq$ N— steht, alk $C_1$-$C_4$-Alkylen, insbesondere Methylen, bedeutet; $R_1$ verzweigtes $C_3$-$C_5$-Alkanoyl, insbesondere Pivaloyl, bedeutet; $R_2$ $C_2$-$C_5$-Alkanoyl, insbesondere Acetyl, Propionyl oder Pivaloyl, ist; $R_3$ und $R_3'$ gemeinsam für eine Bindung stehen; alk-$R_4$ 2,4,6-Tri-$C_1$-$C_4$-alkylphenyl-$C_1$-$C_4$-alkyl, insbesondere 2,4,6-Trimethylphenylmethyl, bedeutet; $R_5$ Acetyl bedeutet; $R_7$ Wasserstoff, ferner Methyl oder Ethyl ist.

19. Verfahren gemäss Anspruch 1 zur Herstellung von Verbindungen der Formel I und ihrer Salze, worin die Strukturelemente $-A_1-A_2-$, $-A_3-A_4-$ und $-A_5-A_6-$ jeweils Ethylen bedeuten; X für $\geq$ N- steht; alk $C_1$-$C_4$-Alkylen, insbesondere Methylen, bedeutet; $R_1$ verzweigtes $C_3$-$C_5$-Alkanoyl, in erster Linie Pivaloyl, bedeutet; $R_2$ $C_2$-$C_5$-Alkanoyl, insbesondere Acetyl, Propionyl oder Pivaloyl, ist; $R_3$ $C_2$-$C_5$-Alkanoyl, insbesondere Acetyl, Propionyl oder Pivaloyl, bedeutet; $R_3'$ Wasserstoff ist; alk-$R_4$ 2,4,6-Tri-$C_1$-$C_4$-alkylphenyl-$C_1$-$C_4$-alkyl, insbesondere 2,4,6-Trimethylphenylmethyl, bedeutet; $R_5$ Acetyl bedeutet; $R_7$ Wasserstoff ist.

20. Verfahren gemäss Anspruch 1 zur Herstellung von 16,17,18,19,28,29-Hexahydro- 4-O-propionyl-8-O-pivaloyl-11-hydroxy-1′-desoxy-11,15-desoxo-1,15-oxy-3-[4-(2,4,6-trimethylbenzyl)-piperazin-1-yl]-rifamycin oder eines Salzes davon.

21. Verfahren gemäss Anspruch 1 zur Herstellung von 16,17,18,19-Tetrahydro-4-O-acetyl-8-O-pivaloyl-11-acetyloxy-1-desoxy-11, 15-desoxo-1,15-oxy-3-[4-(2,4,6-trimethylbenzyl)-piperazin-1-yl]-rifamycin oder eines Salzes davon.

22. Verfahren gemäss Anspruch 1 zur Herstellung von 16,17,18,19,28,29-Hexahydro-4-O-acetyl-8-O-pivaloyl-11-acetyloxy-1-desoxy-11,15-desoxo-1,15-oxy-3-[4-(2,4,6-trimethylbenzyl)-piperazin-1-yl]-rifamycin oder eines Salzes davon.

23. Verfahren gemäss Anspruch 1 zur Herstellung von 16,17,18,19,28,29-Hexahydro-4-O-acetyl-8-O-pivaloyl-11-acetyloxy-1-desoxy-11,15-desoxo-1,15-oxy-3-(4-cyclohexylmethyl-piperazin-1-yl)-rifamycin oder eines Salzes davon.

24. Verfahren gemäss Anspruch 1 zur Herstellung von 16,17,18,19,28,29-Hexahydro-4-O-acetyl-8-O-pivaloyl-11-hydroxy-1-desoxy-11,15-desoxo-1,15-oxy-3-[2-methyl-4-(2,4,6-trimethylbenzyl)-piperazin-1-yl]-rifamycin oder eines Salzes davon.

25. Verfahren gemäss Anspruch 1 zur Herstellung einer Verbindung gemäss einem der Ansprüche 1 bis 24 in Form eines pharmzeutisch verwendbaren Salzes.

26. Verfahren zur Herstellung von pharmazeutischen Präparaten enthaltend eine Verbindung gemäss einem der Ansprüche 1 bis 24 oder ein pharmazeutisch verwendbares Salzes davon, dadurch gekennzeichnet, dass man den Wirkstoff mit geeigneten Hilfs- und Zusatzstoffen zu pharmazeutischen Präparaten verarbeitet.

27. Verwendung von Verbindungen gemäss einem der Ansprüche 1 bis 25 zur Herstellung von pharmazeutischen Präpraten zur Behandlung von Hypolipidaemie und Arteriosklerose.